# EUROPEAN PATENT APPLICATION

(11) **EP 4 806 897 A1**
(43) Date of publication of application: **16.09.2026**
(21) Application number: 24887943.9
(22) Date of filing: 05.11.2024
(51) Int. Cl.: C07D 471/10, A61K 31/506, A61P 35/00, A61P 35/02

(54) **AZA-BRIDGED RING DERIVATIVE**

(30) Priority: 06.11.2023 CN 202311466782; 03.02.2024 CN 202410155014; 31.10.2024 CN 202411547399
(71) Applicant: Chia Tai Tianqing Pharmaceutical Group Co., Ltd., Lianyungang, Jiangsu 222062 (CN)
(72) Inventor: LIU, Yanlong, Lianyungang, Jiangsu 222062 (CN); FENG, Taotao, Lianyungang, Jiangsu 222062 (CN); HU, Zhongyuan, Lianyungang, Jiangsu 222062 (CN); XU, Hongjiang, Lianyungang, Jiangsu 222062 (CN); LIU, Jin, Lianyungang, Jiangsu 222062 (CN); LIU, Fei, Lianyungang, Jiangsu 222062 (CN)
(74) Representative: Potter Clarkson
(86) International application number: PCT/CN2024/129950
(87) International publication number: WO 2025/098331

(57) **Abstract**

Provided are a pharmaceutically acceptable aza-bridge ring derivative, and specifically relates to a compound represented by formula (III) or a pharmaceutically acceptable salt thereof, a preparation method therefor, a pharmaceutical composition containing the compound, and a use thereof in treating diseases.

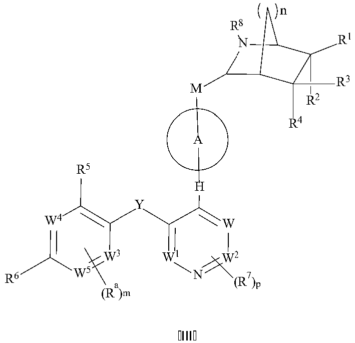

## Description

### CROSS-REFERENCE TO RELATED APPLICATIONS

The present application claims priority to and benefits of the Chinese Patent Application No. 202311466782.3 filed with China National Intellectual Property Administration on Nov. 06, 2023, the Chinese Patent Application No. 202410155014.4 filed with China National Intellectual Property Administration on Feb. 03, 2024, and the Chinese Patent Application No. 202411547399.5 filed with China National Intellectual Property Administration on Oct. 31, 2024, the disclosure of each of which is incorporated herein by reference in its entirety.

### TECHNICAL FIELD

The present disclosure pertains to the technical field of medicines, and relates to a pharmaceutically acceptable aza-bridged ring derivative or a pharmaceutically acceptable salt thereof, a preparation method therefor, a pharmaceutical composition comprising the compound, and use thereof for treating diseases.

### BACKGROUND

The Menin protein is encoded by the multiple endocrine neoplasia type 1 gene *(MENT).* The Menin protein is a widely expressed nuclear protein that mainly regulates tissue-specific gene expression. Menin interacts with DNA via its C-terminal nuclear localization sequence (NLS), including binding to promoters, transcription factors, or enhancers, thereby regulating gene expression and cellular signaling.

In different tissues, the Menin protein can bind to different proteins (e.g., MLL1 (mixed lineage leukemia protein 1), MYC, JunD, etc.), resulting in diverse downstream functions. For example, the direct interaction between the Menin protein and the amino-terminal sequence of an MLL protein is necessary for leukemogenesis mediated by MLL-rearranged (MLLr) fusion proteins. The Menin protein binds to the first 10 amino acids of the MLL protein, a binding site that is conserved across all MLL fusion proteins. It acts as a cofactor for the binding of MLL proteins to the HOX gene promoter regions and also serves as a critical oncogenic cofactor in the development and progression of tumors driven by MLLr proteins.

Currently, no drugs targeting the Menin-MLL protein-protein interaction are available on the market. There remains a need in the art to develop new compounds possessing selective inhibitory activity, better pharmacodynamics, or better pharmacokinetic properties.

### SUMMARY

The present disclosure relates to a compound of formula (III) or a pharmaceutically acceptable salt thereof: 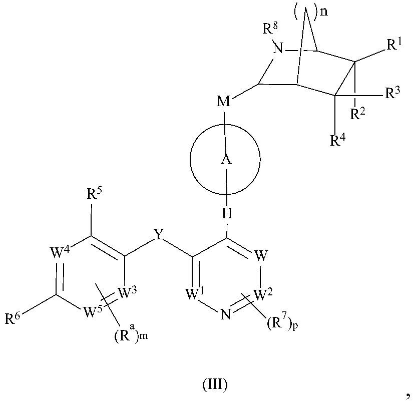 wherein
R¹, R², R³, and R⁴ are each independently selected from the group consisting of hydrogen, halogen, -OH, -CN, C₁₋₆ alkyl, C₂₋₆ alkenyl, C₂₋₆ alkynyl, -C₁₋₆ alkylene-C₃₋₁₀ cycloalkyl, -C₁₋₆ alkylene-3- to 10-membered heterocycloalkyl, -C₁₋₆ alkylene-C₆₋₁₀ aryl, -C₁₋₆ alkylene-5- to 10-membered heteroaryl, -OR^{b1}, -SR^{b1}, -NR^{c1}R^{d1}, -C(O)R^{b1}, -OC(O)R^{b1}, -C(O)OR^{b1}, -S(O)R^{b1}, -S(O)NR^{c1}R^{d1}, -N(R^{c1})S(O)R^{d1}, -S(O)₂R^{b1}, -S(O)₂NR^{c1}R^{d1}, -N(R^{c1})S(O)₂R^{d1}, -OC(O)NR^{c1}R^{d1}, -N(R^{c1})C(O)R^{d1}, and -C(O)NR^{c1}R^{d1}, wherein the C₁₋₆ alkyl, C₂₋₆ alkenyl, C₂₋₆ alkynyl, -C₁₋₆ alkylene-C₃₋₁₀ cycloalkyl, -C₁₋₆ alkylene-3- to 10-membered heterocycloalkyl, -C₁₋₆ alkylene-C₆₋₁₀ aryl, or -C₁₋₆ alkylene-5- to 10-membered heteroaryl is optionally substituted with one or more -OH, -CN, -NH₂, halogen, C₁₋₆ alkyl, or C₁₋₆ heteroalkyl;
or, R¹ and R² are taken together to form =O, =S, or =CR^{c2}R^{d2}; and/or, R³ and R⁴ are taken together to form =O, =S, or =CR^{c2}R^{d2};
R^{b1}, R^{c1}, and R^{d1} are each independently selected from the group consisting of hydrogen, C₁₋₆ alkyl, C₂₋₆ alkenyl, C₂₋₆ alkynyl, C₁₋₆ heteroalkyl, C₃₋₁₀ cycloalkyl, 3- to 10-membered heterocycloalkyl, C₆₋₁₀ aryl, and 5- to 10-membered heteroaryl, wherein the C₁₋₆ alkyl, C₂₋₆ alkenyl, C₂₋₆ alkynyl, C₃₋₁₀ cycloalkyl, 3- to 10-membered heterocycloalkyl, C₆₋₁₀ aryl, or 5- to 10-membered heteroaryl is optionally substituted with one or more halogen, -CN, -OH, -NH₂, C₁₋₆ alkyl, or C₁₋₆ heteroalkyl;
R^{c2} and R^{d2} are each independently selected from the group consisting of hydrogen, C₁₋₆ alkyl, C₂₋₆ alkenyl, C₂₋₆ alkynyl, and C₁₋₆ heteroalkyl, wherein the C₁₋₆ alkyl, C₂₋₆ alkenyl, C₂₋₆ alkynyl, or C₁₋₆ heteroalkyl is optionally substituted with one or more halogen, -CN, -OH, or -NH₂;
R⁸ is selected from the group consisting of hydrogen and C₁₋₆ alkyl, wherein the C₁₋₆ alkyl is optionally substituted with 1 or more groups selected from the group consisting of halogen, -CN, -OH, and -NH₂;
Y is selected from the group consisting of O, S, CR^{x}R^{y}, and NR^{z}, wherein R^{x}, R^{y}, or R^{z} is each independently selected from the group consisting of hydrogen and C₁₋₆ alkyl;
W, W¹, W², W³, W⁴, and W⁵ are each independently selected from the group consisting of N, C, and CH;
ring A is selected from the group consisting of the following groups optionally substituted with one or more R^{a1}: C₃₋₁₂ cycloalkylene, 3- to 12-membered heterocyclylene, C₆₋₁₀ arylene, and 5- to 12-membered heteroarylene;
H is selected from the group consisting of a bond, NR^{z}, C₃₋₈ cycloalkyl, and 3-8 heterocycloalkyl, wherein R^{z} is selected from the group consisting of hydrogen and C₁₋₆ alkyl;
each R^{a1} is independently selected from the group consisting of halogen, -OH, -CN, -NH₂, =O, =S, C₁₋₆ alkyl, C₂₋₆ alkenyl, C₂₋₆ alkynyl, and C₁₋₆ heteroalkyl;
R⁵ is selected from the group consisting of halogen, -OH, -CN, -NH₂, and the following groups optionally substituted with one or more R^{a"}: -C(O)NR^{a'}R^{b'}, -N(R^{b'})C(O)R^{b'}, -N(R^{b'})C(O)OR^{b'}, -OC(O)NR^{b'}R^{b'}, -C(O)R^{b'}, -S(O)R^{b'}, -S(O)₂R^{b'}, -N(R^{b'})S(O)₂R^{b'}, -N(R^{b'})S(O)R^{b'}, -S(O)NR^{b'}R^{b'}, -S(O)₂NR^{b'}R^{b'}, -OC(O)R^{b'}, -C(O)OR^{b'}, C₃₋₁₀ cycloalkyl, 3- to 10-membered heterocycloalkyl, C₆₋₁₀ aryl, and 5- to 10-membered heteroaryl;
R^{a'} is selected from the group consisting of hydrogen, C₁₋₆ alkyl, C₂₋₆ alkenyl, C₂₋₆ alkynyl, C₃₋₆ cycloalkyl, and C₃₋₆ heterocycloalkyl;
each R^{b'} is independently selected from the group consisting of hydrogen, C₁₋₆ alkyl, C₁₋₆ haloalkyl, C₂₋₆ alkenyl, C₂₋₆ alkynyl, C₃₋₁₀ cycloalkyl, 3- to 10-membered heterocycloalkyl, C₆₋₁₀ aryl, and 5- to 10-membered heteroaryl, wherein the C₁₋₆ alkyl, C₃₋₁₀ cycloalkyl, 3- to 10-membered heterocycloalkyl, C₆₋₁₀ aryl, or 5- to 10-membered heteroaryl is optionally substituted with one or more halogen, -CN, -OH, or -NH₂;
each R^{a"} is independently selected from the group consisting of halogen, -OH, -CN, -NH₂, -NH(C₁₋₆ alkyl), -N(C₁₋₆ alkyl)₂, C₁₋₆ alkyl, C₂₋₆ alkenyl, C₂₋₆ alkynyl, C₁₋₆ alkyl-O-, C₃₋₁₀ cycloalkyl, 3- to 10-membered heterocycloalkyl, C₆₋₁₀ aryl, and 5- to 10-membered heteroaryl, wherein the -NH(C₁₋₆ alkyl), -N(C₁₋₆ alkyl)₂, C₁₋₆ alkyl, C₂₋₆ alkenyl, C₂₋₆ alkynyl, C₁₋₆ alkyl-O-, C₃₋₁₀ cycloalkyl, 3- to 10-membered heterocycloalkyl, C₆₋₁₀ aryl, or 5- to 10-membered heteroaryl is optionally substituted with one or more halogen, -OH, -CN, or -NH₂;
R⁶ is selected from the group consisting of hydrogen, halogen, -OH, -CN, -NH₂, C₁₋₆ alkyl, C₂₋₆ alkenyl, C₂₋₆ alkynyl, C₁₋₆ heteroalkyl, C₃₋₁₀ cycloalkyl, 3- to 10-membered heterocycloalkyl, C₆₋₁₀ aryl, and 5- to 10-membered heteroaryl, wherein the C₁₋₆ alkyl, C₂₋₆ alkenyl, C₂₋₆ alkynyl, C₁₋₆ heteroalkyl, C₃₋₁₀ cycloalkyl, 3- to 10-membered heterocycloalkyl, C₆₋₁₀ aryl, or 5- to 10-membered heteroaryl is optionally substituted with one or more halogen, - CN, -OH, -NH₂, C₁₋₆ alkyl, C₁₋₆ alkyl-O-, C₂₋₆ alkenyl, C₂₋₆ alkynyl, C₃₋₁₀ cycloalkyl, or 3- to 10-membered heterocycloalkyl;
R² and R⁷ are each independently selected from the group consisting of halogen, -CN, -OH, -NH₂, C₁₋₆ alkyl, C₂₋₆ alkenyl, C₂₋₆ alkynyl, -OC₁₋₆ alkyl, C₃₋₆ cycloalkyl, and 3- to 6-membered heterocycloalkyl, wherein the C₁₋₆ alkyl, C₂₋₆ alkenyl, C₂₋₆ alkynyl, -OC₁₋₆ alkyl, C₃₋₆ cycloalkyl, or 3- to 6-membered heterocycloalkyl is optionally substituted with one or more halogen, -CN, -OH, or -NH₂;
M is selected from the group consisting of -C(O)- and -(CR^{u}R^{v})_{q}-; R^{u} and R^{v} are each independently selected from the group consisting of hydrogen, halogen, -CN, -OH, -NH₂, C₁₋₆ alkyl, C₂₋₆ alkenyl, C₂₋₆ alkynyl, -OC₁₋₆ alkyl, C₃₋₁₀ cycloalkyl, and 3- to 10-membered heterocycloalkyl;
n is selected from the group consisting of 1, 2, 3, and 4;
m and p are each independently selected from the group consisting of 0, 1, 2, and 3;
q is selected from the group consisting of 1, 2, and 3;
provided that when 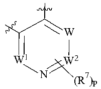 is 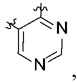 R⁵ is not.

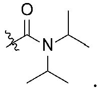

In another aspect, the present disclosure relates to a compound of formula (II) or a pharmaceutically acceptable salt thereof: 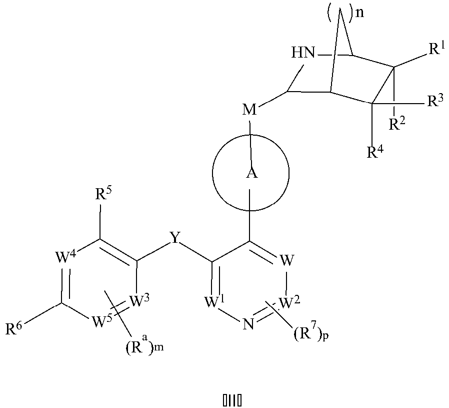 wherein
R¹, R², R³, and R⁴ are each independently selected from the group consisting of hydrogen, halogen, -OH, -CN, C₁₋₆ alkyl, C₂₋₆ alkenyl, C₂₋₆ alkynyl, -C₁₋₆ alkylene-C₃₋₁₀ cycloalkyl, -C₁₋₆ alkylene-3- to 10-membered heterocycloalkyl, -C₁₋₆ alkylene-C₆₋₁₀ aryl, -C₁₋₆ alkylene-5- to 10-membered heteroaryl, -OR^{b1}, -SR^{b1}, -NR^{c1}R^{d1}, -C(O)R^{b1}, -OC(O)R^{b1}, -C(O)OR^{b1}, -S(O)R^{b1}, -S(O)NR^{c1}R^{d1}, -N(R^{c1})S(O)R^{d1}, -S(O)₂R^{b1}, -S(O)₂NR^{c1}R^{d1}, -N(R^{c1})S(O)₂R^{d1}, -OC(O)NR^{c1}R^{d1}, -N(R^{c1})C(O)R^{d1}, and -C(O)NR^{c1}R^{d1}, wherein the C₁₋₆ alkyl, C₂₋₆ alkenyl, C₂₋₆ alkynyl, -C₁₋₆ alkylene-C₃₋₁₀ cycloalkyl, -C₁₋₆ alkylene-3- to 10-membered heterocycloalkyl, -C₁₋₆ alkylene-C₆₋₁₀ aryl, or -C₁₋₆ alkylene-5- to 10-membered heteroaryl is optionally substituted with one or more -OH, -CN, -NH₂, halogen, C₁₋₆ alkyl, or C₁₋₆ heteroalkyl;
or, R¹ and R² are taken together to form =O, =S, or =CR^{c2}R^{d2}; and/or, R³ and R⁴ are taken together to form =O, =S, or =CR^{c2}R^{d2};
R^{b1}, R^{c1}, and R^{d1} are each independently selected from the group consisting of hydrogen, C₁₋₆ alkyl, C₂₋₆ alkenyl, C₂₋₆ alkynyl, C₁₋₆ heteroalkyl, C₃₋₁₀ cycloalkyl, 3- to 10-membered heterocycloalkyl, C₆₋₁₀ aryl, and 5- to 10-membered heteroaryl, wherein the C₁₋₆ alkyl, C₂₋₆ alkenyl, C₂₋₆ alkynyl, C₃₋₁₀ cycloalkyl, 3- to 10-membered heterocycloalkyl, C₆₋₁₀ aryl, or 5- to 10-membered heteroaryl is optionally substituted with one or more halogen, -CN, -OH, -NH₂, C₁₋₆ alkyl, or C₁₋₆ heteroalkyl;
R^{c2} and R^{d2} are each independently selected from the group consisting of hydrogen, C₁₋₆ alkyl, C₂₋₆ alkenyl, C₂₋₆ alkynyl, and C₁₋₆ heteroalkyl, wherein the C₁₋₆ alkyl, C₂₋₆ alkenyl, C₂₋₆ alkynyl, or C₁₋₆ heteroalkyl is optionally substituted with one or more halogen, -CN, -OH, or -NH₂;
Y is selected from the group consisting of O, S, CR^{x}R^{y}, and NR^{z}, wherein R^{x}, R^{y}, or R^{z} is each independently selected from the group consisting of hydrogen and C₁₋₆ alkyl;
W, W¹, W², W³, W⁴, and W⁵ are each independently selected from the group consisting of N, C, and CH;
ring A is selected from the group consisting of the following groups optionally substituted with one or more R^{a1}: C₃₋₁₂ cycloalkylene, 3- to 12-membered heterocyclylene, C₆₋₁₀ arylene, and 5- to 12-membered heteroarylene;
each R^{a1} is independently selected from the group consisting of halogen, -OH, -CN, -NH₂, =O, =S, C₁₋₆ alkyl, C₂₋₆ alkenyl, C₂₋₆ alkynyl, and C₁₋₆ heteroalkyl;
R⁵ is selected from the group consisting of halogen, -OH, -CN, -NH₂, and the following groups optionally substituted with one or more R^{a"}: -C(O)NR^{a'}R^{b'}, -N(R^{b'})C(O)R^{b'}, -N(R^{b'})C(O)OR^{b'}, -OC(O)NR^{b'}R^{b'}, -C(O)R^{b'}, -S(O)R^{b'}, -S(O)₂R^{b'}, -N(R^{b'})S(O)₂R^{b'}, -N(R^{b'})S(O)R^{b'}, -S(O)NR^{b'}R^{b'}, -S(O)₂NR^{b'}R^{b'}, -OC(O)R^{b'}, -C(O)OR^{b'}, C₃₋₁₀ cycloalkyl, 3- to 10-membered heterocycloalkyl, C₆₋₁₀ aryl, and 5- to 10-membered heteroaryl;
R^{a'} is selected from the group consisting of hydrogen, C₁₋₆ alkyl, C₂₋₆ alkenyl, C₂₋₆ alkynyl, C₃₋₆ cycloalkyl, and C₃₋₆ heterocycloalkyl;
R^{b'} is selected from the group consisting of hydrogen, C₁₋₆ alkyl, C₂₋₆ alkenyl, C₂₋₆ alkynyl, C₃₋₁₀ cycloalkyl, 3- to 10-membered heterocycloalkyl, C₆₋₁₀ aryl, and 5- to 10-membered heteroaryl, wherein the C₁₋₆ alkyl, C₃₋₁₀ cycloalkyl, 3- to 10-membered heterocycloalkyl, C₆₋₁₀ aryl, or 5- to 10-membered heteroaryl is optionally substituted with one or more halogen, -CN, -OH, or -NH₂;
each R^{a"} is independently selected from the group consisting of halogen, -OH, -CN, -NH₂, -NH(C₁₋₆ alkyl), -N(C₁₋₆ alkyl)₂, C₁₋₆ alkyl, C₂₋₆ alkenyl, C₂₋₆ alkynyl, C₁₋₆ alkyl-O-, C₃₋₁₀ cycloalkyl, 3- to 10-membered heterocycloalkyl, C₆₋₁₀ aryl, and 5- to 10-membered heteroaryl, wherein the -NH(C₁₋₆ alkyl), -N(C₁₋₆ alkyl)₂, C₁₋₆ alkyl, C₂₋₆ alkenyl, C₂₋₆ alkynyl, C₁₋₆ alkyl-O-, C₃₋₁₀ cycloalkyl, 3- to 10-membered heterocycloalkyl, C₆₋₁₀ aryl, or 5- to 10-membered heteroaryl is optionally substituted with one or more halogen, -OH, -CN, or -NH₂;
R⁶ is selected from the group consisting of hydrogen, halogen, -OH, -CN, -NH₂, C₁₋₆ alkyl, C₂₋₆ alkenyl, C₂₋₆ alkynyl, C₁₋₆ heteroalkyl, C₃₋₁₀ cycloalkyl, 3- to 10-membered heterocycloalkyl, C₆₋₁₀ aryl, and 5- to 10-membered heteroaryl, wherein the C₁₋₆ alkyl, C₂₋₆ alkenyl, C₂₋₆ alkynyl, C₁₋₆ heteroalkyl, C₃₋₁₀ cycloalkyl, 3- to 10-membered heterocycloalkyl, C₆₋₁₀ aryl, or 5- to 10-membered heteroaryl is optionally substituted with one or more halogen, - CN, -OH, -NH₂, C₁₋₆ alkyl, C₁₋₆ alkyl-O-, C₂₋₆ alkenyl, C₂₋₆ alkynyl, C₃₋₁₀ cycloalkyl, or 3- to 10-membered heterocycloalkyl;
R^{a} and R⁷ are each independently selected from the group consisting of halogen, -CN, -OH, -NH₂, C₁₋₆ alkyl, C₂₋₆ alkenyl, C₂₋₆ alkynyl, and -OC₁₋₆ alkyl, wherein the C₁₋₆ alkyl, C₂₋₆ alkenyl, C₂₋₆ alkynyl, or -OC₁₋₆ alkyl is optionally substituted with one or more halogen, -CN, -OH, or -NH₂;
M is selected from the group consisting of -C(O)- and -(CR^{u}R^{v})_{q}-; R^{u} and R^{v} are each independently selected from the group consisting of hydrogen, halogen, -CN, -OH, -NH₂, C₁₋₆ alkyl, C₂₋₆ alkenyl, C₂₋₆ alkynyl, -OC₁₋₆ alkyl, C₃₋₁₀ cycloalkyl, and 3- to 10-membered heterocycloalkyl;
n is selected from the group consisting of 1, 2, 3, and 4;
m and p are each independently selected from the group consisting of 0, 1, 2, and 3;
q is selected from the group consisting of 1, 2, and 3;
provided that R⁵ is not selected from

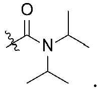

In another aspect, the present disclosure relates to a compound of formula (I) or a pharmaceutically acceptable salt thereof: 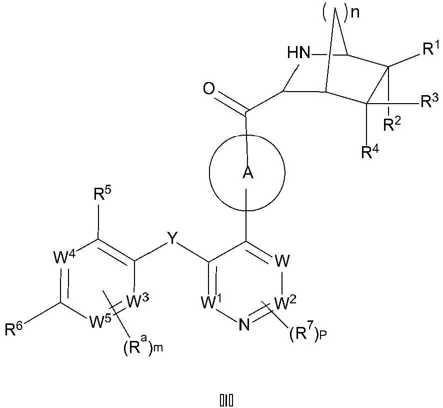 wherein
R¹, R², R³, and R⁴ are each independently selected from the group consisting of hydrogen, halogen, -OH, -CN, C₁₋₆ alkyl, C₂₋₆ alkenyl, C₂₋₆ alkynyl, -C₁₋₆ alkylene-C₃₋₁₀ cycloalkyl, -C₁₋₆ alkylene-3- to 10-membered heterocycloalkyl, -C₁₋₆ alkylene-C₆₋₁₀ aryl, -C₁₋₆ alkylene-5- to 10-membered heteroaryl, -OR^{b1}, -SR^{b1}, -NR^{c1}R^{d1}, -C(O)R^{b1}, -OC(O)R^{b1}, -C(O)OR^{b1}, -S(O)R^{b1}, -S(O)NR^{c1}R^{d1}, -N(R^{c1})S(O)R^{d1}, -S(O)₂R^{b1}, -S(O)₂NR^{c1}R^{d1}, -N(R^{c1})S(O)₂R^{d1}, -OC(O)NR^{c1}R^{d1}, -N(R^{c1})C(O)R^{d1}, and -C(O)NR^{c1}R^{d1}, wherein the C₁₋₆ alkyl, C₂₋₆ alkenyl, C₂₋₆ alkynyl, -C₁₋₆ alkylene-C₃₋₁₀ cycloalkyl, -C₁₋₆ alkylene-3- to 10-membered heterocycloalkyl, -C₁₋₆ alkylene-C₆₋₁₀ aryl, or -C₁₋₆ alkylene-5- to 10-membered heteroaryl is optionally substituted with one or more -OH, -CN, -NH₂, halogen, C₁₋₆ alkyl, or C₁₋₆ heteroalkyl;
or, R¹ and R² are taken together to form =O, =S, or =CR^{c2}R^{d2}; and/or, R³ and R⁴ are taken together to form =O, =S, or =CR^{c2}R^{d2};
R^{b1}, R^{c1}, and R^{d1} are each independently selected from the group consisting of hydrogen, C₁₋₆ alkyl, C₂₋₆ alkenyl, C₂₋₆ alkynyl, C₃₋₁₀ cycloalkyl, 3- to 10-membered heterocycloalkyl, C₆₋₁₀ aryl, and 5- to 10-membered heteroaryl, wherein the C₁₋₆ alkyl, C₂₋₆ alkenyl, C₂₋₆ alkynyl, C₃₋₁₀ cycloalkyl, 3- to 10-membered heterocycloalkyl, C₆₋₁₀ aryl, or 5- to 10-membered heteroaryl is optionally substituted with one or more halogen, -CN, -OH, -NH₂, C₁₋₆ alkyl, or C₁₋₆ heteroalkyl;
R^{c2} and R^{d2} are each independently selected from the group consisting of hydrogen, C₁₋₆ alkyl, C₂₋₆ alkenyl, C₂₋₆ alkynyl, and C₁₋₆ heteroalkyl, wherein the C₁₋₆ alkyl, C₂₋₆ alkenyl, C₂₋₆ alkynyl, or C₁₋₆ heteroalkyl is optionally substituted with one or more halogen, -CN, -OH, or -NH₂;
Y is selected from the group consisting of O, S, CR^{x}R^{y}, and NR^{z}, wherein R^{x}, R^{y}, or R^{z} is each independently selected from the group consisting of hydrogen and C₁₋₆ alkyl;
W, W¹, W², W³, W⁴, and W⁵ are each independently selected from the group consisting of N, C, and CH;
ring A is selected from the group consisting of the following groups optionally substituted with one or more R^{a1}: C₃₋₁₂ cycloalkylene, 3- to 12-membered heterocyclylene, C₆₋₁₀ arylene, and 5- to 12-membered heteroarylene;
each R^{a1} is independently selected from the group consisting of halogen, -OH, -CN, -NH₂, =O, =S, C₁₋₆ alkyl, C₂₋₆ alkenyl, C₂₋₆ alkynyl, and C₁₋₆ heteroalkyl;
R⁵ is selected from the group consisting of halogen, -OH, -CN, -NH₂, and the following groups optionally substituted with one or more R^{a"}: -C(O)NR^{a'}R^{b'}, -N(R^{b'})C(O)R^{b'}, -N(R^{b'})C(O)OR^{b'}, -OC(O)NR^{b'}R^{b'}, -C(O)R^{b'}, -S(O)R^{b'}, -S(O)₂R^{b'}, -N(R^{b'})S(O)₂R^{b'}, -N(R^{b'})S(O)R^{b'}, -S(O)NR^{b'}R^{b'}, -S(O)₂NR^{b'}R^{b'}, -OC(O)R^{b'}, -C(O)OR^{b'}, C₃₋₁₀ cycloalkyl, 3- to 10-membered heterocycloalkyl, C₆₋₁₀ aryl, and 5- to 10-membered heteroaryl;
R^{a'} is selected from the group consisting of hydrogen, C₁₋₆ alkyl, C₂₋₆ alkenyl, C₂₋₆ alkynyl, C₃₋₆ cycloalkyl, and C₃₋₆ heterocycloalkyl;
R^{b'} is selected from the group consisting of hydrogen, C₁₋₆ alkyl, C₂₋₆ alkenyl, C₂₋₆ alkynyl, C₃₋₁₀ cycloalkyl, 3- to 10-membered heterocycloalkyl, C₆₋₁₀ aryl, and 5- to 10-membered heteroaryl, wherein the C₁₋₆ alkyl, C₃₋₁₀ cycloalkyl, 3- to 10-membered heterocycloalkyl, C₆₋₁₀ aryl, or 5- to 10-membered heteroaryl is optionally substituted with one or more halogen, -CN, -OH, or -NH₂;
each R^{a"} is independently selected from the group consisting of halogen, -OH, -CN, -NH₂, -NH(C₁₋₆ alkyl), -N(C₁₋₆ alkyl)₂, C₁₋₆ alkyl, C₂₋₆ alkenyl, C₂₋₆ alkynyl, C₁₋₆ alkyl-O-, C₃₋₁₀ cycloalkyl, 3- to 10-membered heterocycloalkyl, C₆₋₁₀ aryl, and 5- to 10-membered heteroaryl, wherein the -NH(C₁₋₆ alkyl), -N(C₁₋₆ alkyl)₂, C₁₋₆ alkyl, C₂₋₆ alkenyl, C₂₋₆ alkynyl, C₁₋₆ alkyl-O-, C₃₋₁₀ cycloalkyl, 3- to 10-membered heterocycloalkyl, C₆₋₁₀ aryl, or 5- to 10-membered heteroaryl is optionally substituted with one or more halogen, -OH, -CN, or -NH₂;
R⁶ is selected from the group consisting of hydrogen, halogen, -OH, -CN, -NH₂, C₁₋₆ alkyl, C₂₋₆ alkenyl, C₂₋₆ alkynyl, and C₁₋₆ heteroalkyl, wherein the C₁₋₆ alkyl, C₂₋₆ alkenyl, C₂₋₆ alkynyl, or C₁₋₆ heteroalkyl is optionally substituted with one or more halogen, -CN, -OH, or -NH₂;
R^{a} and R⁷ are each independently selected from the group consisting of halogen, -CN, -OH, -NH₂, C₁₋₆ alkyl, C₂₋₆ alkenyl, C₂₋₆ alkynyl, and -OC₁₋₆ alkyl, wherein the C₁₋₆ alkyl, C₂₋₆ alkenyl, C₂₋₆ alkynyl, or -OC₁₋₆ alkyl is optionally substituted with one or more halogen, -CN, -OH, or -NH₂;
n is selected from the group consisting of 1, 2, 3, and 4;
m and p are each independently selected from the group consisting of 0, 1, 2, and 3;
provided that R⁵ is not selected from

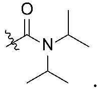

In some embodiments, R¹, R², R³, and R⁴ are each independently selected from the group consisting of hydrogen, halogen, -OH, -CN, -NH₂, C₁₋₄ alkyl, C₂₋₄ alkenyl, C₂₋₄ alkynyl, -C₁₋₄ alkylene-C₃₋₆ cycloalkyl, -C₁₋₄ alkylene-3- to 6-membered heterocycloalkyl, -C₁₋₄ alkylene-phenyl, -C₁₋₄ alkylene-5- to 6-membered heteroaryl, -OR^{b1}, -SR^{b1}, - NR^{c1}R^{d1}, -C(O)R^{b1}, -OC(O)R^{b1}, -C(O)OR^{b1}, -S(O)R^{b1}, -S(O)NR^{c1}R^{d1}, -N(R^{c1})S(O)R^{d1}, -S(O)₂R^{b1}, -S(O)2NR^{c1}R^{d1}, - N(R^{c1})S(O)₂R^{d1}, -OC(O)NR^{c1}R^{d1}, -N(R^{c1})C(O)R^{d1}, and -C(O)NR^{c1}R^{d1}, wherein the C₁₋₄ alkyl, C₂₋₄ alkenyl, C₂₋₄ alkynyl, -C₁₋₄ alkylene-C₃₋₆ cycloalkyl, -C₁₋₄ alkylene-3- to 6-membered heterocycloalkyl, -C₁₋₄ alkylene-phenyl, or -C₁₋₄ alkylene-5- to 6-membered heteroaryl is optionally substituted with one or more -OH, -CN, -NH₂, halogen, C₁₋₆ alkyl, or -C₁₋₆ heteroalkyl;
or, R¹ and R² are taken together to form =O, =S, or =CR^{c2}R^{d2}; and/or, R³ and R⁴ are taken together to form =O, =S, or =CR^{c2}R^{d2};
R^{b1}, R^{c1}, R^{d1}, R^{c2}, and R^{d2} are as defined in the present disclosure.

In some embodiments, R¹, R², R³, and R⁴ are each independently selected from the group consisting of hydrogen, halogen, -OH, -CN, -NH₂, C₁₋₄ alkyl, C₂₋₄ alkenyl, C₂₋₄ alkynyl, -C₁₋₄ alkylene-C₃₋₆ cycloalkyl, -C₁₋₄ alkylene-3- to 6-membered heterocycloalkyl, -C₁₋₄ alkylene-phenyl, -C₁₋₄ alkylene-5- to 6-membered heteroaryl, -OR^{b1}, -SR^{b1}, - NR^{c1}R^{d1}, -C(O)R^{b1}, -OC(O)R^{b1}, -S(O)R^{b1}, -S(O)₂R^{b1}, -OC(O)NR^{c1}R^{d1}, -N(R^{c1})C(O)R^{d1}, and -C(O)NR^{c1}R^{d1}, wherein the C₁₋₄ alkyl, C₂₋₄ alkenyl, C₂₋₄ alkynyl, -C₁₋₄ alkylene-C₃₋₆ cycloalkyl, -C₁₋₄ alkylene-3- to 6-membered heterocycloalkyl, -C₁₋₄ alkylene-phenyl, or -C₁₋₄ alkylene-5- to 6-membered heteroaryl is optionally substituted with one or more halogen, OH, -CN, -NH₂, C₁₋₄ alkyl, or -OC₁₋₄ alkyl;
or, R¹ and R² are taken together to form =O or =CR^{c2}R^{d2}; and/or, R³ and R⁴ are taken together to form =O or =CR^{c2}R^{d2}; R^{b1}, R^{c1}, R^{d1}, R^{c2}, and R^{d2} are as defined in the present disclosure.

In some embodiments, R¹, R², R³, and R⁴ are each independently selected from the group consisting of hydrogen, halogen, -OH, -CN, C₁₋₄ alkyl, C₂₋₄ alkenyl, C₂₋₄ alkynyl, -C₁₋₄ alkylene-C₃₋₆ cycloalkyl, -C₁₋₄ alkylene-3- to 6-membered heterocycloalkyl, -C₁₋₄ alkylene-phenyl, -C₁₋₄ alkylene-5- to 6-membered heteroaryl, -OR^{b1}, -SR^{b1}, - NR^{c1}R^{d1}, -OC(O)R^{b1}, -S(O)R^{b1}, -S(O)₂R^{b1}, and -OC(O)NR^{c1}R^{d1}, wherein the C₁₋₄ alkyl, C₂₋₄ alkenyl, C₂₋₄ alkynyl, - C₁₋₄ alkylene-C₃₋₆ cycloalkyl, -C₁₋₄ alkylene-3- to 6-membered heterocycloalkyl, -C₁₋₄ alkylene-phenyl, or -C₁₋₄ alkylene-5- to 6-membered heteroaryl is optionally substituted with one or more halogen, OH, -CN, -NH₂, C₁₋₃ alkyl, or -OC₁₋₃ alkyl;
or, R¹ and R² are taken together to form =O or =CR^{c2}R^{d2}; and/or, R³ and R⁴ are taken together to form =O or =CR^{c2}R^{d2}; R^{b1}, R^{c1}, R^{d1}, R^{c2}, and R^{d2} are as defined in the present disclosure.

In some embodiments, R¹, R², R³, and R⁴ are each independently selected from the group consisting of hydrogen, halogen, -OH, -CN, -NH₂, C₁₋₃ alkyl, C₂₋₃ alkenyl, C₂₋₃ alkynyl, -CH₂-C₃₋₆ cycloalkyl, -CH₂-3- to 6-membered heterocycloalkyl, -CH₂-phenyl, and -CH₂-5- to 6-membered heteroaryl, wherein the C₁₋₃ alkyl, C₂₋₃ alkenyl, C₂₋₃ alkynyl, -CH₂-C₃₋₆ cycloalkyl, -CH₂-3- to 6-membered heterocycloalkyl, -CH₂-phenyl, or -CH₂-5- to 6-membered heteroaryl is optionally substituted with one or more halogen, OH, -CN, -NH₂, or C₁₋₄ alkyl;
or, R¹ and R² are taken together to form =O or =CH₂; and/or, R³ and R⁴ are taken together to form =O or =CH₂.

In some embodiments, R¹, R², R³, and R⁴ are each independently selected from the group consisting of hydrogen, halogen, -OH, -CN, -NH₂, and C₁₋₃ alkyl.

In some embodiments, R¹, R², R³, and R⁴ are each independently selected from the group consisting of hydrogen, halogen, -OH, -CN, -NH₂, and C₁₋₃ alkyl;
or, R¹ and R² are taken together to form =O or =CH₂; and/or, R³ and R⁴ are taken together to form =O or =CH₂.

In some embodiments, R¹ and R² are taken together to form =O or =CH₂; and/or, R³ and R⁴ are taken together to form =O or =CH₂.

In some embodiments, any one of R³ and R⁴ is hydrogen, and the other is F.

In some embodiments, R¹ and R² are hydrogen; and, one of R³ and R⁴ is hydrogen, and the other is F; or R³ and R⁴ are taken together to form =O or =CH₂. In some embodiments, R¹ and R² are hydrogen; and, R³ and R⁴ are taken together to form =CH₂.

In some embodiments, R³ and R⁴ are hydrogen, and R¹ and R² are taken together to form =O or =CH₂.

In some embodiments, R¹ and R² are hydrogen, and R³ and R⁴ are taken together to form =O or =CH₂.

In some specific embodiments, R¹ and R² are hydrogen; and, one of R³ and R⁴ is hydrogen, and the other is F.

In some embodiments, R^{b1}, R^{c1}, and R^{d1} are each independently selected from the group consisting of hydrogen, C₁₋₄ alkyl, C₂₋₄ alkenyl, C₂₋₄ alkynyl, C₃₋₆ cycloalkyl, 3- to 6-membered heterocycloalkyl, phenyl, and 5- to 6-membered heteroaryl, wherein the C₁₋₆ alkyl, C₂₋₆ alkenyl, C₂₋₆ alkynyl, C₃₋₆ cycloalkyl, 3- to 6-membered heterocycloalkyl, phenyl, or 5- to 6-membered heteroaryl is optionally substituted with one or more halogen, -CN, -OH, -NH₂, C₁₋₄ alkyl, or C₁₋₄ heteroalkyl.

In some embodiments, R^{b1}, R^{c1}, and R^{d1} are each independently selected from the group consisting of hydrogen, C₁₋₄ alkyl, C₂₋₄ alkenyl, and C₂₋₄ alkynyl, wherein the C₁₋₄ alkyl, C₂₋₄ alkenyl, or C₂₋₄ alkynyl is optionally substituted with one or more halogen, -CN, -OH, or -NH₂.

In some embodiments, R^{b1}, R^{c1}, and R^{d1} are each independently selected from the group consisting of hydrogen and C₁₋₃ alkyl, wherein the C₁₋₃ alkyl is optionally substituted with one or more halogen, -CN, -OH, or -NH₂.

In some embodiments, R^{b1}, R^{c1}, and R^{d1} are each independently hydrogen or C₁₋₃ alkyl.

In some embodiments, R^{b1}, R^{c1}, and R^{d1} are each independently methyl.

In some embodiments, R^{c2} and R^{d2} are each independently selected from the group consisting of hydrogen, C₁₋₄ alkyl, C₂₋₄ alkenyl, C₂₋₄ alkynyl, and C₁₋₄ heteroalkyl, wherein the C₁₋₄ alkyl, C₂₋₄ alkenyl, C₂₋₄ alkynyl, or C₁₋₄ heteroalkyl is optionally substituted with one or more halogen, -CN, -OH, or -NH₂.

In some embodiments, R^{c2} and R^{d2} are each independently selected from the group consisting of hydrogen, C₁₋₄ alkyl, C₂₋₄ alkenyl, C₂₋₄ alkynyl, and C₁₋₄ heteroalkyl.

In some embodiments, R^{c2} and R^{d2} are each independently selected from the group consisting of hydrogen and C₁₋₃ alkyl.

In some embodiments, R^{c2} and R^{d2} are each independently hydrogen.

In some embodiments, R⁸ is selected from the group consisting of hydrogen and C₁₋₄ alkyl. In some embodiments, R⁸ is selected from the group consisting of hydrogen and methyl. In some embodiments, R⁸ is hydrogen.

In some embodiments, Y is selected from the group consisting of O, S, CR^{x}R^{y}, and NR^{z}, wherein R^{x}, R^{y}, or R^{z} is each independently selected from the group consisting of hydrogen and C₁₋₃ alkyl.

In some embodiments, Y is selected from the group consisting of O, S, CR^{x}R^{y}, and NR^{z}, wherein R^{X}, R^{y}, or R^{z} is each independently selected from the group consisting of hydrogen and methyl.

In some embodiments, Y is selected from the group consisting of O, S, CH₂, and NH.

In some embodiments, Y is selected from the group consisting of O and S.

In some embodiments, Y is O.

In some embodiments, in the structural fragment 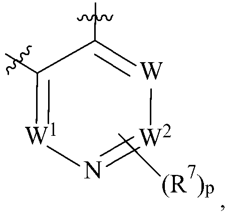 when W, W¹, and/or W² are attached to R⁷, W, W¹, and/or W² are C; when W, W¹, and/or W² are not attached to R⁷, W, W¹, and/or W² are N or CH.

In some embodiments, in the structural fragment 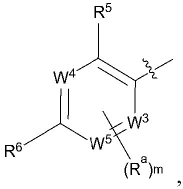 when W³, W⁴, and/or W⁵ are attached to R^{a}, W³, W⁴, and/or W⁵ are C; when W³, W⁴, and/or W⁵ are not attached to R², W³, W⁴, and/or W⁵ are N or CH.

In some embodiments, W is selected from the group consisting of N, C, and CH. In some embodiments, W is selected from the group consisting of N and CH.

In some embodiments, W¹ is selected from the group consisting of N, C, and CH. In some embodiments, W¹ is selected from the group consisting of N and CH.

In some embodiments, W² is selected from the group consisting of N, C, and CH. In some embodiments, W² is CH.

In some embodiments, W³ is selected from the group consisting of N and CH.

In some embodiments, W⁴ and W⁵ are CH.

In some embodiments, W³, W⁴, and W⁵ are CH.

In some embodiments, W¹, W², W³, W⁴, and W⁵ are CH. In some embodiments, W is N, and W¹, W², W³, W⁴, and W⁵ are each independently selected from the group consisting of C and CH.

In some embodiments, W¹ is N, and W, W², W³, W⁴, and W⁵ are each independently selected from the group consisting of C and CH.

In some embodiments, W and W¹ are N, and W², W³, W⁴, and W⁵ are each independently selected from the group consisting of C and CH.

In some embodiments, W and W³ are each independently selected from the group consisting of N and CH, and W¹, W², W³, W⁴, and W⁵ are CH.

In some embodiments, W, W¹, and W³ are each independently selected from the group consisting of CH and N, and W², W⁴, and W⁵ are CH.

In some embodiments, W is N, and W¹ and W² are CH. In some embodiments, W and W¹ are N, and W² is CH or C.

In some embodiments, W and W² are CH, and W¹ is N.

In some embodiments, W⁴ is CH or C, and W³ and W⁵ are selected from the group consisting of N, C, and CH.

In some embodiments, when 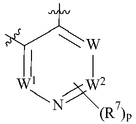 is 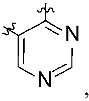 R⁵ is not.

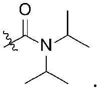

In some embodiments, R⁵ is not

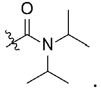

In some embodiments, each R^{a} is independently selected from the group consisting of halogen, CN, OH, NH₂, and C₁₋₃ alkyl.

In some embodiments, R^{a} is fluorine.

In some embodiments, R^{z} is hydrogen.

In some embodiments, the structural unit 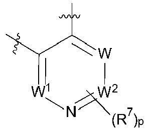 is selected from the group consisting of 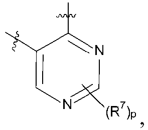 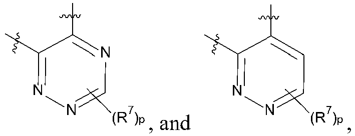 wherein W, W¹, W², R⁷, and p are as defined in the present disclosure.

In some embodiments, the structural unit 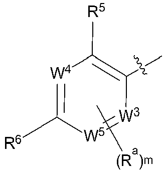 is selected from the group consisting of 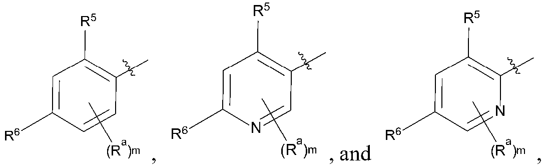 wherein W³, W⁴, W⁵, R⁵, R⁶, R^{a}, and m are as defined in the present disclosure. In some embodiments, the structural unit 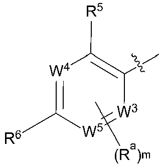 is selected from the group consisting of 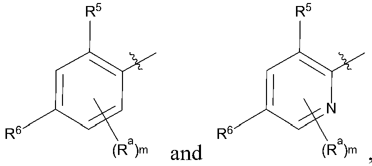 , wherein W³, W⁴, W⁵, R⁵, R⁶, R^{a}, and m are as defined in the present disclosure.

In some embodiments, the structural unit 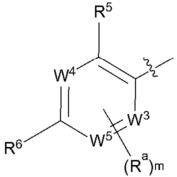 is selected from the group consisting of 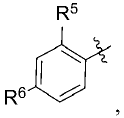 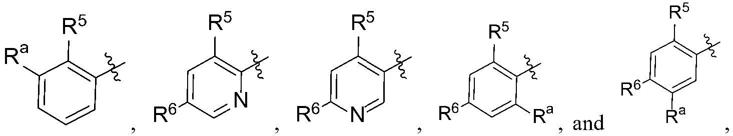 wherein R⁵, R⁶, and R^{a} are as defined in the present disclosure.

In some embodiments, the structural unit 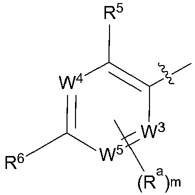 is selected from the group consisting of

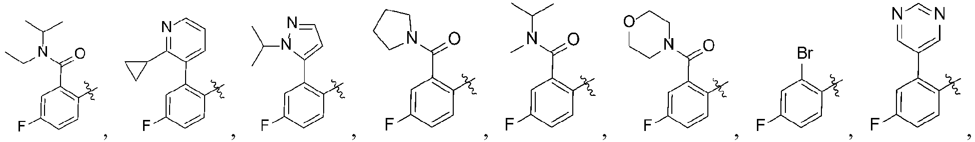

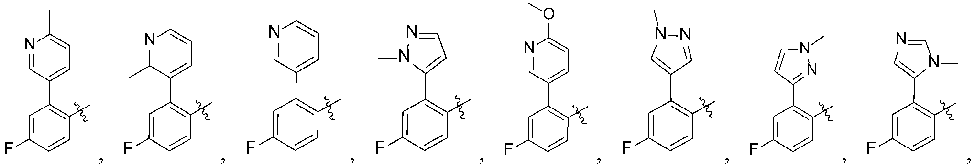

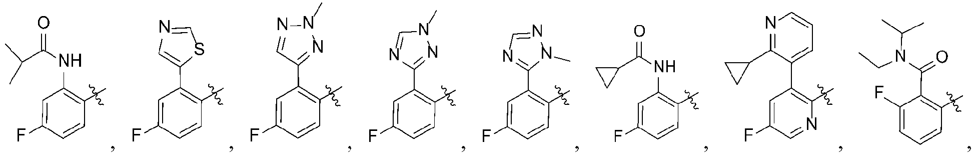

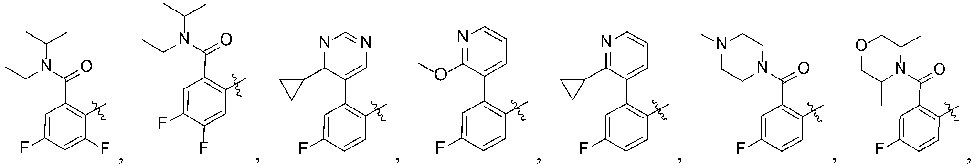

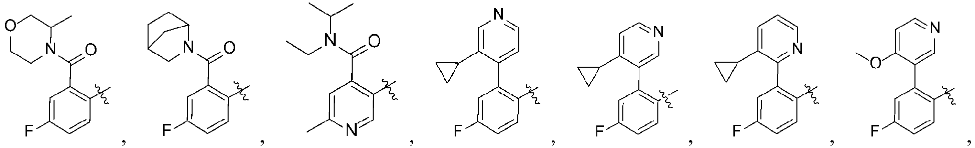

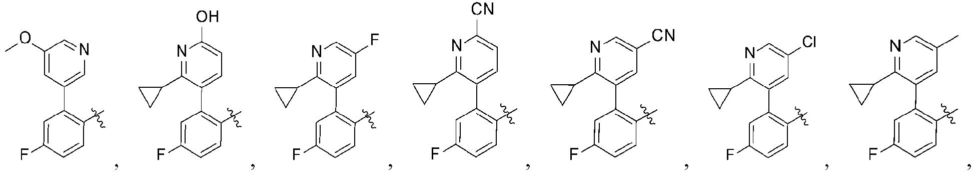

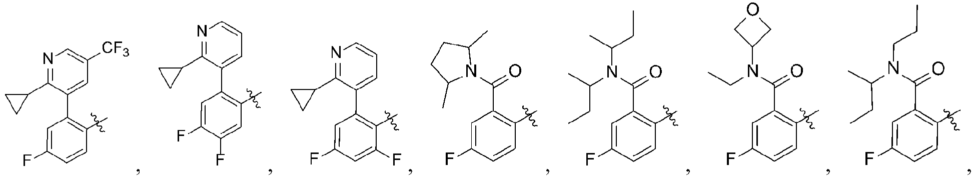

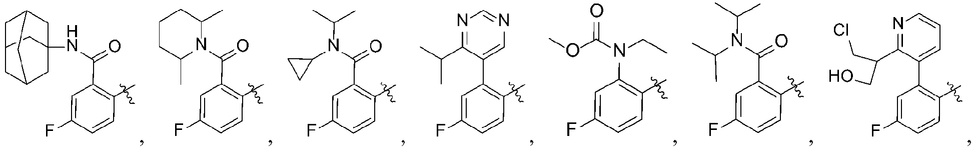

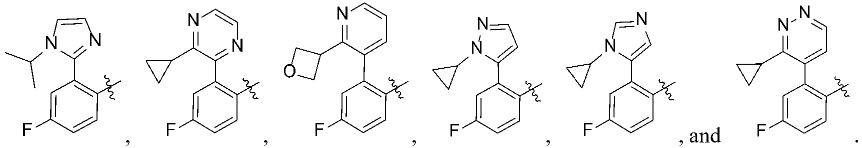

In some embodiments, the structural unit 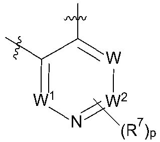 is selected from the group consisting of 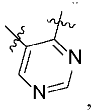 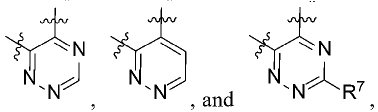 wherein * indicates that a bond at the position is linked to moiety H or ring A (when H is a bond), and the other side is linked to moiety Y, wherein R⁷ is as defined in the present disclosure.

In some embodiments, the structural unit 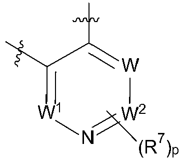 is selected from the group consisting of 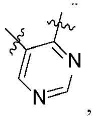 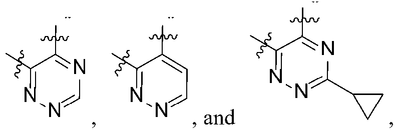 wherein * indicates that a bond at the position is linked to moiety H or ring A (when H is a bond), and the other side is linked to moiety Y.

In some embodiments, ring A is selected from the group consisting of the following groups optionally substituted with one or more R^{a1}: C₅₋₁₂ membered cycloalkylene, 5- to 12-membered heterocyclylene, C₆ arylene, and 5- to 6-membered heteroarylene.

In some embodiments, ring A is selected from the group consisting of the following groups optionally substituted with one or more R^{a1}: C₆₋₁₀ membered cycloalkylene and 6- to 10-membered heterocyclylene.

In some embodiments, ring A is selected from the group consisting of the following groups optionally substituted with one or more R^{a1}: 6-, 7-, 8-, 9-, and 10-membered heterocycloalkylene.

In some embodiments, ring A is selected from 7- to 9-membered heterocycloalkylene optionally substituted with one or more R^{a1}, wherein R^{a1} is as defined in the present application.

In some embodiments, ring A is selected from 7- to 9-membered heterospirocycloalkylene optionally substituted with one or more R^{a1}, wherein R^{a1} is as defined in the present application. In some embodiments, ring A is selected from 9-membered heterospirocycloalkylene optionally substituted with one or more R^{a1}, wherein R^{a1} is as defined in the present application. Optionally, ring A contains 1, 2, or 3 heteroatoms selected from the group consisting of N, O, and S; or, ring A contains 2 heteroatoms selected from the group consisting of N, O, and S; or, ring A contains 2 N atoms. In some embodiments, ring A is selected from 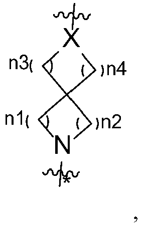 wherein n1, n2, n3, and n4 are each independently selected from the group consisting of 1, 2, and 3, and X is selected from the group consisting of N and CH. In some embodiments, n1, n2, n3, and n4 are each independently selected from the group consisting of 1 and 2.

In some embodiments, ring A is selected from 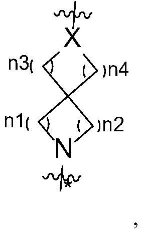 wherein n1, n2, n3, and n4 are each independently selected from the group consisting of 1, 2, and 3, and X is selected from the group consisting of N and CH, wherein * indicates that a bond at the position is linked to the structural fragment 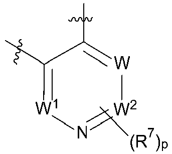 (when H is a bond), and the other side is linked to group M (which represents a linkage to C(O) corresponding to M in the compound of formula (I)). In some embodiments, n1 and n2 are selected from 1, n3 and n4 are selected from 2, and X is selected from N.

In some embodiments, ring A is selected from 7- to 10-membered heterospirocycloalkylene optionally substituted with one or more R^{a1}, wherein R^{a1} is as defined in the present application.

In some embodiments, ring A is selected from 7- to 10-membered heterospirocycloalkylene optionally substituted with one or more R^{a1}, and optionally, ring A contains 1, 2, or 3 heteroatoms selected from the group consisting of N, O, and S; or, ring A contains 1 or 2 heteroatoms selected from the group consisting of N, O, and S; or, ring A contains 1 or 2 N atoms; R^{a1} is as defined in the present application. In some embodiments, ring A is selected from 7- to 9-membered heterospirocycloalkylene (e.g., [3,3]heterospirocycloalkylene or [3,5]heterospirocycloalkylene) optionally substituted with one or more R^{a1}, wherein R^{a1} is selected from the group consisting of =O and C₁₋₆ alkyl; optionally, ring A contains 1 or 2 N atoms.

In some embodiments, ring A is selected from 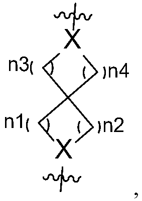 wherein n1, n2, n3, and n4 are each independently selected from the group consisting of 1, 2, and 3 (e.g., n1, n2, n3, and n4 are each independently selected from the group consisting of 1 and 2; or, n1 and n2 are 1, and n3 and n4 are 2), each X is independently selected from the group consisting of N and CH (e.g., one X is N, and the other is CH; or both X are N), and ring A is optionally substituted with 1 =O or methyl.

In some embodiments, ring A is selected from the group consisting of the following groups optionally substituted with one or more R^{a1}:

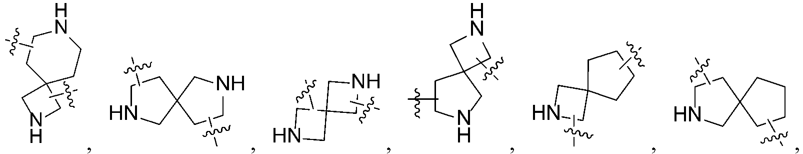

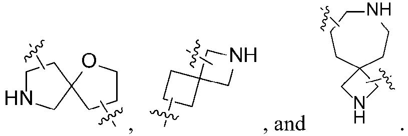

In some embodiments, ring A is selected from the group consisting of the following groups optionally substituted with one or more R^{a1}: 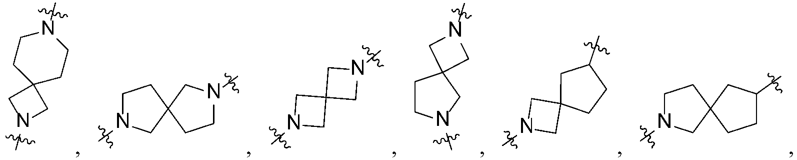 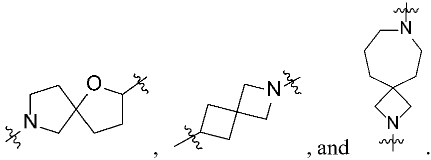 In some embodiments, R^{a1} is selected from the group consisting of =O and C₁₋₆ alkyl. In some embodiments, the structural fragment described above is optionally substituted with 1 =O or methyl.

In some embodiments, ring A is selected from the group consisting of the following groups optionally substituted with one or more R^{a1}: 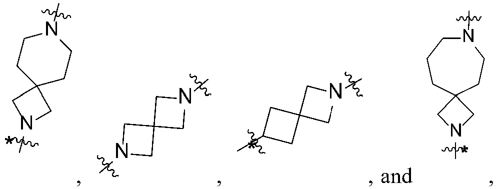 wherein * indicates that a bond at the position is linked to the structural fragment 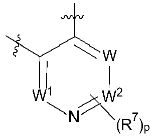 (when H is absent, i.e., when H is a bond), and the other side is linked to M. In some embodiments, R^{a1} is selected from the group consisting of =O and C₁₋₆ alkyl. In some embodiments, the structural fragment described above is optionally substituted with 1 =O or methyl.

In some embodiments, ring A is selected from the group consisting of

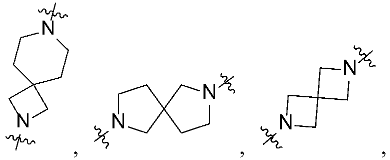

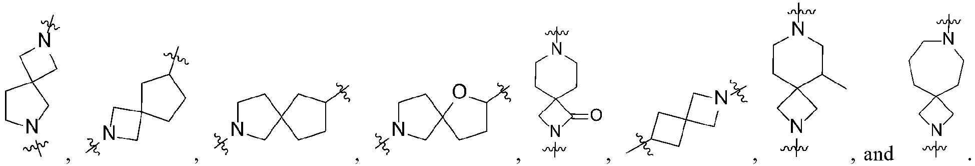

In some embodiments, in the compound of formula (I) or the pharmaceutically acceptable salt thereof, ring A is selected from the group consisting of 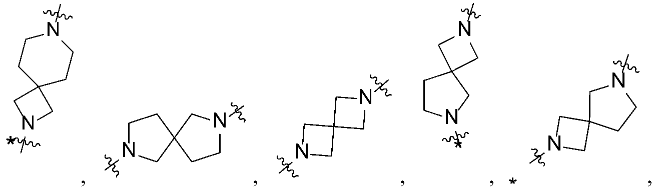 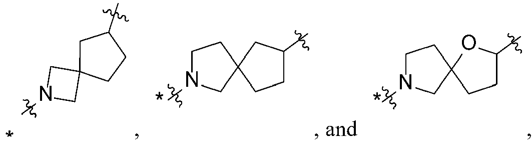 wherein * indicates that a bond at the position is linked to the structural fragment 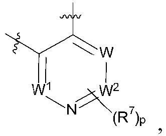 and the other side is linked to the structural fragment 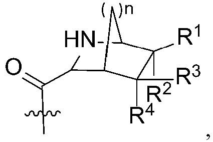 wherein W, W¹, W², n, R¹, R², R³, and R⁴ are as defined in the present disclosure.

In some embodiments, ring A is selected from the group consisting of 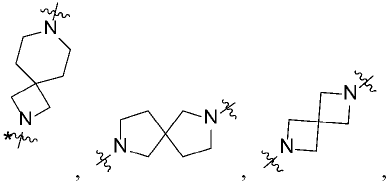 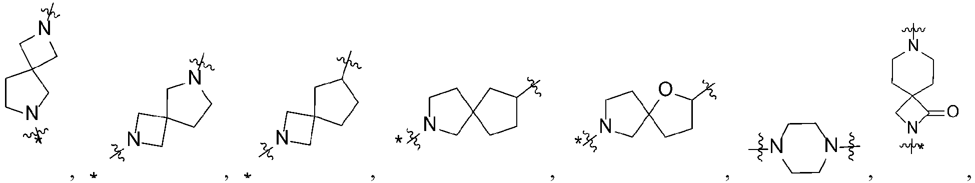 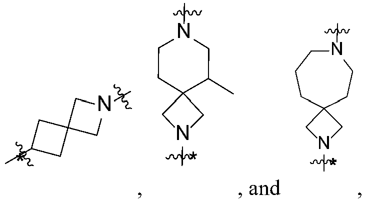 wherein * indicates that a bond at the position is linked to moiety H or the structural fragment 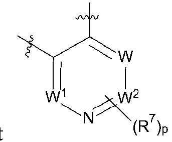 (when H is absent, i.e., when H is a bond), and the other side is linked to moiety M, wherein W, W¹, W², n, R¹, R², R³, R⁴, M, and R⁸ are as defined in the present disclosure.

In some embodiments, ring A is selected from the group consisting of 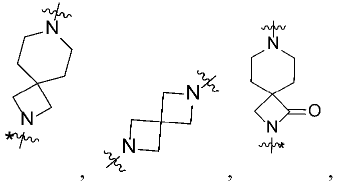 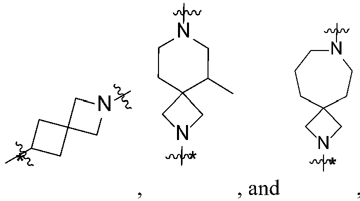 , wherein * indicates that a bond at the position is linked to moiety H or the structural fragment 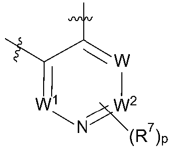 (when H is a bond), and the other side is linked to moiety M.

In some embodiments, ring A is selected from the group consisting of 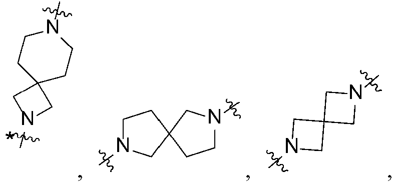 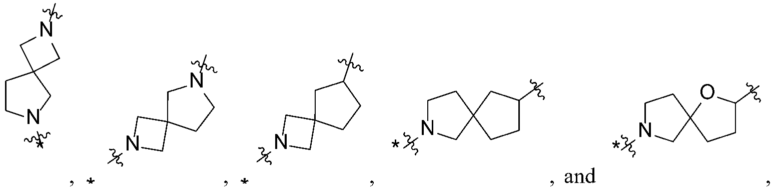 wherein * indicates that a bond at the position is linked to the structural fragment 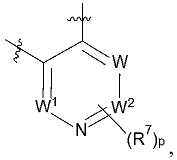 and the other side is linked to the structural fragment 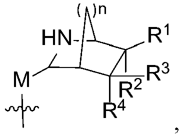 wherein W, W¹, W², n, R¹, R², R³, R⁴, and M are as defined in the present disclosure.

In some embodiments, ring A is selected from 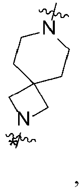 wherein * indicates the same meaning as described above.

In some embodiments, R^{u} and R^{v} are each independently selected from the group consisting of hydrogen, halogen, - CN, -OH, -NH₂, and C₁₋₃ alkyl. In some embodiments, R^{u} and R^{v} are hydrogen.

In some embodiments, M is selected from the group consisting of -CH₂- and -C(O)-. In some embodiments, M is - C(O)-.

In some embodiments, H is a bond; that is, the structural unit 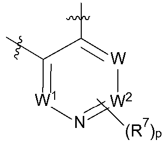 is directly linked to ring A. In some embodiments, H is NH.

In some embodiments, each R^{a1} is independently selected from the group consisting of halogen, -OH, -CN, -NH₂, =O, =S, and C₁₋₆ alkyl.

In some embodiments, each R^{a1} is independently selected from the group consisting of halogen, -OH, -CN, -NH₂, =O, =S, and C₁₋₃ alkyl.

In some embodiments, each R^{a1} is independently selected from the group consisting of =O, halogen, and C₁₋₃ alkyl.

In some embodiments, each R^{a1} is independently selected from the group consisting of =O and C₁₋₃ alkyl.

In some specific embodiments, each R^{a1} is independently selected from the group consisting of =O and methyl.

In some embodiments, R⁵ is selected from the group consisting of halogen, -OH, -CN, -NH₂, and the following groups optionally substituted with one or more R^{a"}: -C(O)NR^{a'}R^{b'}, -N(R^{b'})C(O)R^{b'}, -N(R^{b'})(CO)OR^{b'}, -O(CO)NR^{b'}R^{b'}, - C(O)R^{b'}, -S(O)R^{b'}, -S(O)₂R^{b'}, -N(R^{b'})S(O)₂R^{b'}, -N(R^{b'})S(O)R^{b'}, -S(O)NR^{b'}R^{b'}, -S(O)₂NR^{b'}R^{b'}, -OC(O)R^{b'}, -C(O)OR^{b'}, C₃₋₆ cycloalkyl, 3- to 6-membered heterocycloalkyl, phenyl, and 5- to 6-membered heteroaryl, wherein R^{a'}, R^{b'}, and R^{a"} are as defined in the present disclosure.

In some embodiments, R⁵ is selected from the group consisting of halogen, -OH, -CN, -NH₂, and the following groups optionally substituted with one or more R^{a"}: -C(O)R^{b'}, -C(O)NR^{a'}R^{b'}, -N(R^{b'})C(O)R^{b'}, -N(R^{b'})C(O)OR^{b'}, and 5- to 6-membered heteroaryl, wherein R^{a'}, R^{b'}, and R^{a"} are as defined in the present disclosure.

In some embodiments, R⁵ is selected from the group consisting of halogen and the following groups optionally substituted with one or more R^{a"}: 3- to 10-membered heterocycloalkyl C(O)-, C₃₋₁₀ cycloalkyl NHC(O)-, -C(O)N(C₁₋₆ alkyl)₂, -C(0)N(C₁₋₆ alkyl)C₃₋₁₀ cycloalkyl, -C(O)N(C₁₋₆ alkyl)(5- to 10-membered heterocycloalkyl), -NHC(O)C₁₋₆ alkyl, -NHC(O)C₃₋₁₀ cycloalkyl, -N(C₁₋₆ alkyl)C(O)OC₁₋₃ alkyl, and 5- to 10-membered heteroaryl, wherein R^{a"} is as defined in the present disclosure.

In some embodiments, R⁵ is selected from the group consisting of halogen and the following groups optionally substituted with one or more R^{a"}: 4- to 8-membered heterocycloalkyl C(O)-, C₈₋₁₀ cycloalkyl NHC(O)-, -C(O)N(C₁₋₄ alkyl)₂, -C(O)N(C₁₋₄ alkyl)C₃₋₆ cycloalkyl, -C(O)N(C₁₋₄ alkyl)(4- to 6-membered heterocycloalkyl), -NHC(O)C₁₋₄ alkyl, -NHC(O)C₃₋₆ cycloalkyl, -N(C₁₋₄ alkyl)C(O)OC₁₋₄ alkyl, and 5- to 6-membered heteroaryl, wherein R^{a"} is as defined in the present disclosure.

In some embodiments, R⁵ is selected from the group consisting of halogen, -C(O)R^{b'}, -C(O)NR^{a'}R^{b'}, -N(R^{b'})C(O)OR^{b'}, -N(R^{b'})C(O)R^{b'}, and 5- to 6-membered heteroaryl, wherein the -C(O)NR^{a'}R^{b'}, -C(O)R^{b'}, -N(R^{b'})C(O)OR^{b'}, - N(R^{b'})C(O)R^{b'}, or 5- to 6-membered heteroaryl is optionally substituted with one or more of the following groups: halogen, -OH, -CN, -NH₂, C₁₋₃ alkyl optionally substituted with one or more halogen or -OH, -OC₁₋₃ alkyl, C₃₋₆ cycloalkyl, or 4- to 6-membered heterocycloalkyl, and R^{a'} and R^{b'} are as defined in the present application. In some embodiments, R⁵ is selected from the group consisting of F, Cl, Br, 5- to 7-membered heterocycloalkyl C(O)-, - C(O)N(C₁₋₄ alkyl)₂, C₉₋₁₀ cycloalkyl NHC(O)-, -C(O)N(C₁₋₃ alkyl)C₃₋₅ cycloalkyl, -C(O)N(C₁₋₃ alkyl)(4- to 6-membered heterocycloalkyl), -NHC(O)C₁₋₃ alkyl, -NHC(O)C₃₋₅ cycloalkyl, -N(C₁₋₃ alkyl)C(O)OC₁₋₃ alkyl, and 5- to 6-membered heteroaryl, wherein the 5- to 7-membered heterocycloalkyl C(O)-, -C(O)N(C₁₋₄ alkyl)₂, C₉₋₁₀ cycloalkyl NHC(O)-, -C(O)N(C₁₋₃ alkyl)C₃₋₅ cycloalkyl, -C(O)N(C₁₋₃ alkyl)(4- to 6-membered heterocycloalkyl), -NHC(O)C₁₋₃ alkyl, -NHC(O)C₃₋₅ cycloalkyl, -N(C₁₋₃ alkyl)C(O)OC₁₋₃ alkyl, or 5- to 6-membered heteroaryl is optionally substituted with one or more of the following groups: halogen, -OH, -CN, -NH₂, C₁₋₃ alkyl optionally substituted with one or more halogen or -OH, -OC₁₋₃ alkyl, C₃₋₄ cycloalkyl, or 4- to 5-membered heterocycloalkyl.

In some embodiments, R⁵ is selected from the group consisting of Br, pyrazolyl, imidazolyl, thiazolyl, triazolyl, pyridinyl, pyrimidinyl, pyrazinyl, pyridazinyl, -C(O)N(C₁₋₄ alkyl)₂, -C(O)-pyrrolidinyl, -C(O)-piperidinyl, -C(O)-morpholinyl, -C(O)-piperazinyl, -C(O)azabicycloheptyl, adamantyl NHC(O)-, -C(O)N(C₁₋₃ alkyl)cyclopropyl, - C(O)N(C₁₋₃ alkyl)oxetanyl, -NHC(O)C₁₋₃ alkyl, -NHC(O)-cyclopropyl, and -N(C₁₋₃ alkyl)C(O)OC₁₋₃ alkyl, wherein the pyrazolyl, imidazolyl, thiazolyl, triazolyl, pyridinyl, pyrimidinyl, pyrazinyl, pyridazinyl, -C(O)-pyrrolidinyl, - C(O)-piperidinyl, -C(O)-morpholinyl, or -C(O)-piperazinyl is optionally substituted with 1 or 2 groups selected from the group consisting of F, Cl, -OH, -CN, methyl, -CF₃, -OCH₃, cyclopropyl, isopropyl, 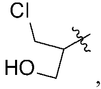 and oxetanyl.

In some other embodiments, R⁵ is selected from the group consisting of F, Cl, Br, -OH, -CN, -NH₂, -C(O)R^{b'}, - C(O)NR^{a'}R^{b'}, -N(R^{b'})C(O)R^{b'}, -N(R^{b'})C(O)OR^{b'}, and 5- to 6-membered heteroaryl, wherein the -C(O)R^{b'}, - C(O)NR^{a'}R^{b'}, -N(R^{b'})C(O)OR^{b'}, -N(R^{b'})C(O)R^{b'}, or 5- to 6-membered heteroaryl is optionally substituted with one or more of the following groups: halogen, -OH, -CN, -NH₂, C₁₋₆ alkyl, -OC₁₋₆ alkyl, C₁₋₆ haloalkyl, C₃₋₆ cycloalkyl, or 3- to 6-membered heterocycloalkyl, and R^{a'} and R^{b'} are as defined in the present disclosure.

In some other embodiments, R⁵ is selected from the group consisting of halogen, 5- to 7-membered heterocycloalkyl C(O)-, C₉₋₁₀ cycloalkyl NHC(O)-, -C(O)N(C₁₋₄ alkyl)₂, -C(O)N(C₁₋₃ alkyl)C₃₋₅ cycloalkyl, -C(O)N(C₁₋₃ alkyl)(5- to 6-membered heterocycloalkyl), -NHC(O)C₁₋₃ alkyl, -NHC(O)C₃₋₅ cycloalkyl, -N(C₁₋₃ alkyl)C(O)OC₁₋₃ alkyl, and 5-to 6-membered heteroaryl, wherein the 5- to 7-membered heterocycloalkyl C(O)-, C₉₋₁₀ cycloalkyl NHC(O)-, - C(O)N(C₁₋₄ alkyl)₂, -C(O)N(C₁₋₃ alkyl)C₃₋₅ cycloalkyl, -C(O)N(C₁₋₃ alkyl)-5- to 6-membered heterocycloalkyl, - NHC(O)C₁₋₃ alkyl, -NHC(O)C₃₋₅ cycloalkyl, -N(C₁₋₃ alkyl)C(O)OC₁₋₃ alkyl, or 5- to 6-membered heteroaryl is optionally substituted with one or more of the following groups: halogen, -OH, -CN, -NH₂, C₁₋₃ alkyl optionally substituted with one or more halogen, -OC₁₋₃ alkyl, or C₃₋₄ cycloalkyl.

In some other embodiments, R⁵ is selected from the group consisting of Br, pyrazolyl, imidazolyl, thiazolyl, triazolyl, pyridinyl, pyrimidinyl, -C(O)-pyrrolidinyl, -C(O)-piperidinyl, -C(O)-morpholinyl, -C(O)-piperazinyl, - C(O)azabicycloheptyl, -C(O)NH-adamantyl, -C(O)N(C₁₋₄ alkyl)₂, -C(O)N(C₁₋₃ alkyl)cyclopropyl, -C(O)N(C₁₋₃ alkyl)oxetanyl, -NHC(O)C₁₋₃ alkyl, -NHC(O)-cyclopropyl, and -N(C₁₋₃ alkyl)C(O)OC₁₋₃ alkyl, wherein the pyrazolyl, imidazolyl, thiazolyl, triazolyl, pyridinyl, pyrimidinyl, -C(O)-pyrrolidinyl, -C(O)-piperidinyl, -C(O)-morpholinyl, - C(O)-piperazinyl, -C(O)azabicycloheptyl, -C(O)N(C₁₋₄ alkyl)₂, -C(O)N(C₁₋₃ alkyl)cyclopropyl, -C(O)N(C₁₋₃ alkyl)oxetanyl, -NHC(O)C₁₋₃ alkyl, -NHC(O)-cyclopropyl, -C(O)NH-adamantyl, or -N(C₁₋₃ alkyl)C(O)OC₁₋₃ alkyl is optionally substituted with one or more groups selected from the group consisting of F, Cl, CH₃, CF₃, -OH, -CN, - OCH₃, isopropyl, and cyclopropyl.

In some specific embodiments, R⁵ is selected from the group consisting of the following groups optionally substituted with one or more R^{a"}: -C(O)NR^{a'}R^{b'} and 5- to 10-membered heteroaryl, wherein R^{a'} and R^{b'} are as defined in the present disclosure. In some specific embodiments, R⁵ is selected from the group consisting of the following groups optionally substituted with one or more R^{a"}: -C(O)NR^{a'}R^{b'} and 5- to 6-membered heteroaryl, wherein R^{a'} and R^{b'} are as defined in the present disclosure. In some specific embodiments, R⁵ is selected from the group consisting of the following groups optionally substituted with one or more R^{a"}: -C(O)N(C₁₋₆ alkyl)₂ and 5- to 6-membered heteroaryl, wherein R^{a'} and R^{b'} are as defined in the present disclosure.

In some specific embodiments, R⁵ is selected from the group consisting of the following groups optionally substituted with one or more R^{a"}: 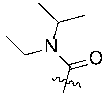 and 5- to 6-membered heteroaryl, wherein R^{a'} and R^{b'} are as defined in the present disclosure. In some specific embodiments, R⁵ is selected from the group consisting of 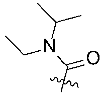 and 5- to 6-membered heteroaryl optionally substituted with one or more R^{a"}, wherein R^{a"} is as defined in the present application.

In some specific embodiments, R⁵ is selected from the group consisting of 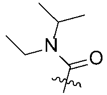 and 5- to 6-membered heteroaryl, wherein the 5- to 6-membered heteroaryl is optionally substituted with one or more of the following groups: halogen, -OH, -CN, -NH₂, C₁₋₆ alkyl optionally substituted with one or more halogen, -OC₁₋₆ alkyl, or C₃₋₆ cycloalkyl.

In some specific embodiments, R⁵ is 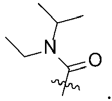 In some specific embodiments, R⁵ is selected from 5- to 6-membered heteroaryl optionally substituted with one or more R^{a"}, wherein R^{a"} is as defined in the present application. In some embodiments, R⁵ is selected from N heteroatom-containing 6-membered heteroaryl optionally substituted with one or more R^{a"}.

In some embodiments, R⁵ is selected from the group consisting of Br, 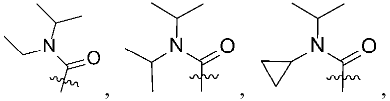 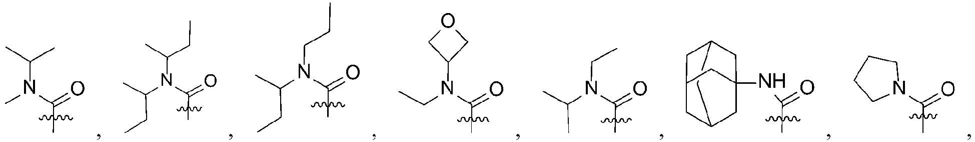 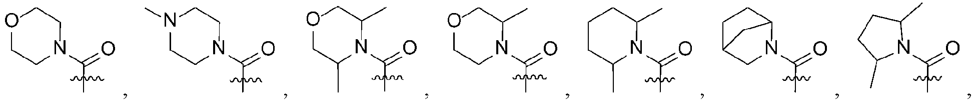 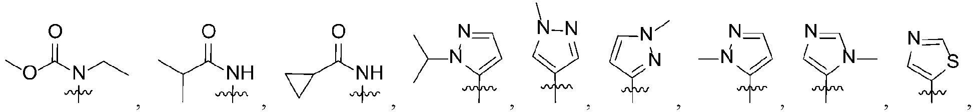 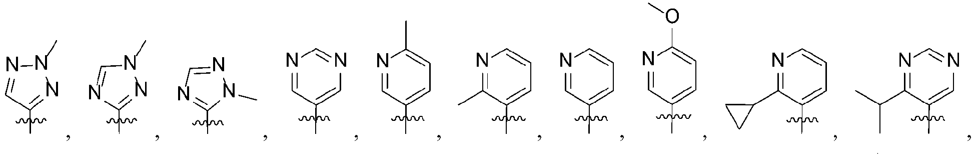 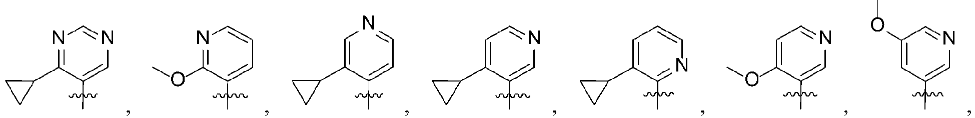 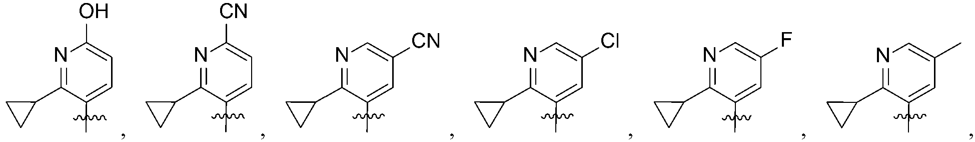 and 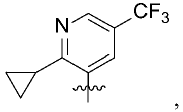 or is selected from the group consisting of

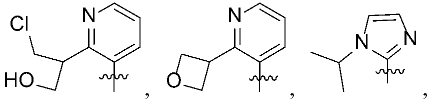

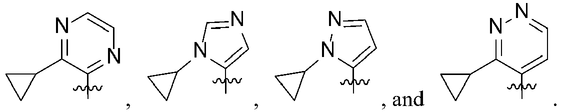

In some specific embodiments, R⁵ is selected from the group consisting of the following groups optionally substituted with one or more R^{a"}: pyrazolyl, imidazolyl, thiazolyl, triazolyl, pyridinyl, pyrimidinyl, pyrazinyl, and pyridazinyl. In some specific embodiments, R⁵ is selected from the group consisting of the following groups optionally substituted with one or more R^{a"}: pyrazolyl, imidazolyl, thiazolyl, and triazolyl. In some specific embodiments, R⁵ is selected from the group consisting of the following groups optionally substituted with one or more R^{a"}: pyridinyl, pyrimidinyl, pyrazinyl, and pyridazinyl.

In some specific embodiments, R⁵ is selected from the group consisting of

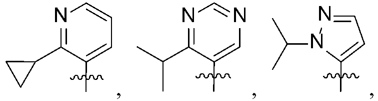

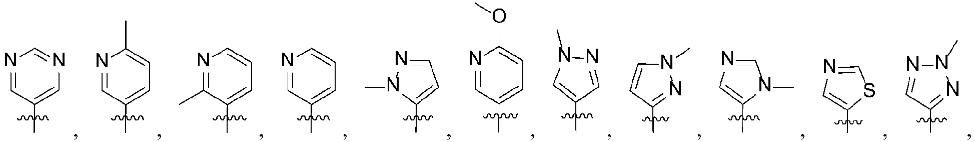

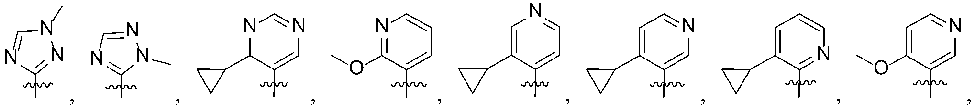

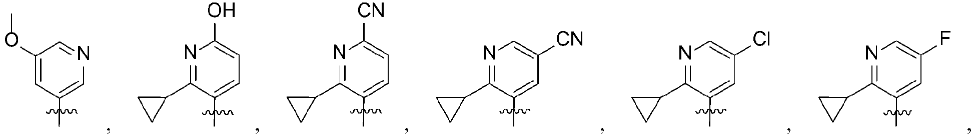

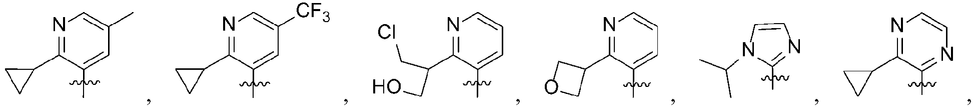

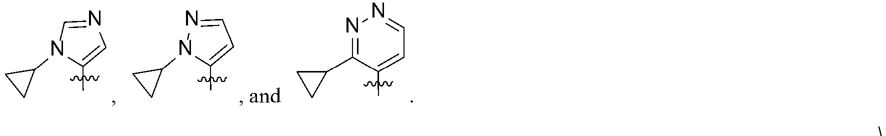

In some specific embodiments, R⁵ is selected from the group consisting of 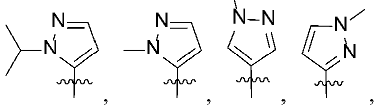 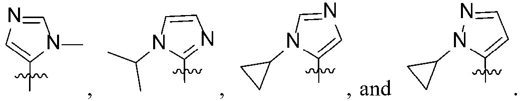 In some specific embodiments, R⁵ is selected from the group consisting of

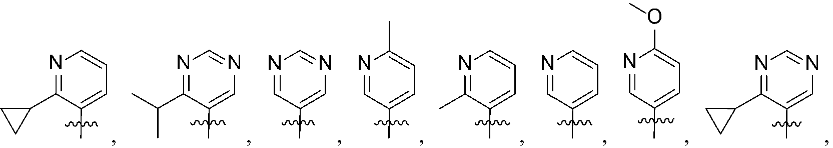

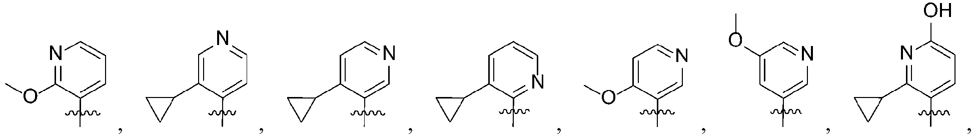

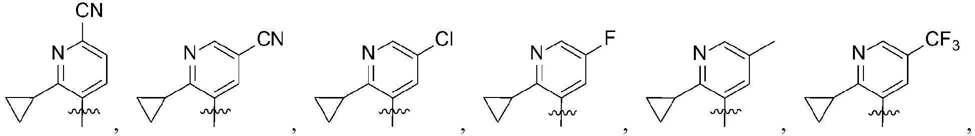

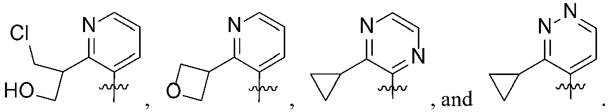

In some other embodiments, R⁵ is selected from the group consisting of halogen, -OH, -CN, -NH₂, and the following groups optionally substituted with one or more R^{a"}: -C(O)NR^{a'}R^{b'}, -C(O)-(5- to 6-membered heterocycloalkyl), -N(C₁₋₄ alkyl)(CO)OC₁₋₄ alkyl, -C(O)-C₃₋₆ cycloalkyl, phenyl, and 5- to 6-membered heteroaryl, wherein R^{a'}, R^{b'}, and R^{a"} are as defined in the present disclosure.

In some other embodiments, R⁵ is selected from the group consisting of halogen, -OH, -CN, -NH₂, or the following groups optionally substituted with one or more R^{a"}: -C(O)NR^{a'}R^{b'}, -C(O)-(5- to 6-membered heterocycloalkyl), -N(C₁₋₃ alkyl)(CO)OC₁₋₃ alkyl, and 5- to 6-membered heteroaryl, wherein R^{a'}, R^{b'}, and R^{a"} are as defined in the present disclosure.

In some other embodiments, R⁵ is selected from the group consisting of F, Cl, Br, -C(O)(NR^{a'}R^{b'}), -C(O)-5-membered heterocycloalkyl, -N(C₁₋₂ alkyl)(CO)OC₁₋₂ alkyl, and 5- to 6-membered heteroaryl, wherein the -C(O)-(5-membered heterocycloalkyl) or 5- to 6-membered heteroaryl is optionally substituted with 1 or 2 R^{a"}, and R^{a'}, R^{b'}, and R^{a"} are as defined in the present disclosure.

In some other embodiments, R⁵ is selected from the group consisting of Br, -C(O)(NR^{a'}R^{b'}), -C(O)-aza-cyclopentyl, pyridinyl, pyrimidinyl, pyrazolyl, and -N(CH₂CH₃)(CO)OCH₃, wherein the -C(O)-aza-cyclopentyl, pyridinyl, pyrimidinyl, or pyrazolyl is optionally substituted with one or more isopropyl or cyclopropyl, and R^{a'} and R^{b'} are as defined in the present disclosure.

In some other embodiments, R⁵ is selected from the group consisting of Br,

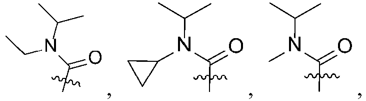

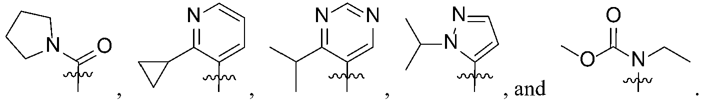

In some embodiments, R^{a'} is selected from the group consisting of hydrogen, methyl, ethyl, C₄₋₆ alkyl, C₃₋₆ cycloalkyl, and 3- to 6-membered heterocycloalkyl.

In some embodiments, each R^{a'} is independently selected from the group consisting of hydrogen, C₁₋₆ alkyl, C₃₋₆ cycloalkyl, and 3- to 6-membered heterocycloalkyl.

In some embodiments, each R^{a'} is independently selected from the group consisting of hydrogen, C₁₋₄ alkyl, and C₃₋₄ cycloalkyl.

In some embodiments, each R^{a'} is independently selected from the group consisting of hydrogen, methyl, ethyl, isopropyl, 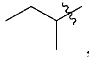 and cyclopropyl.

In some embodiments, each R^{a'} is independently selected from the group consisting of hydrogen, methyl, ethyl, 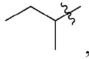 and cyclopropyl.

In some other embodiments, R^{a'} is selected from the group consisting of methyl, ethyl, C₄₋₅ alkyl, C₃₋₆ cycloalkyl, and 3- to 6-membered heterocycloalkyl.

In some other embodiments, R^{a'} is selected from the group consisting of methyl, ethyl, and C₃₋₅ cycloalkyl.

In some other embodiments, R^{a'} is selected from the group consisting of methyl, ethyl, and cyclopropyl.

In some embodiments, each R^{b'} is independently selected from the group consisting of hydrogen, C₁₋₆ alkyl, C₃₋₁₀ cycloalkyl, 4- to 10-membered heterocycloalkyl, phenyl, and 5- to 6-membered heteroaryl, wherein the C₁₋₆ alkyl, C₃₋₁₀ cycloalkyl, 4- to 10-membered heterocycloalkyl, phenyl, and 5- to 6-membered heteroaryl is optionally substituted with one or more halogen, -CN, -OH, or -NH₂.

In some embodiments, each R^{b'} is independently selected from the group consisting of hydrogen and the following groups optionally substituted with one or more halogen: C₁₋₆ alkyl, C₃₋₉ cycloalkyl, and 4- to 9-membered heterocycloalkyl.

In some embodiments, each R^{b'} is independently selected from the group consisting of hydrogen, C₁₋₄ alkyl, C₃₋₅ cycloalkyl, C₉₋₁₀ cycloalkyl, and 4- to 7-membered heterocycloalkyl.

In some embodiments, each R^{b'} is independently selected from the group consisting of hydrogen, methyl, ethyl, n-propyl, isopropyl, 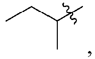 cyclopropyl, adamantyl, oxetanyl, pyrrolidinyl, morpholinyl, piperazinyl, piperidinyl, and azabicycloheptyl.

In some embodiments, each R^{b'} is independently selected from the group consisting of hydrogen, methyl, ethyl, n-propyl, isopropyl, 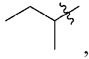 cyclopropyl, 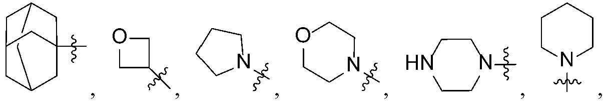 and

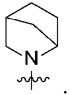

In some embodiments, each R^{b'} is independently selected from the group consisting of hydrogen, methyl, ethyl, n-propyl, isopropyl, 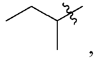 cyclopropyl,

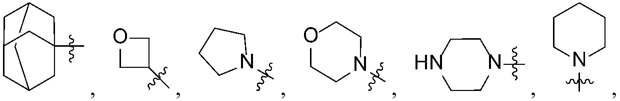

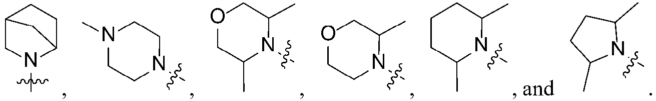

In some other embodiments, R^{b'} is selected from the group consisting of hydrogen, C₁₋₄ alkyl, C₃₋₆ cycloalkyl, 3- to 6-membered heterocycloalkyl, phenyl, and 5- to 6-membered heteroaryl, wherein the C₁₋₄ alkyl, C₃₋₆ cycloalkyl, 3-to 6-membered heterocycloalkyl, phenyl, or 5- to 6-membered heteroaryl is optionally substituted with one or more halogen, -CN, -OH, or -NH₂.

In some other embodiments, R^{b'} is selected from the group consisting of C₁₋₄ alkyl, 3- to 5-membered cycloalkyl, and 3- to 5-membered heterocycloalkyl, wherein the C₁₋₄ alkyl, 3- to 5-membered cycloalkyl, or 3- to 5-membered heterocycloalkyl is optionally substituted with one or more halogen.

In some other embodiments, R^{b'} is C₁₋₃ alkyl.

In some other embodiments, R^{b'} is isopropyl.

In some embodiments, each R^{a"} is independently selected from the group consisting of halogen, -OH, -CN, -NH₂, - NH(C₁₋₄ alkyl), -N(C₁₋₄ alkyl)₂, C₁₋₄ alkyl, C₂₋₄ alkenyl, C₂₋₄ alkynyl, C₁₋₄ alkyl-O-, C₃₋₆ cycloalkyl, and 3- to 6-membered heterocycloalkyl, wherein the -NH(C₁₋₄ alkyl), -N(C₁₋₄ alkyl)₂, C₁₋₄ alkyl, C₂₋₄ alkenyl, C₂₋₄ alkynyl, C₁₋₄ alkyl-O-, C₃₋₆ cycloalkyl, or 3- to 6-membered heterocycloalkyl is optionally substituted with one or more halogen, -OH, -CN, or -NH₂.

In some embodiments, each R^{a"} is independently selected from the group consisting of halogen, -OH, -CN, -NH₂, C₁₋₄ alkyl, -NH(C₁₋₄ alkyl), -OC₁₋₄ alkyl, C₃₋₆ cycloalkyl, and 3- to 6-membered heterocycloalkyl, wherein the C₁₋₄ alkyl, -NH(C₁₋₄ alkyl), -OC₁₋₄ alkyl, C₃₋₆ cycloalkyl, or 3- to 6-membered heterocycloalkyl is optionally substituted with one or more halogen or -OH.

In some embodiments, each R^{a"} is independently selected from the group consisting of halogen, -OH, -CN, -NH₂, C₁₋₄ alkyl, -OC₁₋₄ alkyl, C₃₋₅ cycloalkyl, and 3- to 5-membered heterocycloalkyl, wherein the C₁₋₄ alkyl, -OC₁₋₄ alkyl, C₃₋₅ cycloalkyl, or 3- to 5-membered heterocycloalkyl is optionally substituted with 1, 2, or 3 groups selected from the group consisting of: halogen, -OH, -CN, and -NH₂.

In some embodiments, each R^{a"} is independently selected from the group consisting of F, Cl, -OH, -CN, -OCH₃, - CF₃, methyl, isopropyl, cyclopropyl, 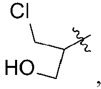 and oxetanyl.

In some other embodiments, each R^{a"} is independently selected from the group consisting of F, Cl, -OH, -CN, C₁₋₃ alkyl, -OC₁₋₃ alkyl, and C₃₋₄ cycloalkyl, wherein the C₁₋₃ alkyl is optionally substituted with 1, 2, or 3 F. In some other embodiments, each R^{a"} is independently selected from the group consisting of F, Cl, -OH, -CN, -OCH₃, -CF₃, methyl, isopropyl, and cyclopropyl.

In some other embodiments, each R^{a"} is independently selected from the group consisting of halogen, -OH, -CN, - NH₂, C₁₋₄ alkyl, C₃₋₆ cycloalkyl, and C₃₋₆ heterocycloalkyl, wherein the C₁₋₄ alkyl, C₃₋₅ cycloalkyl, or C₃₋₅ heterocycloalkyl is optionally substituted with one or more halogen.

In some other embodiments, each R^{a"} is independently selected from the group consisting of F, Cl, Br, C₁₋₄ alkyl, C₃₋₆ cycloalkyl, and C₃₋₆ heterocycloalkyl.

In some other embodiments, each R^{a"} is independently selected from the group consisting of C₁₋₄ alkyl and C₃₋₆ cycloalkyl.

In some other embodiments, each R^{a"} is independently selected from the group consisting of C₁₋₃ alkyl and C₃₋₄ cycloalkyl.

In some other embodiments, each R^{a"} is independently selected from the group consisting of isopropyl and cyclopropyl.

In some specific embodiments, each R^{a"} is independently selected from the group consisting of halogen, -OH, -CN, -NH₂, C₁₋₄ alkyl, C₂₋₄ alkenyl, C₂₋₄ alkynyl, C₁₋₄ alkyl-O-, C₃₋₆ cycloalkyl, and 3- to 6-membered heterocycloalkyl, wherein the C₁₋₄ alkyl is optionally substituted with one or more halogen, -OH, -CN, or -NH₂.

In some specific embodiments, each R^{a"} is independently selected from the group consisting of halogen, -OH, -CN, -NH₂, C₁₋₃ alkyl, C₁₋₃ alkyl-O-, C₃₋₄ cycloalkyl, and 3- to 4-membered heterocycloalkyl, wherein the C₁₋₄ alkyl is optionally substituted with one or more halogen, -OH, -CN, or -NH₂.

In some embodiments, R⁶ is selected from the group consisting of hydrogen, halogen, -OH, -CN, -NH₂, C₁₋₄ alkyl, C₂₋₄ alkenyl, C₂₋₄ alkynyl, and C₁₋₄ heteroalkyl, wherein the C₁₋₄ alkyl, C₂₋₄ alkenyl, C₂₋₄ alkynyl, or C₁₋₄ heteroalkyl is optionally substituted with one or more halogen, -CN, -OH, or -NH₂.

In some embodiments, R⁶ is selected from the group consisting of hydrogen, halogen, -OH, -CN, -NH₂, C₁₋₆ alkyl, and C₁₋₆ haloalkyl.

In some embodiments, R⁶ is selected from the group consisting of hydrogen, halogen, -OH, -CN, -NH₂, C₁₋₃ alkyl, and C₁₋₃ haloalkyl.

In some embodiments, R⁶ is selected from the group consisting of hydrogen, halogen, -OH, -CN, -NH₂, C₁₋₄ alkyl, C₂₋₄ alkenyl, C₂₋₄ alkynyl, and C₁₋₄ heteroalkyl.

In some embodiments, R⁶ is selected from the group consisting of hydrogen, halogen, -OH, -CN, -NH₂, C₁₋₄ alkyl, and C₁₋₄ heteroalkyl. In some embodiments, R⁶ is selected from the group consisting of hydrogen, halogen, and C₁₋₆ alkyl. In some embodiments, R⁶ is selected from the group consisting of hydrogen, halogen (e.g., F, Cl, Br, or I), and C₁₋₃ alkyl.

In some embodiments, R⁶ is selected from the group consisting of hydrogen, F, Cl, Br, and methyl.

In some embodiments, R⁶ is selected from the group consisting of hydrogen, F, and methyl.

In some other embodiments, R⁶ is F.

In some embodiments, R^{a} and R⁷ are each independently selected from the group consisting of halogen, -CN, -OH, - NH₂, C₁₋₄ alkyl, C₂₋₄ alkenyl, C₂₋₄ alkynyl, -OC₁₋₄ alkyl, C₃₋₆ cycloalkyl, and 3- to 6-membered heterocycloalkyl, wherein the C₁₋₄ alkyl, C₂₋₄ alkenyl, C₂₋₄ alkynyl, -OC₁₋₄ alkyl, C₃₋₆ cycloalkyl, or 3- to 6-membered heterocycloalkyl is optionally substituted with one or more halogen, -CN, -OH, or -NH₂.

In some other embodiments, R^{a} and R⁷ are each independently selected from the group consisting of halogen, -CN, - OH, -NH₂, C₁₋₄ alkyl, C₂₋₄ alkenyl, C₂₋₄ alkynyl, and -OC₁₋₄ alkyl, wherein the C₁₋₄ alkyl, C₂₋₄ alkenyl, C₂₋₄ alkynyl, or -OC₁₋₄ alkyl is optionally substituted with one or more halogen, -CN, -OH, or -NH₂.

In some embodiments, each R⁷ is independently selected from the group consisting of halogen, -CN, -OH, -NH₂, C₁₋₄ alkyl, C₂₋₄ alkenyl, C₂₋₄ alkynyl, -OC₁₋₄ alkyl, C₃₋₆ cycloalkyl, and 3- to 6-membered heterocycloalkyl.

In some embodiments, each R⁷ is independently selected from the group consisting of halogen, C₁₋₃ alkyl, C₃₋₄ cycloalkyl, and 3- to 4-membered heterocycloalkyl.

In some embodiments, each R⁷ is independently selected from the group consisting of C₃₋₆ cycloalkyl and 3- to 6-membered heterocycloalkyl.

In some embodiments, each R⁷ is independently selected from C₃₋₄ cycloalkyl.

In some embodiments, each R⁷ is independently cyclopropyl.

In some other embodiments, each R⁷ is independently selected from the group consisting of halogen, -CN, -OH, - NH₂, C₁₋₄ alkyl, C₂₋₄ alkenyl, C₂₋₄ alkynyl, and -OC₁₋₄ alkyl.

In some other embodiments, each R⁷ is independently selected from the group consisting of halogen, -CN, -OH, - NH₂, and C₁₋₄ alkyl.

In some other embodiments, each R⁷ is independently selected from the group consisting of halogen and C₁₋₃ alkyl. In some other embodiments, each R⁷ is independently selected from the group consisting of F, Cl, and Br.

In some embodiments, each R^{a} is independently selected from the group consisting of halogen, -CN, -OH, -NH₂, C₁₋₄ alkyl, C₂₋₄ alkenyl, C₂₋₄ alkynyl, and -OC₁₋₄ alkyl.

In some embodiments, each R^{a} is independently selected from the group consisting of halogen, -CN, -OH, -NH₂, and C₁₋₄ alkyl.

In some embodiments, each R^{a} is independently selected from the group consisting of halogen and C₁₋₃ alkyl.

In some embodiments, each R^{a} is independently selected from the group consisting of F, Cl, and Br.

In some embodiments, each R^{a} is independently F.

In some embodiments, m is selected from the group consisting of 0, 1, and 2.

In some embodiments, m is selected from the group consisting of 0 and 1.

In some embodiments, m is selected from 1.

In some embodiments, m is selected from 0.

In some embodiments, n is selected from the group consisting of 1 and 2. In some embodiments, n is selected from 2.

In some embodiments, p is selected from the group consisting of 0, 1, and 2.

In some embodiments, p is selected from the group consisting of 0 and 1. In some embodiments, p is selected from 1.

In some embodiments, p is selected from 0.

In some embodiments, q is selected from 1.

In some embodiments, the structural fragment 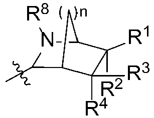 is selected from the group consisting of

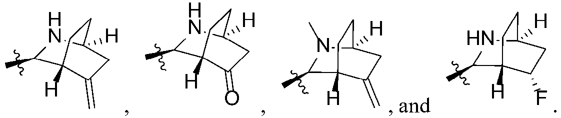

In some embodiments, the structural fragment 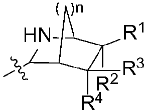 is selected from the group consisting of

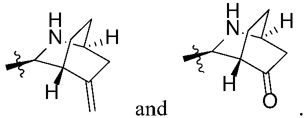

In some embodiments, the heteroalkyl, heteroaryl, heteroarylene, heterocycloalkyl, heterocyclyl, heterocyclylene, heterocycloalkylene (e.g., heteromonocycloalkylene), or heterospirocycloalkylene comprises 1, 2, 3, or 4 heteroatoms selected from the group consisting of B, N, O, S, and P, and the remaining ring atoms are selected from carbon. In some embodiments, the heteroalkyl, heteroaryl, heteroarylene, heterocycloalkyl, heterocyclyl, heterocyclylene, heterocycloalkylene, or heterospirocycloalkylene comprises 1, 2, or 3 heteroatoms selected from the group consisting of N, O, and S, and the remaining ring atoms are selected from carbon. In some embodiments, the heteroalkyl, heteroaryl, heteroarylene, heterocycloalkyl, heterocyclyl, heterocyclylene, heterocycloalkylene, or heterospirocycloalkylene comprises 1, 2, or 3 heteroatoms selected from the group consisting of N and O, and the remaining ring atoms are selected from carbon. In some embodiments, the heteroalkyl, heteroaryl, heteroarylene, heterocycloalkyl, heterocyclyl, heterocyclylene, heterocycloalkylene, or heterospirocycloalkylene comprises 1 or 2 heteroatoms selected from the group consisting of N and O, and the remaining ring atoms are selected from carbon. In some embodiments, the halo is selected from the group consisting of fluoro, chloro, and bromo. In some embodiments, the halo is selected from the group consisting of fluoro and chloro. In some embodiments, the halo is selected from fluoro.

In some embodiments, the C₁₋₁₀ is selected from the group consisting of C₁₋₉, C₁₋₈, C₁₋₇, C₁₋₆, C₁₋₄, C₁₋₃, and C₁₋₂. In some embodiments, the C₁₋₆ is selected from the group consisting of C₁₋₄, C₁₋₃, and C₁₋₂. In some embodiments, the C₁₋₄ is selected from the group consisting of C₄, C₃, C₂, and C₁. In some embodiments, the C₁₋₃ is selected from the group consisting of C₃, C₂, and C₁.

In some embodiments, the C₂₋₁₀ is selected from the group consisting of C₂₋₈, C₂₋₆, C₂₋₅, C₂₋₄, and C₂₋₃. In some embodiments, the C₂₋₆ is selected from the group consisting of C₂₋₄ and C₂₋₃. In some embodiments, the C₂₋₄ is selected from the group consisting of C₄, C₃, and C₂.

In some embodiments, the C₃₋₆ is selected from the group consisting of C₃₋₅, C₃₋₄, C₄₋₆, C₄₋₅, and C₅₋₆. In some embodiments, the C₆₋₁₀ is selected from the group consisting of C₆₋₉, C₆₋₈, C₆₋₇, C₇₋₁₀, C₇₋₉, C₇₋₈, C₈₋₁₀, C₈₋₉, and C₉₋₁₀.

In some embodiments, the C₃₋₁₀ is selected from the group consisting of C₃₋₉, C₃₋₈, C₃₋₇, C₃₋₆, C₃₋₅, C₃₋₄, C₄₋₁₀, C₄₋₉, C₄₋₈, C₄₋₇, C₄₋₆, C₄₋₅, C₅₋₁₀, C₅₋₉, C₅₋₈, C₅₋₇, C₅₋₆, C₆₋₁₀, C₆₋₉, C₆₋₈, C₆₋₇, C₇₋₁₂, C₇₋₁₀, C₇₋₉, C₇₋₈, C₈₋₁₂, C₈₋₁₀, C₈₋₉, C₉₋₁₂, and C₉₋₁₀. In some embodiments, the C₃₋₁₅ is selected from the group consisting of C₃₋₁₂ and C₃₋₁₀. In some embodiments, the C₃₋₁₂ is selected from C₃₋₁₀. In some embodiments, the C₆₋₁₂ is selected from C₆₋₁₀.

In some embodiments, the 3- to 6-membered is selected from the group consisting of 3- to 5-membered, 3- to 4-membered, 4- to 6-membered, 4- to 5-membered, and 5- to 6-membered. In some embodiments, the 5- to 10-membered is selected from the group consisting of 5- to 8-membered, 5- to 7-membered, 5- to 6-membered, 6- to 10-membered, 6- to 9-membered, 6- to 8-membered, 6- to 7-membered, 7- to 10-membered, 7- to 9-membered, 7- to 8-membered, 8- to 10-membered, 8- to 9-membered, and 9- to 10-membered. In some embodiments, the 3- to 10-membered is selected from the group consisting of 3- to 9-membered, 3- to 8-membered, 3- to 7-membered, 3- to 6-membered, 3- to 5-membered, 3- to 4-membered, 4- to 10-membered, 4- to 9-membered, 4- to 8-membered, 4- to 7-membered, 4- to 6-membered, 4- to 5-membered, 5- to 10-membered, 5- to 9-membered, 5- to 8-membered, 5- to 7-membered, 5- to 6-membered, 6- to 10-membered, 6- to 9-membered, 6- to 8-membered, 6- to 7-membered, 7- to 10-membered, 7- to 9-membered, 7- to 8-membered, 8- to 10-membered, 8- to 9-membered, and 9- to 10-membered. In some embodiments, the 3- to 15-membered is selected from the group consisting of 3- to 12-membered and 3- to 10-membered. In some embodiments, the 3- to 12-membered is selected from 3- to 10-membered. In some embodiments, the 5- to 12-membered is selected from 5- to 10-membered.

In some embodiments, the compound of formula (I), formula (II), or formula (III) or the pharmaceutically acceptable salt thereof of the present disclosure is selected from the group consisting of the following compounds of formula (I'), formula (II'), formula (III'), formula (IA), formula (IIA), formula (IB), formula (IIB), formula (IC), formula (IIC), formula (IID), formula (IA-1), formula (IA-2), formula (IIA-1), formula (IB-1), and formula (IC-1) or pharmaceutically acceptable salts thereof: 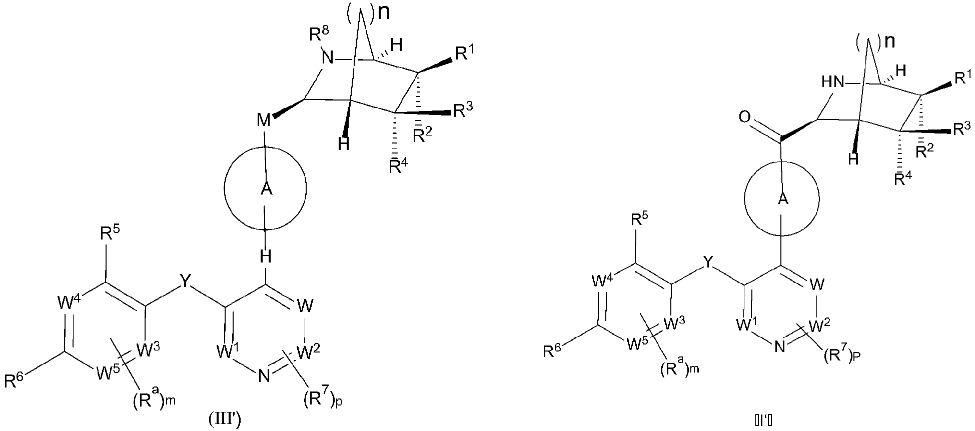 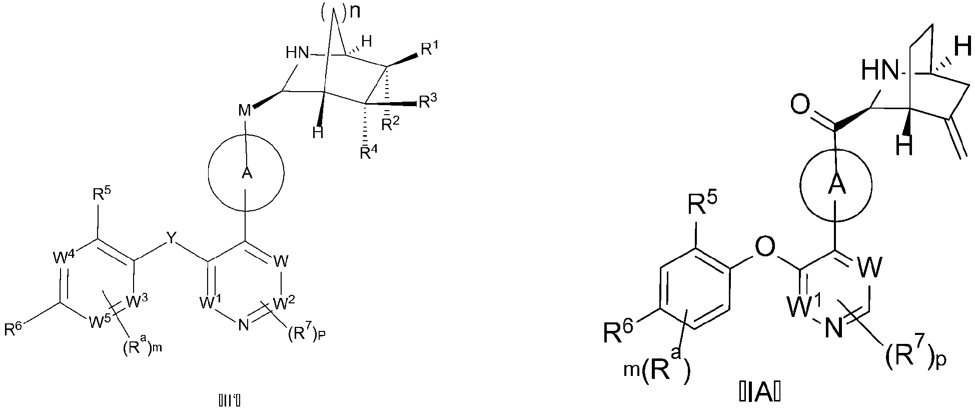 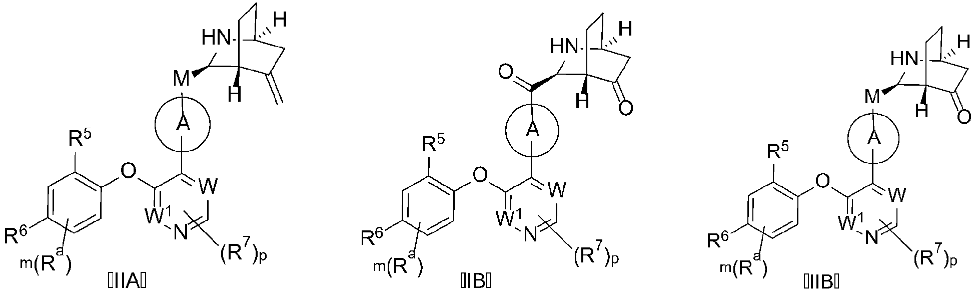 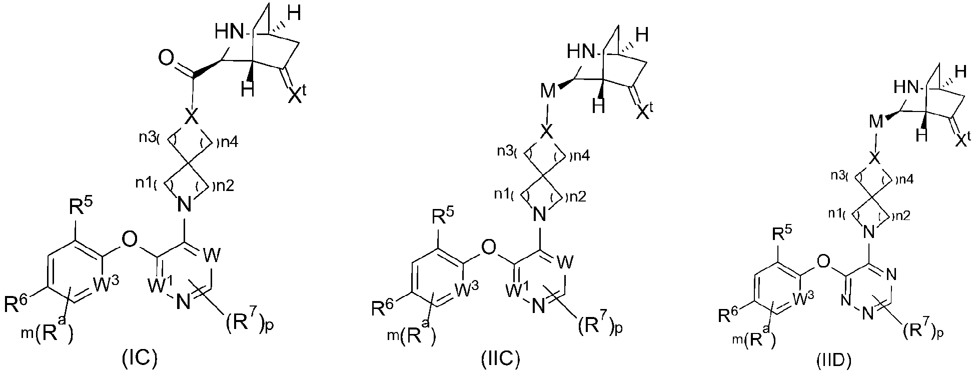 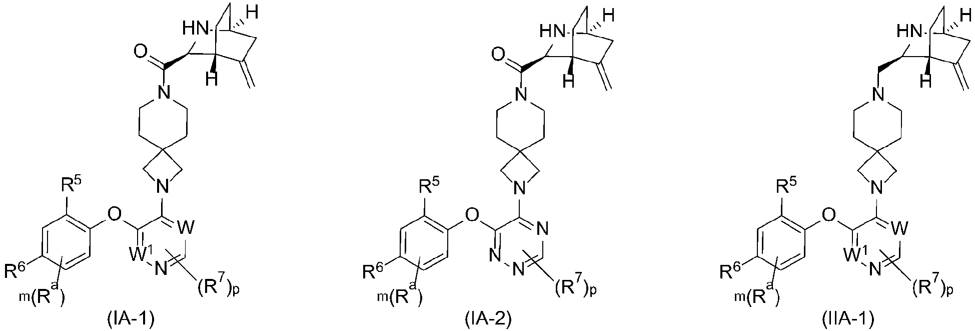 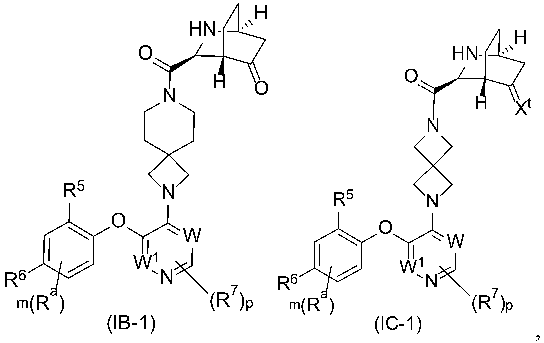
wherein Y, H, n1, n2, n3, n4, W, W¹, W², W³, W⁴, W⁵, m, p, n, ring A, R¹, R², R³, R⁴, R⁵, R⁶, R⁷, R⁸, R^{a}, and M are as defined in the present disclosure;
X^{t} is selected from the group consisting of O and CH(R^{c2}), wherein R^{c2} is selected from the group consisting of hydrogen, C₁₋₆ alkyl, C₂₋₆ alkenyl, C₂₋₆ alkynyl, and C₁₋₆ heteroalkyl, wherein the C₁₋₆ alkyl, C₂₋₆ alkenyl, C₂₋₆ alkynyl, or C₁₋₆ heteroalkyl being optionally substituted with one or more halogen, -CN, -OH, or -NH₂.

In some embodiments, X^{t} is O. In some embodiments, X^{t} is selected from CH(R^{c2}). In some embodiments, X^{t} is CH₂. In some embodiments, R^{c2} is selected from the group consisting of hydrogen, C₁₋₄ alkyl, C₂₋₄ alkenyl, C₂₋₄ alkynyl, and C₁₋₄ heteroalkyl, wherein the C₁₋₄ alkyl, C₂₋₄ alkenyl, C₂₋₄ alkynyl, or C₁₋₄ heteroalkyl is optionally substituted with one or more halogen, -CN, -OH, or -NH₂.

In some embodiments, R^{c2} is selected from the group consisting of hydrogen, C₁₋₄ alkyl, C₂₋₄ alkenyl, C₂₋₄ alkynyl, and C₁₋₄ heteroalkyl.

In some embodiments, R^{c2} is selected from the group consisting of hydrogen and C₁₋₃ alkyl.

In some embodiments, R^{c2} is hydrogen.

In some embodiments, the present disclosure encompasses the variables defined above and embodiments thereof, as well as any combination thereof.

It should be understood that any embodiment of the compounds of the present disclosure as described above and any specific substituent set forth herein for specific Y, ring A, H, W, M, R^{u}, R^{v}, W¹, W², W³, W⁴, W⁵, R¹, R², R³, R⁴, R⁵, R⁶, R⁷, R⁸, R^{a}, R^{a'}, R^{b'}, R^{a"}, R^{a1}, R^{b1}, R^{c1}, R^{d1}, R^{c2}, and R^{d2} in the compounds of the present disclosure as described above may be independently combined with other embodiments and/or substituents of the compounds of the present disclosure to form embodiments of the present disclosure not specifically set forth above. In addition, in the event that a range of substituents is disclosed for any specific Y, ring A, H, W, M, R^{u}, R^{v}, W¹, W², W³, W⁴, W⁵, R¹, R², R³, R⁴, R⁵, R⁶, R⁷, R⁸, R^{a}, R^{a'}, R^{b'}, Ra", R^{a1}, R^{b1}, R^{c1}, R^{d1}, R^{c2}, and R^{d2} in specific embodiments and/or claims, it should be understood that one or more substituents may be deleted from the range, and the remaining range of substituents shall also be considered an embodiment of the present disclosure. It should be understood that when substituent R^{a} is present (i.e., when m is not 0), its substitution occurs at the position of W³, W⁴, or W⁵; when substituent R⁷ is present (i.e., when p is not 0), its substitution occurs at the position of W, W¹, or W².

In some embodiments, provided is a compound below or a pharmaceutically acceptable salt thereof:

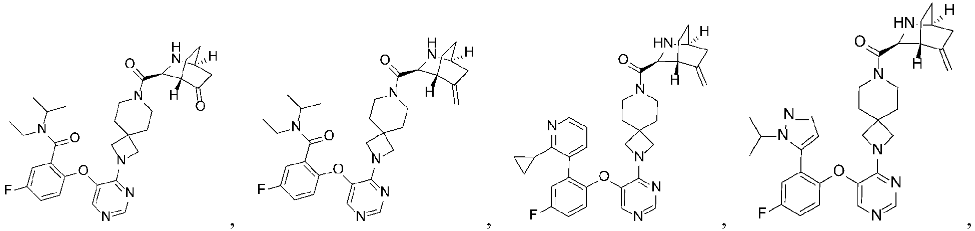

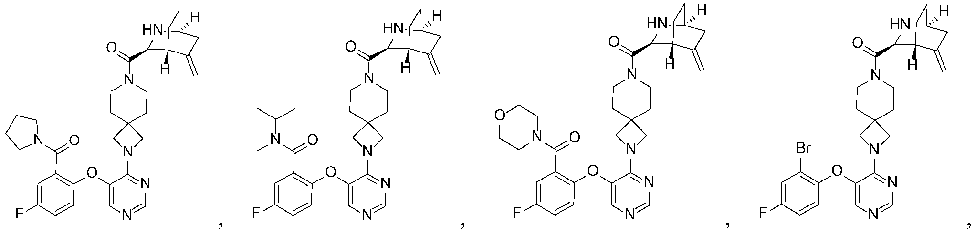

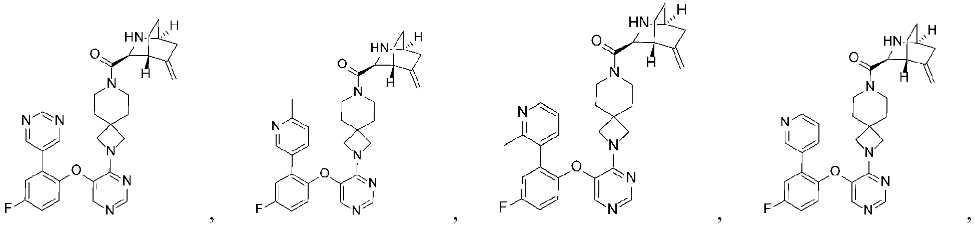

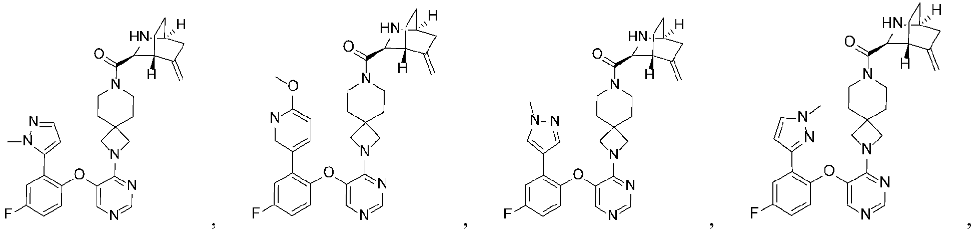

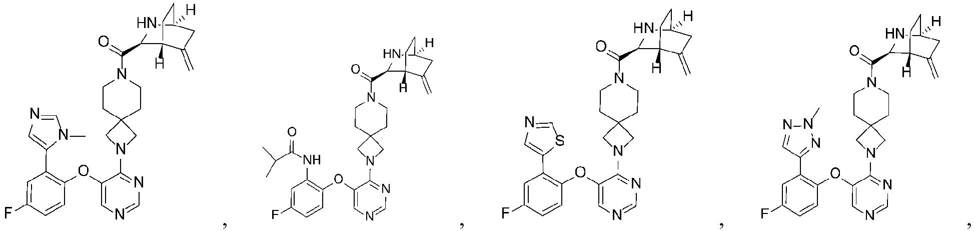

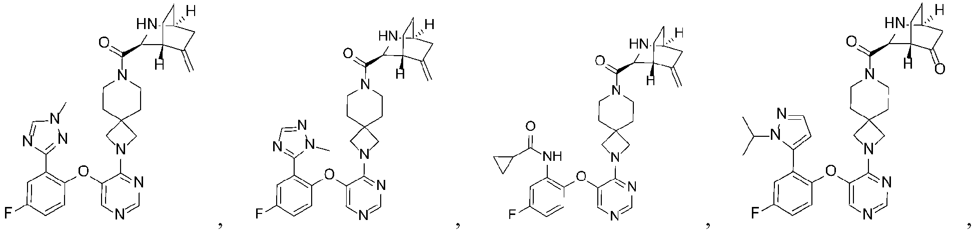

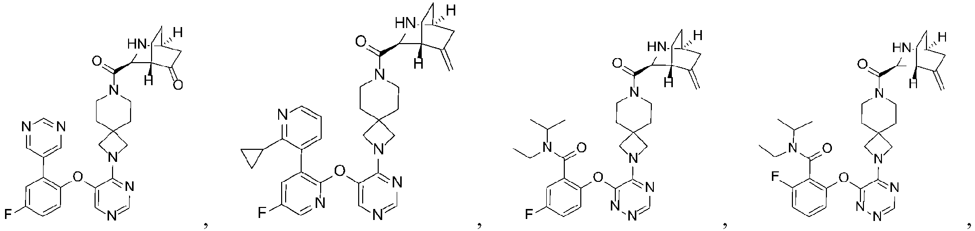

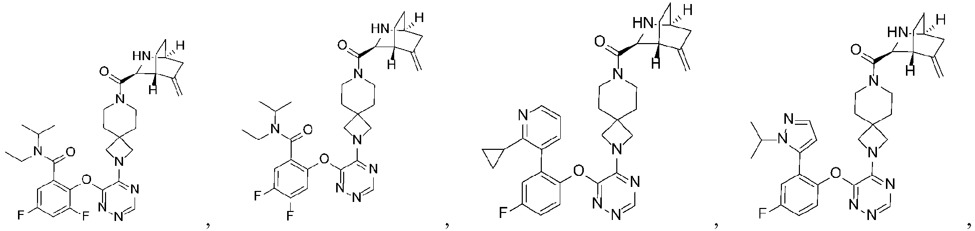

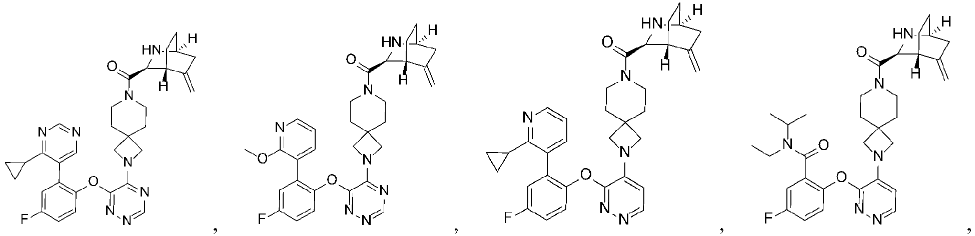

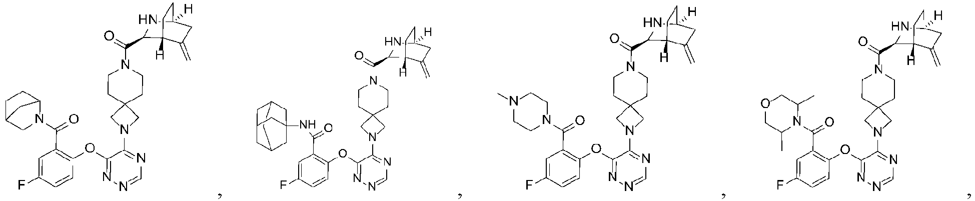

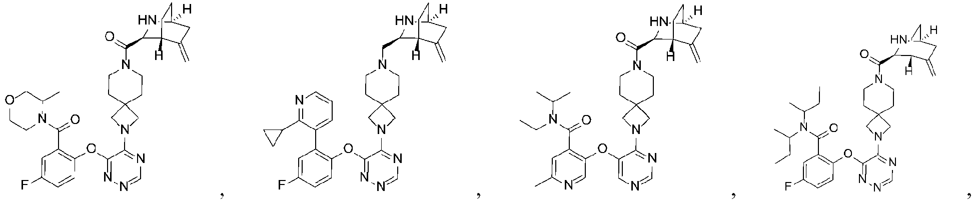

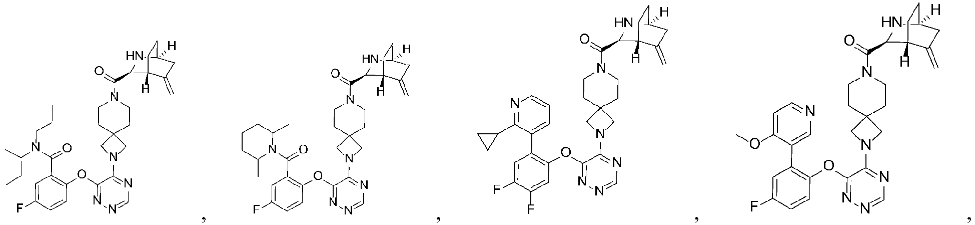

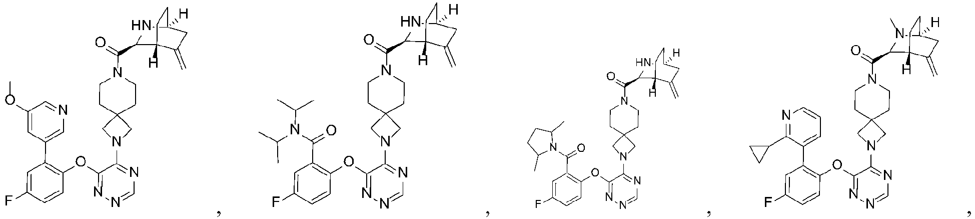

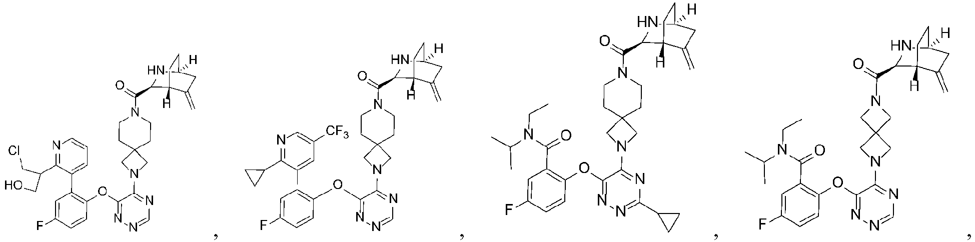

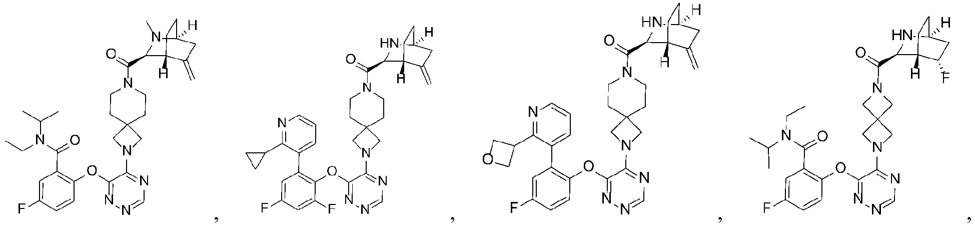

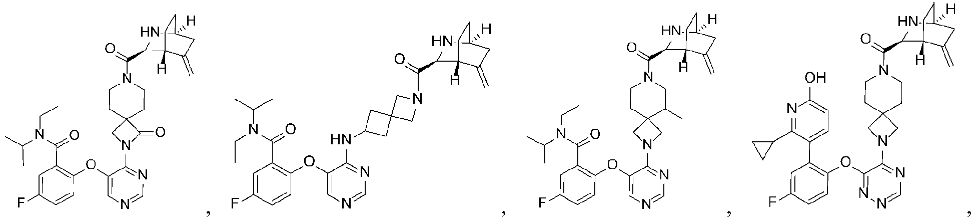

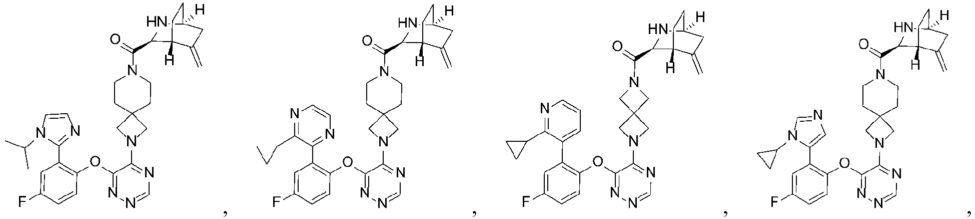

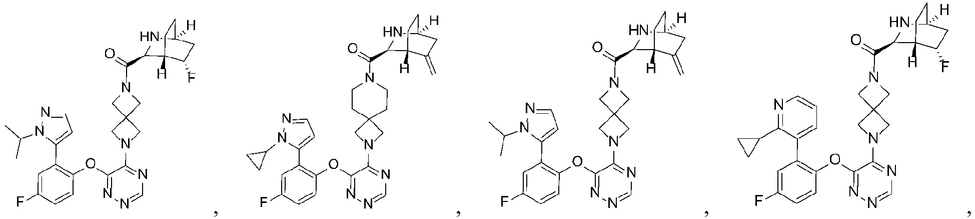

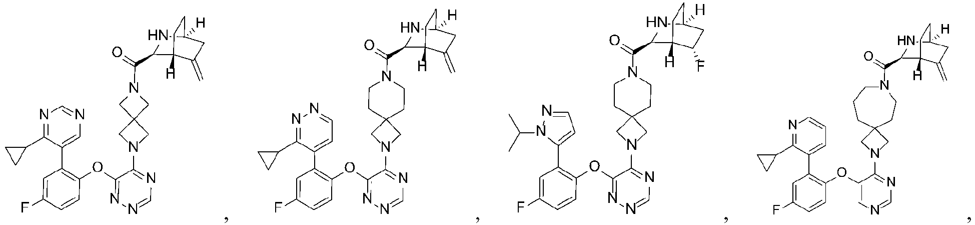

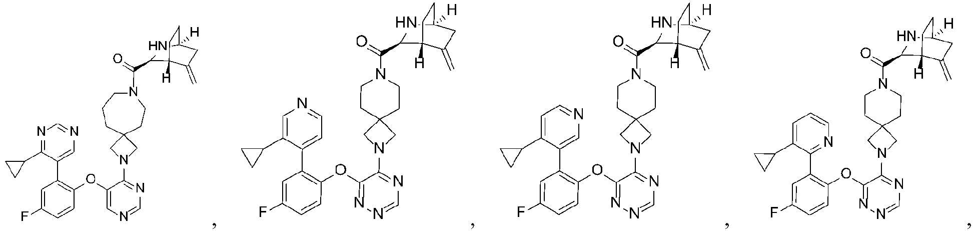

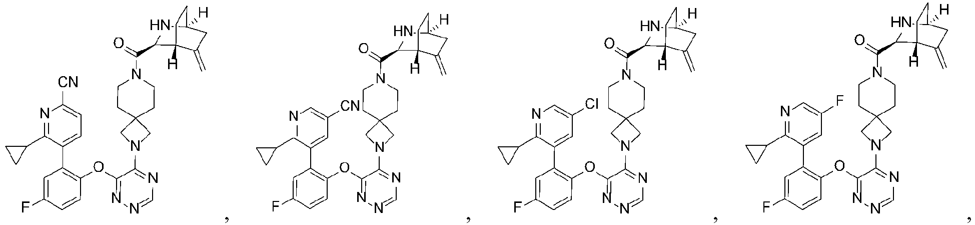

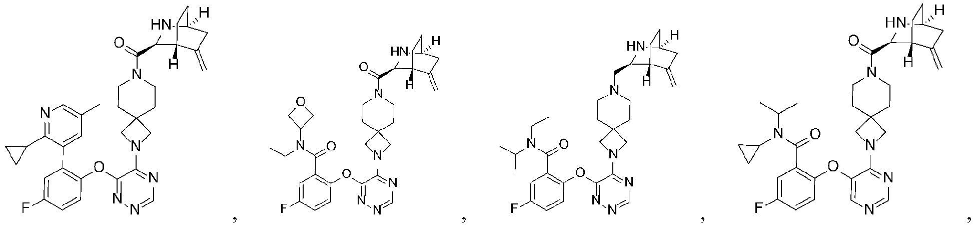

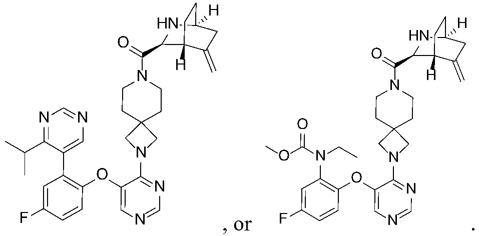

In another aspect, the present disclosure provides a pharmaceutical composition, which comprises the compound or the pharmaceutically acceptable salt thereof of the present disclosure described above. In some embodiments, the pharmaceutical composition of the present disclosure further comprises a pharmaceutically acceptable excipient.

In another aspect, the present disclosure provides a method for treating a disease in a mammal, which comprises administering to a mammal, preferably a human, in need of the treatment a therapeutically effective amount of the compound or the pharmaceutically acceptable salt thereof, or the pharmaceutical composition thereof described above.

In another aspect, the present disclosure provides use of the compound or the pharmaceutically acceptable salt thereof, or the pharmaceutical composition thereof described above for preparing a medicament for treating a disease.

In another aspect, the present disclosure provides use of the compound or the pharmaceutically acceptable salt thereof, or the pharmaceutical composition thereof described above for treating a disease.

In another aspect, the present disclosure provides the compound or the pharmaceutically acceptable salt thereof, or the pharmaceutical composition thereof described above for treating a disease.

In some embodiments, the disease is selected from a Menin protein-related disease. In some embodiments, the disease is selected from cancer. Preferably, the cancer is selected from leukemia; in some embodiments, the cancer or leukemia is selected from myeloid leukemia (or acute myeloid leukemia).

### Technical Effects

The compounds of the present disclosure have one or more of the following beneficial effects: relatively good inhibitory activity against the proliferation of MOLM-13 cells and/or MV-4-11 cells, while possessing inhibitory activity against Menin-MLL protein binding; metabolic stability in liver microsomes; good *in vivo* pharmacokinetic properties (e.g., AUC, Cmax, or T1/2); and good *in vivo* drug effects.

### Definitions

Unless otherwise stated, the following terms used in the present disclosure shall have the following meanings. A certain term, unless otherwise specifically defined, should not be considered uncertain or unclear, but interpreted according to its common meaning in the art. When referring to a trade name, it is intended to refer to its corresponding commercial product or its active ingredient.

The term "substituted" means that any one or more hydrogen atoms on a specific atom are substituted with a substituent, as long as the valence of the specific atom is normal and the resulting compound is stable. When the substituent is oxo (i.e., =O), it means that two hydrogen atoms are substituted; oxo substitutions do not occur on aromatic groups.

The term "optional" or "optionally" means that the subsequently described event or circumstance may or may not occur, and the description includes instances where the event or circumstance occurs and instances where it does not. For example, ethyl being "optionally" substituted with halogen means that the ethyl may be unsubstituted (-CH₂CH₃), monosubstituted (e.g., -CH₂CH₂F), polysubstituted (e.g., -CHFCH₂F, -CH₂CHF₂, etc.), or fully substituted (-CF₂CF₃). It will be appreciated by those skilled in the art that for any group comprising one or more substituents, no substitution or substitution pattern that is sterically impossible and/or cannot be synthesized is introduced.

"One or more" used herein refers to an integer ranging from one to ten. For example, "one or more" refers to one, two, three, four, five, six, seven, eight, nine, or ten; or "one or more" refers to one, two, three, four, five, or six; or "one or more" refers to one, two, or three.

Cₘ₋ₙ used herein means that the moiety has an integer number of carbon atoms in the given range. For example, "C₁₋₆" means that the group may have 1 carbon atom, 2 carbon atoms, 3 carbon atoms, 4 carbon atoms, 5 carbon atoms, or 6 carbon atoms. For example, C₁₋₃ means that the group may have 1 carbon atom, 2 carbon atoms, or 3 carbon atoms. m- to n-membered herein means that the moiety has an integer number of members within the given range. For example, "3- to 12-membered" means that the group may be 3-membered, 4-membered, 5-membered, 6-membered, 7-membered, 8-membered, 9-membered, 10-membered, 11-membered, or 12-membered. For example, "5- to 10-membered" means that the group may be 5-membered, 6-membered, 7-membered, 8-membered, 9-membered, or 10-membered.

When any variable (e.g., R) occurs more than once in the constitution or structure of a compound, the definition of the variable in each case is independent. Therefore, for example, if a group is substituted with 2 R, the definition of each R is independent.

When a bond of a substituent is cross-linked to two atoms on a ring, the substituent can be bonded to any atom on the ring. For example, the structural unit 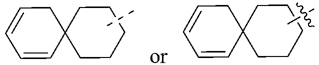 represents that substitution may occur at any one position of cyclohexyl or cyclohexadienyl.

The term "halo" or "halogen" refers to fluorine, chlorine, bromine, and iodine.

The term "hydroxyl" refers to an -OH group.

The term "amino" refers to an -NH₂ group.

The term "nitro" refers to an -NO₂ group.

The term "cyano" refers to a -CN group.

The term "alkyl" refers to hydrocarbyl with a general formula of CₙH₂ₙ₊₁. The alkyl may be linear or branched. For example, the term "C₁₋₆ alkyl" refers to alkyl containing 1 to 6 carbon atoms (e.g., methyl, ethyl, n-propyl, isopropyl, n-butyl, isobutyl, sec-butyl, tert-butyl, n-pentyl, 1-methylbutyl, 2-methylbutyl, 3-methylbutyl, neopentyl, hexyl, 2-methylpentyl, etc.). Similarly, the alkyl moieties (i.e., alkyl) of alkoxy, alkylamino, dialkylamino, alkylsulfonyl, and alkylthio have the same definition as described above.

The term "alkylene" refers to a divalent group formed by the removal of one hydrogen at any position of alkyl, for example, the term "C₁₋₆ alkyl" refers to alkylene containing 1 to 6 carbon atoms; the term "C₁₋₄ alkyl" refers to alkylene containing 1 to 4 carbon atoms, including but not limited to -CH₂-, -CH₂CH₂-, -CH₂CH₂CH₂-, or - CH₂CH₂CH₂CH₂-. Other groups such as "cycloalkylene", "heterocyclylene", "heterospirocycloalkylene", "heterocycloalkylene", "arylene", or "heteroarylene" are defined similarly and represent divalent groups. For example, the term "C₃₋₁₂ cycloalkylene" refers to cycloalkylene containing 3 to 12 carbon atoms; the term "6- to 10-membered cycloalkylene" refers to cycloalkylene containing 6 to 10 carbon atoms, including but not limited to 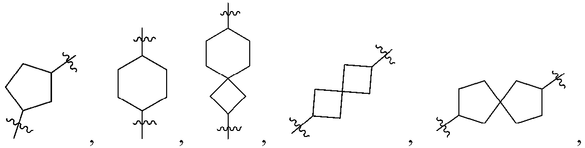 or the like. Non-limiting examples of the heterospirocycloalkylene include 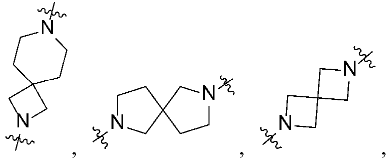 or the like. Non-limiting examples of the heterocycloalkylene include 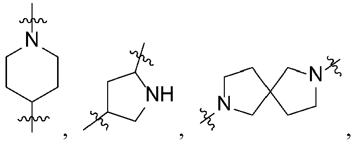 or the like. Examples of the arylene include, but are not limited to, 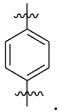 Examples of the heteroarylene include, but are not limited to, 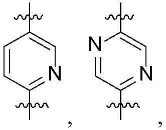 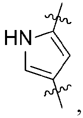 or the like.

The term "alkoxy" refers to -O-alkyl.

The term "alkenyl" refers to linear or branched unsaturated aliphatic hydrocarbyl consisting of carbon atoms and hydrogen atoms and having at least one double bond. Non-limiting examples of the alkenyl include, but are not limited to, ethenyl, 1-propenyl, 2-propenyl, 1-butenyl, isobutenyl, 1,3-butadienyl, and the like.

The term "alkynyl" refers to linear or branched unsaturated aliphatic hydrocarbyl consisting of carbon atoms and hydrogen atoms and having at least one triple bond. Non-limiting examples of the alkynyl include, but are not limited to, ethynyl (-C≡CH), 1-propynyl (-C≡C-CH₃), 2-propynyl (-CH₂-C≡CH), 1,3-butadiynyl (-C≡C-C≡CH), and the like. The term "bicyclic ring" or "bicyclic group" refers to a cyclic group containing two rings, which may be fully saturated, partially saturated, or aromatic. The bicyclic ring may consist entirely of C atoms or may contain one or more heteroatoms, for example, selected from the group consisting of N, O, S, and P. The bicyclic ring may be a fused ring, a bridged ring, or a spiro ring.

The term "cycloalkyl" refers to a fully saturated carbocyclic ring. Unless otherwise specified, the carbocyclic ring is usually a 5- to 20-membered or 3- to 10-membered ring. Unless otherwise specified, the cycloalkyl may be monocyclic, bicyclic, or tricyclic. Non-limiting examples of the cycloalkyl include, but are not limited to, cyclopropyl, cyclobutyl, cyclopentyl, cyclohexyl, norbornyl (bicyclo[2.2.1]heptyl), bicyclo[2.2.2]octyl, adamantyl, and the like. Unless otherwise specified, the term "hetero" refers to a heteroatom or a heteroatom group (i.e., a heteroatom-containing group), including atoms other than carbon (C) and hydrogen (H) and groups containing such heteroatoms. For example, heteroatoms include, but are not limited to, oxygen (O), nitrogen (N), sulfur (S), phosphorus (P), silicon (Si), germanium (Ge), aluminum (Al), and boron (B); specific heteroatoms or heteroatom groups are, e.g., -O-, -S-, -N=, =O, =S, -P(=O)-, -P(=O)₂-, -P(=O)O-, -P(=O)₂O-, -C(=O)O-, -C(=O)-, -C(=S)-, -S(=O), -S(=O)₂-, and optionally substituted -C(=O)N(H)-, -N(H)-, -C(=NH)-, -S(=O)₂N(H)-, or -S(=O)N(H)-. Preferably, the term "hetero" means that a heteroatom or a heteroatom of a heteroatom group (i.e., a heteroatom-containing group) is selected from the group consisting of oxygen, nitrogen, and sulfur.

The term "heteroalkyl" refers to linear or branched alkyl consisting of a certain number of carbon atoms and at least one heteroatom; it has preferably 1 to 14 carbons, more preferably 1 to 10 carbons, even more preferably 1 to 6 carbons, and most preferably 1 to 3 carbons in the chain, wherein the heteroatom is preferably selected from the group consisting of S, O, and N heteroatoms, and the number of the heteroatoms is preferably selected from the group consisting of 1, 2, and 3. The nitrogen and sulfur atoms are optionally oxidized, and the nitrogen atom is optionally quaternized. The heteroatom or heteroatom group may be located at any internal position of heterohydrocarbyl, including the position where the hydrocarbyl is attached to the rest of the molecule; exemplary heteroalkyl includes alkyl ether, secondary alkylamine and tertiary alkylamine, amide, alkyl sulfide, and the like, including alkoxy, alkylthio, and alkylamino; unless otherwise specified, C₁₋₆ heteroalkyl includes C₁, C₂, C₃, C₄, C₅, and C₆ heteroalkyl, e.g., C₁₋₆ alkoxy, C₁₋₆ alkylthio, or C₁₋₆ alkylamino.

The term "heterocyclyl" refers to a non-aromatic ring that is fully saturated or partially unsaturated (but not a fully unsaturated heteroaromatic group) and may exist as a monocyclic ring, a bridged ring, a fused ring, or a spiro ring.

Unless otherwise specified, the heterocyclic ring is usually a 3- to 20-membered ring, a 3- to 15-membered ring, a 3-to 12-membered ring, a 3- to 10-membered ring (e.g., a 3-membered, 4-membered, 5-membered, 6-membered, 7-membered, 8-membered, 9-membered, or 10-membered ring), a 4- to 8-membered ring, a 5- to 8-membered ring, or a 5- to 6-membered ring containing 1 to 3 heteroatoms (preferably 1 or 2 heteroatoms) independently selected from the group consisting of sulfur, oxygen, nitrogen, phosphorus, silicon, and/or boron. Non-limiting examples of the heterocyclyl include, but are not limited to, oxiranyl, tetrahydrofuranyl, dihydrofuranyl, pyrrolidinyl, N-methylpyrrolidinyl, dihydropyrrolyl, piperidinyl, piperazinyl, pyrazolidinyl, 4H-pyranyl, morpholinyl, thiomorpholinyl, tetrahydrothienyl, and the like.

The term "spiro ring" refers to a fully saturated or partially unsaturated polycyclic ring system in which monocyclic rings share one carbon atom (referred to as a spiro atom), including carbocyclic rings and heterocyclic rings. Unless otherwise specified, the spiro ring is 5- to 20-membered, preferably 6- to 14- membered, and more preferably 9- to 14-membered. When the spiro ring is a heterocyclic ring, one or more ring atoms in the polycyclic ring are heteroatoms (preferably 1 or 2 heteroatoms) selected from the group consisting of N, O, S(O)ₙ, and P(O)ₙ (wherein n is 0, 1, or 2), and the remaining ring atoms are carbon atoms.

The term "spirocycloalkyl" refers to a fully saturated all-carbon polycyclic ring in which monocyclic rings share one carbon atom (referred to as a spiro atom). Unless otherwise specified, the spirocycloalkyl is 5- to 20-membered, preferably 6- to 14-membered, and more preferably 9- to 14-membered. According to the number of spiro atoms shared among the rings, the spirocycloalkyl is monospirocycloalkyl, bispirocycloalkyl, or polyspirocycloalkyl, preferably monospirocycloalkyl or bispirocycloalkyl, and more preferably 4-membered/4-membered, 4-membered/5-membered, 4-membered/6-membered, 5-membered/5-membered, or 5-membered/6-membered monospirocycloalkyl. Non-limiting examples of the spirocycloalkyl include 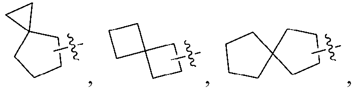 and

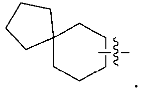

The term "heterospirocycloalkyl" refers to a fully saturated polycyclic ring in which monocyclic rings share one carbon atom (referred to as a spiro atom), wherein one or more ring atoms in the polycyclic ring are heteroatoms (preferably 1 or 2 heteroatoms) selected from the group consisting of N, O, S(O)ₙ, and P(O)ₙ (wherein n is 0, 1, or 2), and the remaining ring atoms are carbon atoms. Unless otherwise specified, the spiro-heterocycloalkyl is 5- to 20-membered, preferably 6- to 14-membered, and more preferably 6- to 10-membered. According to the number of spiro atoms shared among the rings, the spiro heterocyclic ring is a monospiro heterocyclic ring, a bispiro heterocyclic ring, or a polyspiro heterocyclic ring, preferably a monospiro heterocyclic ring or a bispiro heterocyclic ring, and more preferably a 4-membered/4-membered, 4-membered/5-membered, 4-membered/6-membered, 5-membered/5-membered, or 5-membered/6-membered monospiro heterocyclic ring. Non-limiting examples of the spiro-heterocycloalkyl include 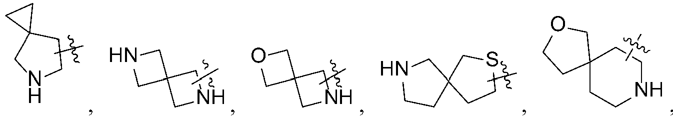 or the like.

The term "bridged ring" refers to a fully saturated or partially unsaturated polycyclic ring system in which two rings share three or more atoms, including carbocyclic rings and heterocyclic rings. Unless otherwise specified, the bridged ring is 5- to 20-membered or 5- to 14-membered, preferably 6- to 14-membered, and more preferably 6- to 10-membered. According to the number of the formed rings, the bridged ring may be a bicyclic, tricyclic, tetracyclic, or polycyclic bridged ring, preferably a bicyclic or tricyclic bridged ring, and more preferably a bicyclic bridged ring. When the bridged ring is a heterocyclic ring, one or more ring atoms in the polycyclic ring are heteroatoms (preferably 1 or 2 heteroatoms) selected from the group consisting of N, O, S(O)ₙ, and P(O)ₙ (wherein n is 0, 1, or 2), and the remaining ring atoms are carbon atoms.

The term "heterocycloalkyl" refers to a fully saturated cyclic group containing heteroatoms. Unless otherwise specified, the heterocycloalkyl is usually a ring containing 1 to 3 heteroatoms (preferably 1 or 2 heteroatoms) independently selected from the group consisting of N, O, S(O)ₙ, and P(O)ₙ (wherein n is 0, 1, or 2). Unless otherwise specified, the heterocycloalkyl may be a monocyclic, bicyclic, or tricyclic group. Unless otherwise specified, the heterocycloalkyl may be a monocyclic ring, a spiro ring, a bridged ring, or a fused ring. Unless otherwise specified, the heterocycloalkyl includes, but is not limited to, a 3- to 20-membered ring, a 3- to 12-membered ring, a 3- to 8-membered ring, or a 5- to 8-membered ring. Examples of 3-membered heterocycloalkyl include, but are not limited to, oxiranyl, thiiranyl, and aziranyl. Non-limiting examples of 4-membered heterocycloalkyl include, but are not limited to, azetidinyl, oxetanyl, and thietanyl. Examples of 5-membered heterocycloalkyl include, but are not limited to, tetrahydrofuranyl, tetrahydrothienyl, pyrrolidinyl, isoxazolidinyl, oxazolidinyl, isothiazolidinyl, thiazolidinyl, imidazolidinyl, and tetrahydropyrazolyl. Examples of 6-membered heterocycloalkyl include, but are not limited to, piperidinyl, tetrahydropyranyl, tetrahydrothiopyranyl, morpholinyl, piperazinyl, 1,4-oxathianyl, 1,4-dioxanyl, thiomorpholinyl, 1,3-dithianyl, or 1,4-dithianyl. Examples of 7-membered heterocycloalkyl include, but are not limited to, azepanyl, oxepanyl, and thiepanyl. Monocyclic heterocycloalkyl having 5 or 6 ring atoms is preferred.

The term "aryl" refers to an all-carbon aromatic monocyclic or fused polycyclic group having a conjugated π-electron system. Unless otherwise specified, aryl may have 6-20 carbon atoms, 6-14 carbon atoms, or 6-12 carbon atoms. Non-limiting examples of the aryl include, but are not limited to, phenyl, naphthyl, anthryl, 1,2,3,4-tetrahydronaphthalene, and the like.

The term "heteroaryl" refers to a monocyclic or polycyclic ring system that contains at least one ring atom selected from the group consisting of N, O, S(O)ₙ, and P(O)ₙ (wherein n is 0, 1, or 2), with the remaining ring atoms being C, and that has at least one aromatic ring. Unless otherwise specified, the heteroaryl may be monocyclic, bicyclic, or tricyclic. Unless otherwise specified, the heteroaryl may have a single 5- to 8-membered ring, or multiple fused rings containing 6 to 20 or 6 to 14, especially 6 to 10 ring atoms. Non-limiting examples of the heteroaryl include, but are not limited to, pyrrolyl, furanyl, thienyl, imidazolyl, oxazolyl, pyrazolyl, pyridinyl, pyrimidinyl, pyrazinyl, quinolyl, isoquinolyl, tetrazolyl, triazolyl, triazinyl, benzofuranyl, benzothienyl, indolyl, isoindolyl, and the like.

The term "treat" or "treatment" means administering the compound or formulation described in the present disclosure to ameliorate or eliminate a disease or one or more symptoms associated with the disease, including:
(i) inhibiting a disease or disease state, i.e., arresting its progression; and
(ii) alleviating a disease or disease state, i.e., causing the regression of the disease or disease state.

The term "therapeutically effective amount" refers to an amount of the compound of the present disclosure for (i) treating the specific disease, condition, or disorder described herein; (ii) alleviating, ameliorating, or eliminating one or more symptoms of the specific disease, condition, or disorder described herein, or (iii) preventing or delaying onset of one or more symptoms of the specific disease, condition, or disorder described herein. The amount of the compound of the present disclosure constituting the "therapeutically effective amount" varies depending on the compound, the disease state and its severity, the mode of administration, and the age of the mammal to be treated, but can be determined routinely by those skilled in the art in accordance with their knowledge and the present disclosure.

The term "pharmaceutically acceptable" is used herein for those compounds, materials, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio.

The pharmaceutically acceptable salt, for example, may be a metal salt, an ammonium salt, a salt formed with an organic base, a salt formed with an inorganic acid, a salt formed with an organic acid, a salt formed with a basic or acidic amino acid, and the like.

The term "pharmaceutical composition" refers to a mixture consisting of one or more of the compounds or the salts thereof of the present disclosure and a pharmaceutically acceptable excipient. The pharmaceutical composition is intended to facilitate the administration of the compound of the present disclosure to an organism.

The term "pharmaceutically acceptable excipient" refers to those that do not have a significant irritating effect on an organism and do not impair the biological activity and properties of the active compound. Suitable excipients are well known to those skilled in the art, such as carbohydrates, wax, water-soluble and/or water-swellable polymers, hydrophilic or hydrophobic materials, gelatin, oil, solvents, and water.

The word "comprise" and variations thereof such as "comprises" or "comprising" should be understood in an open, non-exclusive sense, i.e., "including but not limited to".

Unless otherwise specified, terms in the singular form shall be deemed to include the plural form and vice versa. Unless otherwise specified, the word "a" or "an" refers to "at least one". Unless otherwise stated, "or" is used to mean "and/or".

The compounds of the present disclosure may have particular geometric or stereoisomeric forms. All such compounds are contemplated in the present disclosure, including *cis* and *trans* isomers, (-)- and (+)-enantiomers, (*R*)*-* and (*S*)-enantiomers, diastereoisomers, (D)-isomers, (L)-isomers, and racemic mixtures and other mixtures thereof, such as enantiomerically or diastereoisomerically enriched mixtures, all of which are encompassed within the scope of the present disclosure. Additional asymmetric carbon atoms may be present in substituents such as alkyl. All such isomers and mixtures thereof are encompassed within the scope of the present disclosure.

Unless otherwise stated, "(*D*)" or "(+)" stands for dextrorotation, "(*L*)" or "(-)" stands for levorotation, and "(*DL*)" or "(+)" stands for racemization.

Unless otherwise stated, the absolute configuration of a stereogenic center is represented by a wedged solid bond ( 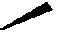 ) and a wedged dashed bond ( 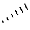 ), and the relative configuration of a stereogenic center is represented by a straight solid bond ( 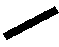 ) and a straight dashed bond ( 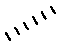 ).

Optically active (*R*)*-* and (*S*)-isomers and *D* and *L* isomers can be prepared by chiral synthesis, chiral reagents, or other conventional techniques. An enantiomer of a certain compound of the present disclosure can be prepared by asymmetric synthesis or derivatization using a chiral additive, wherein the resulting diastereoisomeric mixture is separated and the auxiliary group is cleaved so as to provide the desired pure enantiomer. Alternatively, when the molecule comprises a basic functional group (e.g., amino) or an acidic functional group (e.g., carboxyl), the compound reacts with an appropriate optically active acid or base to form a salt of the diastereoisomer, which is then subjected to diastereoisomeric resolution through conventional methods well known in the art to give the pure enantiomer. Furthermore, the enantiomer and the diastereoisomer are generally separated through chromatography using a chiral stationary phase, optionally in combination with chemical derivatization (e.g., carbamate generated from amines).

The present disclosure also includes isotopically labeled compounds of the present disclosure, which are identical to those recited herein but have one or more atoms replaced by an atom having an atomic mass or mass number different from the atomic mass or mass number generally found in nature. Examples of isotopes that can be incorporated into the compounds of the present disclosure include isotopes of hydrogen, carbon, nitrogen, oxygen, phosphorus, sulfur, fluorine, iodine, and chlorine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ¹²³I, ¹²⁵I, ³⁶Cl, and the like.

Certain isotopically labeled compounds of the present disclosure (e.g., those labeled with ³H and ¹⁴C) can be used to analyze compounds and/or substrate tissue distribution. Tritium (i.e., ³H) and carbon-14 (i.e., ¹⁴C) isotopes are particularly preferred for their ease of preparation and detectability. Positron emitting isotopes, such as ¹⁵O, ¹³N, ¹¹C, and ¹⁸F, can be used in positron emission tomography (PET) studies to determine substrate occupancy. The isotopically labeled compounds of the present disclosure can generally be prepared by following procedures analogous to those disclosed in the schemes and/or examples below while substituting a non-isotopically labeled reagent with an isotopically labeled reagent.

Furthermore, substitution with heavier isotopes such as deuterium (i.e., ²H or D) may afford certain therapeutic advantages resulting from greater metabolic stability (for example, increased *in vivo* half-life or reduced dosage requirements) and hence may be preferred in some circumstances in which deuterium substitution may be partial or complete; partial deuterium substitution refers to substitution of at least one hydrogen with at least one deuterium, and complete deuterium substitution refers to substitution of all hydrogens of the group with deuterium, for example, methyl (-CH₃) completely substituted with deuterium is -CD₃.

The compounds of the present disclosure may be present in their tautomeric forms, and all such forms are included within the scope of the present disclosure. The term "tautomer" or "tautomeric form" refers to structural isomers of different energies that can interconvert via a low energy barrier. For example, a proton tautomer (also referred to as a prototropic tautomer) includes interconversion via proton transfer, such as keto-enol and imine-enamine isomerizations. A specific example of a proton tautomer may be an imidazole moiety in which a proton can transfer between two ring nitrogens.

The pharmaceutical composition of the present disclosure can be prepared by combining the compound of the present disclosure with a suitable pharmaceutically acceptable excipient.

Typical routes of administration of the compound or the pharmaceutically acceptable salt thereof or the pharmaceutical composition thereof of the present disclosure include, but are not limited to, oral, local, inhalation, parenteral, intranasal, intraocular, intramuscular, subcutaneous, and intravenous administration.

The pharmaceutical composition of the present disclosure can be manufactured using methods well known in the art, such as conventional mixing, dissolving, granulating, dragee-making, levigating, emulsifying, lyophilizing, etc.

In all of the administration methods of the compound of (I) disclosed herein, the daily dose administered is from 0.001 mg/kg body weight to 2000 mg/kg body weight, given in individual or separated doses.

The compounds of the present disclosure can be prepared using a variety of synthetic methods well known to those skilled in the art, including the specific embodiments listed below, embodiments formed by combinations thereof with other chemical synthetic methods, and equivalent substitutions well known to those skilled in the art; preferred embodiments include, but are not limited to, the examples of the present disclosure.

The chemical reactions in the specific embodiments of the present disclosure are conducted in a suitable solvent that must be suitable for the chemical changes in the present disclosure and the reagents and materials required. In order to obtain the compounds of the present disclosure, it is sometimes necessary for those skilled in the art to modify or select a synthetic procedure or a reaction process based on the existing embodiments.

An important consideration in synthetic route planning in the art is the selection of suitable protective groups for reactive functional groups (e.g., amino in the present disclosure); for example, reference may be made to Greene's Protective Groups in Organic Synthesis (4th Ed.) Hoboken, New Jersey: John Wiley & Sons, Inc, and all references cited herein are incorporated by reference in their entirety.

All patents, patent applications, and other identified publications are explicitly incorporated herein by reference for the purpose of description and disclosure. These publications are provided solely because they were disclosed prior to the filing date of the present disclosure. All statements as to the dates of these documents or descriptions as to the content of these documents are based on the information available to the applicant and do not constitute any admission as to the correctness of the dates or the content of these documents. Moreover, in any country or region, any reference to these publications herein shall not be construed as an admission that the publications form part of the commonly recognized knowledge in the art.

### The following abbreviations are used in the present disclosure:

DCM represents dichloromethane; PE represents petroleum ether; THF represents tetrahydrofuran; EA represents ethyl acetate; EDCI represents 1-ethyl-3-(3-dimethylaminopropyl)carbodiimide hydrochloride; HOBt represents 1-hydroxybenzotriazole; DMF represents N,N-dimethylformamide; TEA represents triethylamine; m-CPBA represents m-chloroperoxybenzoic acid; Pd(dppf)Cl₂ represents [1,1'-bis(diphenylphosphino)ferrocene]palladium(II) dichloride; DBU represents 1,8-diazabicyclo[5.4.0]undec-7-ene; DMAP represents 4-dimethylaminopyridine.

### DETAILED DESCRIPTION

For clarity, the present disclosure is further described with the following examples, which are, however, not intended to limit the scope of the present disclosure. It will be apparent to those skilled in the art that various changes and modifications can be made to the specific embodiments of the present disclosure without departing from the spirit and scope of the present disclosure. All the reagents used in the present disclosure are commercially available and can be used without further purification.

The compounds of the present disclosure can be obtained by similar preparation methods to those in the examples, including but not limited to adjustment of structurally similar starting materials, reagents, or process parameters. The structural characterization of the compounds of the present disclosure can be obtained by MS or HNMR. The results for the compounds of the present disclosure can also be obtained by the same effect test method.

### Example 1: Preparation of Compound 1

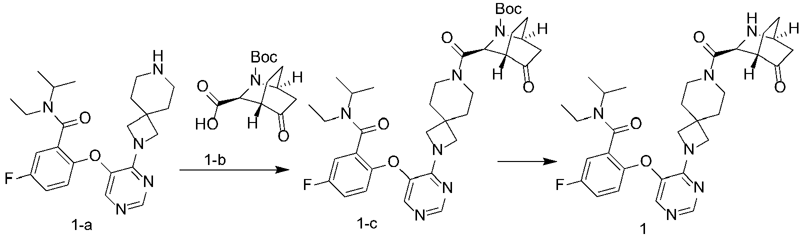

### Step 1: Preparation of compound 1-c

Compound 1-a (35 mg), compound 1-b (22 mg), EDCI (23 mg), HOBt (17 mg), triethylamine (25 mg), and DMF (2 mL) were added to a reaction flask, and the mixture was stirred at room temperature overnight. After the reaction was completed as detected, ethyl acetate and saturated brine were added, and the mixture was stirred and subjected to phase separation. The aqueous phase was extracted with ethyl acetate, and the organic phases were combined, washed with saturated brine, dried, filtered, and concentrated under reduced pressure to give compound 1-c (50 mg), which was directly used in the next step without purification. ESI-MS: m/z = 679.44 [M+H]⁺.

### Step 2: Preparation of compound 1

Compound 1-c (50 mg) was added to a reaction flask containing dichloromethane (6 mL) and dissolved by stirring, and trifluoroacetic acid (2 mL) was slowly added dropwise. After the dropwise addition was completed, the mixture was stirred at room temperature. After the reaction was completed as detected, water was added, and the pH was adjusted to about 8 with TEA, followed by phase separation. The aqueous phase was extracted with DCM. The organic phases were combined, dried, filtered, concentrated, and purified and separated by preparative liquid phase chromatography to give compound 1 (23 mg). ESI-MS: m/z = 579.28 [M+H]⁺.

¹H-NMR (DMSO-*d*₆): *δ*_{H} 1.24-1.84 (11H, m), 1.90 (6H, m), 2.28-2.46 (3H, m), 3.13-3.53 (6H, m), 3.74 (1H, m), 3.94-3.84 (4H, m), 4.21-4.16 (1H, m), 4.41 (1H, m), 7.06 (1H, m), 7.23-7.31 (2H, m), 7.67-7.74 (1H, m), 8.26-8.28 (1H, m).

### Example 2: Preparation of Compound 2

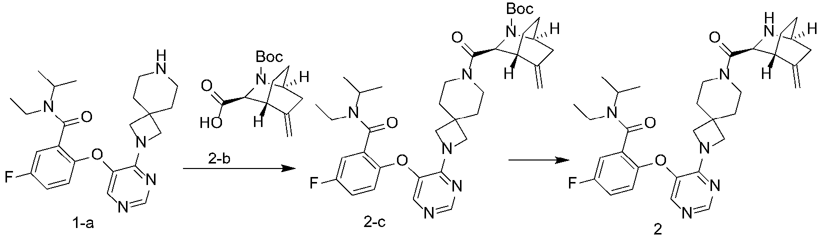

### Step 1: Preparation of compound 2-c

Referring to the preparation method for compound 1-c in Example 1, compound 1-b was replaced by compound 2-b to give compound 2-c. ESI-MS: m/z = 677.47 [M+H]⁺.

### Step 2: Preparation of compound 2

Referring to the preparation method for compound 1-d in Example 1, compound 1-c was replaced by compound 2-c to give compound 2. ESI-MS: m/z = 577.30 [M+H]⁺.

¹H-NMR (CDCl₃): *δ*_{H} 1.08-1.90 (11H, m), 1.99 (6H, m), 2.38-2.45 (2H, m), 2.92 (1H, d, J = 17.81 Hz), 3.17-4.10 (12H, m), 4.66 (1H, m), 4.91 (1H, s), 5.01 (1H, d, J = 11.75 Hz), 6.74 (1H, m), 7.02-6.99 (2H, m), 7.84 (1H, m), 8.41 (1H, d, J = 5.30 Hz).

### Example 3: Preparation of Compound 3

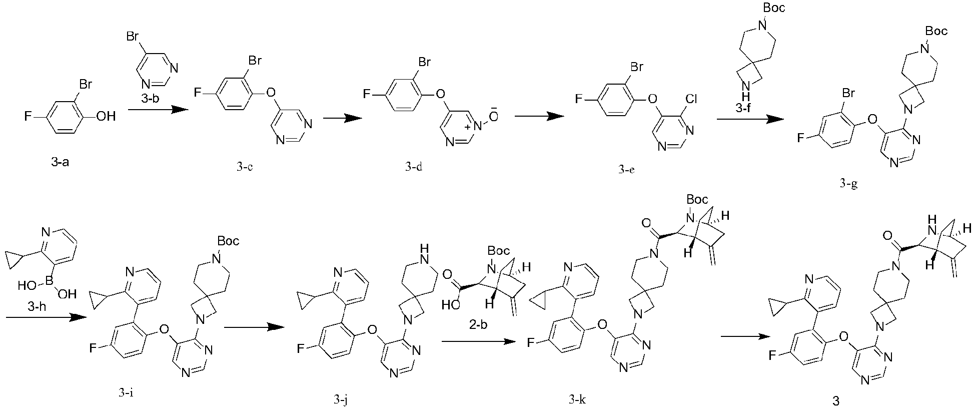

### Step 1: Preparation of compound 3-c

3-a (1000 mg), 3-b (832 mg), Cs₂CO₃ (3.42 mg), and DMF (10 mL) were added to a 25 mL reaction flask, and the mixture was stirred at 100 °C for 72 h. After most of the starting materials were consumed completely, the reaction solution was filtered, and the filter cake was washed with DMF. The filtrates were combined, purified by silica gel column chromatography (PE:EA = 4:1), and concentrated to give compound 3-c (800 mg). ESI-MS: m/z = 268.84 [M+H]⁺.

### Step 2: Preparation of compound 3-d

3-c (800 mg) and DCM (10 mL) were added to a reaction flask, and m-CPBA (1.54 g) was added in an ice bath. After the addition was completed, the mixture was stirred at room temperature for 24 h until the reaction was completed. A NaHSO₃ solution and a NaHCO₃ solution were added to the reaction solution, and the resulting mixture was stirred and subjected to phase separation. The organic phase was washed with a NaHCO₃ solution, dried, filtered, and concentrated to dryness to give compound 3-d (422 mg). ESI-MS: m/z = 284.90 [M+H]⁺.

### Step 3: Preparation of compound 3-e

POCl₃ (341 mg) was added to a solution of Et₃N (225 mg) in CHCl₃ (10 mL) in an ice bath. The mixed solution was added dropwise to a solution of 3-d (422 mg) in CHCl₃ (5 mL) in an ice bath. After the dropwise addition was completed, the resulting mixture was stirred at 65 °C for 4 h until the starting materials were consumed completely. A NaHCO₃ solution was slowly added to adjust the pH to 7-8, followed by phase separation. The organic phase was purified by silica gel column chromatography (PE:EA = 4:1) to give compound 3-e (180 mg). ESI-MS: m/z = 302.93 [M+H]⁺.

### Step 4: Preparation of compound 3-g

3-e (180 mg), 3-f (134 mg), Na₂CO₃ (126 mg), and acetonitrile (10 mL) were added to a 5 mL reaction flask, and the mixture was stirred at room temperature for 12 h until the reaction was completed. The reaction solution was filtered, and the filtrate was concentrated to dryness to give compound 3-g (100 mg). ESI-MS: m/z = 493.13 [M+H]⁺.

### Step 5: Preparation of compound 3-i

3-g (100 mg), 3-h (33 mg), Pd(dppf)Cl₂ (15 mg), K₂CO₃ (56 mg), 1,4-dioxane (5 mL), and H₂O (1 mL) were added to a reaction flask, and the mixture was stirred at 80 °C for 7 h under a N2 atmosphere until the reaction was completed. Water and ethyl acetate were added to the reaction solution, followed by phase separation. The organic phase was purified by silica gel column chromatography (PE:EA = 2:1) to give compound 3-i (100 mg). ESI-MS: m/z = 532.27 [M+H]⁺.

### Step 6: Preparation of compound 3-j

3-i (100 mg) was added to a reaction flask containing DCM (6 mL) and dissolved by stirring, and CF₃COOH (2 mL) was slowly added dropwise. After the dropwise addition was completed, the mixture was stirred at room temperature until the reaction was completed. Water was added, and the pH was adjusted to about 8 with TEA, followed by phase separation. The aqueous phase was extracted with DCM, and the organic phases were combined, dried, filtered, and concentrated until no liquid flowed out to give compound 3-j (60 mg). ESI-MS: m/z = 432.25[M+H]⁺.

### Step 7: Preparation of compound 3-k

Referring to the preparation method for compound 1-c in Example 1, compound 1-a was replaced by compound 3-j, and compound 1-b was replaced by compound 2-b, thus giving compound 3-k. ESI-MS: m/z = 681.41 [M+H]⁺.

### Step 8: Preparation of compound 3

Referring to the preparation method for compound 1-d in Example 1, compound 1-c was replaced by compound 3-k to give compound 3. ESI-MS: m/z = 581.42 [M+H]⁺. ¹H-NMR (CDCl₃): δ_{H} 8.45 (1H, dd, J = 1.40, 4.70 Hz), 8.28-8.30 (3H, m), 7.68 (1H, s), 7.37 (1H, dd, J = 1.50), 7.12-7.15 (2H, m), 7.01-7.07 (2H, m), 3.78-3.90 (6H, m), 3.47 (1H, m), 3.23-3.33 (2H, m), 2.97 (1H, d, J = 18.26 Hz), 2.51 (1H, s), 2.44 (1H, dd, J = 1.70, 18.11 Hz), 1.66-2.06 (10H, m), 1.07 (2H, s), 0.83 (2H, m).

### Example 4: Preparation of Compound 4

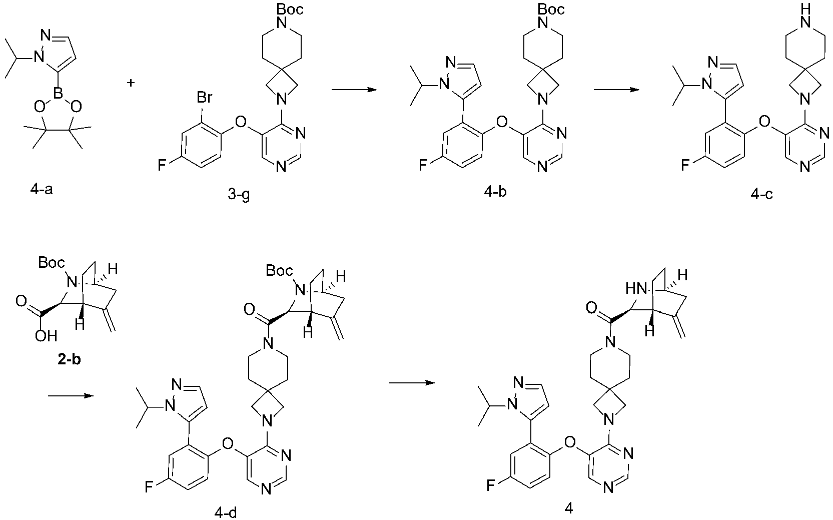

### Step 1: Preparation of compound 4-b

Referring to the preparation method for compound 3-i in Example 3, compound 3-h was replaced by compound 4-a to give compound 4-b. ESI-MS: m/z = 523.3 [M+H]⁺.

### Step 2: Preparation of compound 4-c

Referring to the preparation method for compound 3-j in Example 3, compound 3-i was replaced by compound 4-b to give compound 4-c.

### Step 3: Preparation of compound 4-d

Referring to the preparation method for compound 1-c in Example 1, compound 1-a was replaced by compound 4-c, and compound 1-b was replaced by compound 2-b, thus giving compound 4-d.

### Step 4: Preparation of compound 4

Referring to the preparation method for compound 1-d in Example 1, compound 1-c was replaced by compound 4-d to give compound 4. ESI-MS: m/z = 572.28 [M+H]⁺. ¹H-NMR (DMSO-d₆): δ_{H} 7.41-7.47 (3H, m), 6.89 (1H, s), 6.77 (1H, d), 6.49 (1H, dt, J = 3.03), 6.42 (1H, dd, J = 3.08), 6.32 (1H, dd, J = 4.50), 5.48 (1H, d, J = 1.70 Hz), 3.72 (1H, s), 3.60 (1H, m), 3.07-3.11 (4H, m), 2.60-2.94 (4H, m), 2.03 (1H, d, J = 18.16 Hz), 1.90 (1H, s), 1.79 (1H, d, J = 17.91 Hz), 1.29 (1H, m), 1.04 (2H, t, J = 4.88 H), 0.98 (2H, t, J = 5.43 Hz), 0.91 (4H, m), 0.58 (6H, d, J = 6.60 Hz).

### Example 5: Preparation of Compound 5

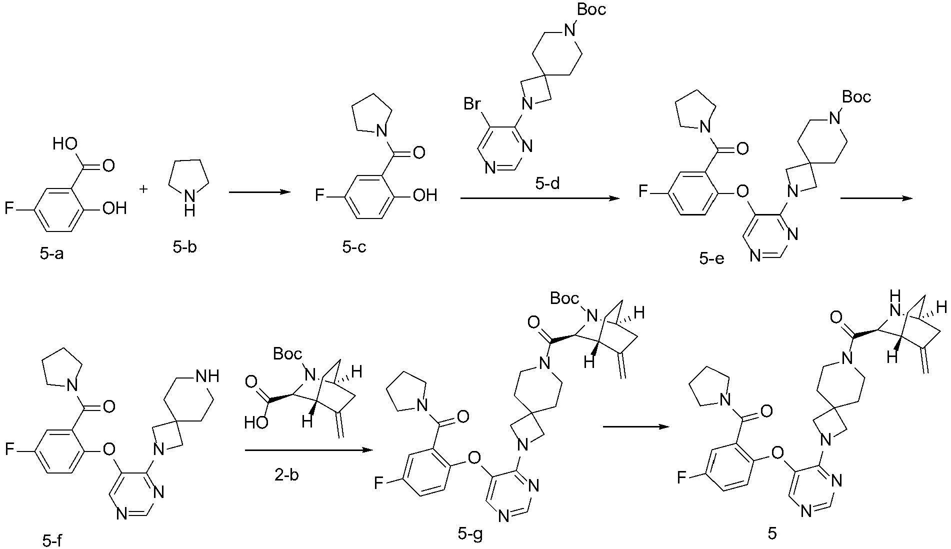

### Step 1: Preparation of compound 5-c

Compound 5-a (200 mg), compound 5-b (91 mg), EDCI (368 mg), HOBt (259 mg), triethylamine (258 mg), and DMF (3 mL) were added to a reaction flask, and the mixture was stirred at room temperature overnight. After the reaction was completed as detected, ethyl acetate and saturated brine were added, and the mixture was stirred and subjected to phase separation. The aqueous phase was extracted with ethyl acetate, and the organic phases were combined, washed with saturated brine, dried, filtered, and concentrated under reduced pressure to give intermediate 5-c (121 mg). ESI-MS: m/z = 210.05 [M+H]⁺.

### Step 2: Preparation of compound 5-e

Intermediate 5-c (100 mg), compound 5-d (184 mg), copper iron oxide (58 mg), acetylacetone (1 drop), cesium carbonate (313 mg), and DMF (3 mL) were added to a reaction flask. The mixture was purged with N₂, heated to 120 °C, and stirred for 20 h. After the reaction was completed as detected, the mixture was purified by silica gel column chromatography to give intermediate 5-e (40 mg). ESI-MS: m/z = 512.27 [M+H]⁺.

### Step 3: Preparation of compound 5-f

Intermediate 5-e (40 mg), trifluoroacetic acid (1 mL), and dichloromethane (3 mL) were added to a reaction flask, and the mixture was stirred at room temperature for 1 h. After the reaction was completed as detected, an aqueous NaHCO₃ solution was added to adjust the pH to neutral, and the resulting mixture was stirred and subjected to phase separation. The organic phase was concentrated under reduced pressure to give intermediate 5-f (40 mg). ESI-MS: m/z = 412.25 [M+H]⁺.

### Step 4: Preparation of compound 5-g

Intermediate 5-f (40 mg), intermediate 2-b (26 mg), EDCI (28 mg), HOBt (20 mg), triethylamine (20 mg), and DMF (3 mL) were added to a reaction flask, and the mixture was stirred at room temperature overnight. After the reaction was completed as detected, ethyl acetate and saturated brine were added, and the mixture was stirred and subjected to phase separation. The aqueous phase was extracted with ethyl acetate, and the organic phases were combined, washed with saturated brine, dried, filtered, and concentrated under reduced pressure to give intermediate 5-g (50 mg). ESI-MS: m/z = 661.44 [M+H]⁺.

### Step 5: Preparation of compound 5

Intermediate 5-g (40 mg), trifluoroacetic acid (1 mL), and dichloromethane (3 mL) were added to a reaction flask, and the mixture was stirred at room temperature for 1 h. After the reaction was completed as detected, the mixture was stirred and subjected to phase separation. The organic phase was concentrated under reduced pressure and purified and separated by silica gel column chromatography to give compound 5 (10 mg). ESI-MS: m/z = 561.28 [M+H]⁺.

¹H-NMR (CDCl₃): δ_{H} 1.21-2.05 (13H), 2.43 (1H, dd, J = 1.58, 18.15 Hz), 2.50 (1H, s), 2.96 (1H, d, J = 18.11 Hz), 3.27-3.38 (4H), 3.48 (1H, m), 3.56 (2H, t, J = 6.88 Hz), 3.81 (1H, s), 3.89 (1H, m), 3.98-4.02 (4H), 6.79 (1H, dd, J = 4.01, 8.86 Hz), 7.03-7.09 (2H), 7.84 (1H, s), 8.39 (3H).

### Examples 6-7

Referring to the preparation method for compound **5** in Example 5, compound 5-b was replaced by the starting compounds shown in the table below to give target compounds 6 and 7.

| Example | Starting compound | Structures of compounds 6-7 | ESI-MS: m/z [M+H]⁺ | ¹H-NMR |
|---|---|---|---|---|
| 6 | 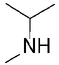 | 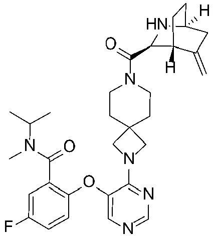 | 563.27 | ¹H NMR (500MHz CDCl₃) δ_{H} 8.40 (1H, m), 7.83 (1H, m), 7.03 (1H, m), 6.82 (1H, m), 6.70 (1H, m), 5.05 (1H, s), 4.95 (1H, s), 4.75 (1H, s), 3.98 (6H, m), 3.47 (1H, m), 3.33 (2H, m), 2.95 (3H, s), 2.73 (1H, s), 2.51 (1H, s), 2.43 (1H, d, J = 18.0), 2.51 (1H, m), 2.45 (1H, d, J = 18.1), 2.04 (1H, m), 1.93 (1H, m), 1.83 (3H, m), 1.68 (3H, m), 1.16 (6H, d, J = 6.6). |
| 7 | 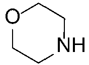 | 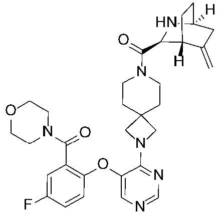 | 577.31 | |

### Example 8: Preparation of Compound 8

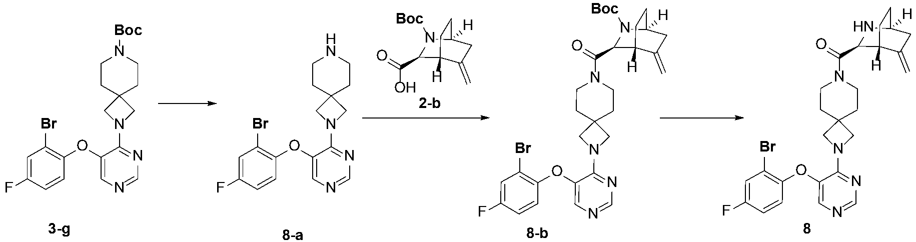

### Step 1: Preparation of compound 8-a

Intermediate 3-g (120 mg), trifluoroacetic acid (1 mL), and dichloromethane (3 mL) were added to a reaction flask, and the mixture was stirred at room temperature for 1 h. After the reaction was completed as detected, an aqueous NaHCO₃ solution was added to adjust the pH to neutral, and the resulting mixture was stirred and subjected to phase separation. The organic phase was concentrated under reduced pressure to give intermediate 8-a (100 mg).

### Step 2: Preparation of compound 8-b

Intermediate 8-a (100 mg), intermediate 2-b (68 mg), EDCI (52 mg), HOBt (73 mg), triethylamine (76 mg), and DMF (8 mL) were added to a reaction flask, and the mixture was stirred at room temperature overnight. After the reaction was completed as detected, ethyl acetate and saturated brine were added, and the mixture was stirred and subjected to phase separation. The aqueous phase was extracted with ethyl acetate, and the organic phases were combined, washed with saturated brine, dried, filtered, and concentrated under reduced pressure to give intermediate 8-b (80 mg).

### Step 3: Preparation of compound 8

Intermediate 8-b (80 mg), trifluoroacetic acid (1 mL), and dichloromethane (3 mL) were added to a reaction flask, and the mixture was stirred at room temperature for 1 h. After the reaction was completed as detected, the mixture was stirred and subjected to phase separation. The organic phase was concentrated under reduced pressure and purified and separated by silica gel column chromatography to give compound 8 (30 mg). ESI-MS: m/z = 542.10 [M+H]⁺.

¹H-NMR (CDCl₃): δ_{H} 1.59-1.98 (8H, m, J = 7.50 Hz), 2.40 (1H, d, J = 17.91 Hz), 2.44 (1H, s), 2.91 (1H, d, J = 17.91 Hz), 3.32 (1H, m), 3.42 (1H, m), 3.48 (1H, m), 3.67 (1H, m), 3.85 (1H, m), 4.06 (4H, s), 4.66 (1H, s), 4.91 (1H, s), 5.00 (1H, s), 6.86 (1H, dd, J = 4.78, 8.98 Hz), 7.03 (1H, m, J = 4.84 Hz), 7.40 (1H, dd, J = 2.88, 7.53 Hz), 7.69 (1H, s), 8.40 (1H, s).

### Example 9: Preparation of Compound 9

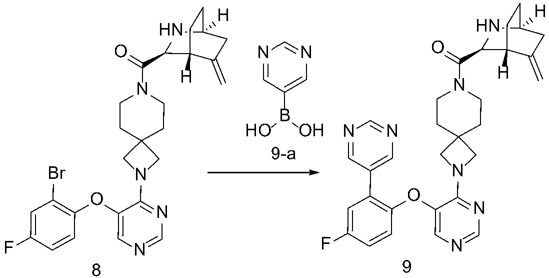

Compound 8 (54 mg), 9-a (12 mg), Pd(dppf)Cl₂ (7 mg), K₂CO₃ (27 mg), 1,4-dioxane (5 mL), and H₂O (1 mL) were added to a reaction flask. The mixture was stirred at 80 °C for 7 h under a N₂ atmosphere. After the starting materials were consumed completely as detected, water and ethyl acetate were added to the reaction solution, followed by phase separation. The organic phase was separated and purified by silica gel column chromatography to give compound 9 (23 mg). ESI-MS: m/z = 542.21 [M+H]⁺.

¹H-NMR (CDCl₃): δ_{H} 1.74-2.11 (8H, m), 2.52 (1H, d, J = 18.31 Hz), 2.57 (1H, s), 2.97 (1H, d, J = 18.41 Hz), 3.34 (2H, m), 3.54 (1H, m), 3.93 (1H, s), 4.02-4.23 (5H, m), 4.77 (1H, s), 5.02 (1H, s), 5.11 (1H, s), 7.20 (1H, dd, J = 4.30, 8.70 Hz), 7.24-7.32 (2H, m), 7.61 (1H, m), 8.40 (1H, s), 8.97 (2H, s), 9.27 (1H, s), 10.84 (1H, m).

### Examples 10-23

Referring to the preparation method for compound 9 in Example 9, compound 9-a was replaced by the starting compounds shown in the table below to give target compounds 10-23.

| Exampl e | Starting compound | Structures of compounds 10-23 | ESI-MS: m/z [M+H]⁺ | ¹H-NMR |
|---|---|---|---|---|
| 10 | 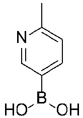 | 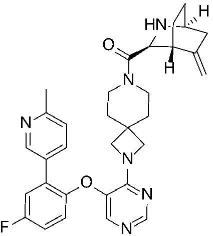 | 555.31 | |
| 11 | 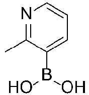 | 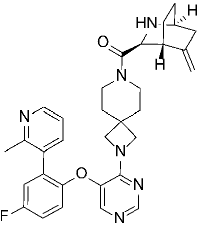 | 555.27 | ¹H NMR (500MHz DMSO-*d*₆) *δ*_{H} 8.47 (1H, dd, *J =* 4.8, 1.6), 8.21 (1H, s), 7.70 (1H, s), 7.65 (1H, dd, *J =* 7.7, 1.6), 7.30 (3H, m), 7.13 (1H, m), 5.10 (1H, s), 4.87 (1H, s), 4.39 (1H, s), 3.74 (4H, m), 3.58 (1H, m), 3.50 (1H, m), 3.42 (1H, m), 3.31 (2H, m), 2.68 (1H, m), 2.56 (1H, brs), 2.41 (1H, m), 2.34 (3H, s), 1.88 (1H, m), 1.70 (4H, m), 1.54 (3H, m). |
| 12 | 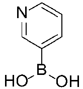 | 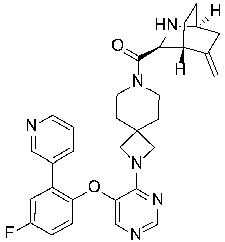 | 541.29 | ¹H NMR (500MHz DMSO-*d*₆) *δ*_{H} 8.76 (1H, d, *J =* 1.8), 8.59 (1H, dd, *J =* 4.6, 1.1), 8.25 (1H, m), 7.99 (1H, m), 7.73 (1H, s), 7.48 (2H, m), 7.30 (1H, td, *J =* 8.8, 3.1), 7.10 (1H, dd, *J* = 9.1, 4.8), 5.06 (1H, s), 4.85 (1H, s), 4.33 (1H, s), 3.84 (4H, m), 3.59 (1H, m), 3.38 (4H, m), 2.66 (1H, m), 2.51 (1H, m), 2.38 (1H, m), 1.85 (1H, m), 1.75 (4H, m), 1.53 (3H, m). |
| 13 | 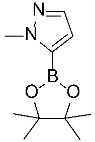 | 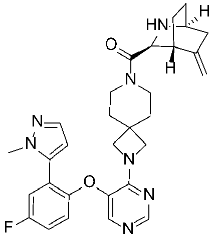 | 544.22 | ¹H NMR (500MHz CDCl₃) δ_{H} 8.30 (1H, m), 7.75 (1H, s), 7.52 (1H, s), 7.15 (2H, m), 6.95 (1H, dd, J = 8.9, 4.5), 6.24 (1H, m), 5.06 (1H, s), 4.96 (1H, s), 4.78 (1H, s), 3.82 (9H, m), 3.71 (1H, m), 3.47 (1H, m), 3.29 (2H, m), 2.97 (1H, d, J = 18.0), 2.51 (1H, s), 2.45 (1H, d, J = 18.0), 2.07 (1H, m), 1.91 (1H, m), 1.73 (6H, m). |
| 14 | 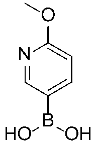 | 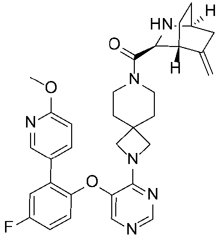 | 571.21 | ¹H-NMR(CDCl₃)δ_{H} 1.61~1.91(8H), 2.37~2,41 (2H), 2.88 (1H, d, J=17.61 Hz), 3.25~3.82 (5H, m), 3.90(4H, m), 3.97(3H, s), 4.58 (1H, m), 4.89 (1H, s), 4.99 (1H, s), 6.80 (1H, d, J=8.55 Hz), 6.90 (1H, dd, J=4.55, 8.80 Hz), 7.05 (1H, m), 7.14 (1H, dd, J=2.65, 8.55 Hz), 7.67~7.68 (2H), 8.28 (1H, d, J=1.40 Hz), 8.33 (1H, s). |
| 15 | 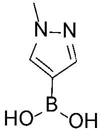 | 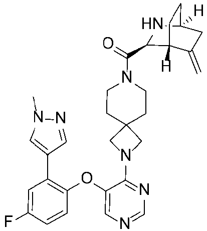 | 544.31 | ¹H NMR (500MHz CDCl₃) δ_{H} 8.41 (1H, s), 8.30 (2H, s), 7.83 (1H, s), 7.75 (2H, m), 7.28 (1H, m), 6.88 (1H, m), 6.72 (1H, dd, J = 9.0, 5.0), 5.03 (1H, s), 4.94 (1H, s), 4.76 (1H, s), 3.95 (8H, m), 3.76 (1H, s), 3.48 (1H, m), 3.28 (2H, m), 2.95 (1H, m), 2.44 (2H, m), 1.83 (3H, m), 1.70 (1H, m), 1.64 (1H, m), 1.27 (2H, m). |
| 16 | 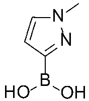 | 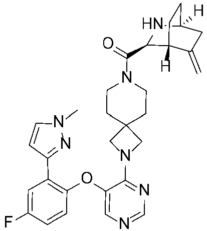 | 544.28 | ¹H NMR (500MHz CDCl₃) δ_{H} 8.38 (1H, s), 8.31 (1H, s), 7.78 (1H, m), 7.73 (1H, s), 7.38 (1H, s), 6.95 (1H, m), 6.77 (1H, m), 6.63 (1H, s), 5.02 (1H, s), 4.93 (1H, s), 4.75 (1H, s), 3.96 (3H, s), 3.89 (1H, m), 3.75 (1H, s), 3.47 (1H, m), 3.29 (2H, m), 2.95 (1H, d, J = 17.0), 2.44 (2H, m), 1.83 (10H, m), 1.31 (2H, m) |
| 17 | 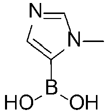 | 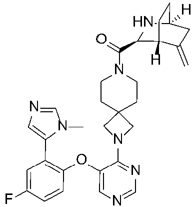 | 544.37 | ¹H NMR (500MHz CDCl₃) δ_{H} 8.37 (1H, s), 7.77 (1H, s), 7.60 (1H, s), 7.11 (3H, m), 6.87 (1H, dd, J = 9.5, 4.5), 4.99 (1H, s), 4.89 (1H, s), 4.47 (1H, m), 3.82 (1H, m), 3.61 (3H, s), 3.57 (1H, m), 3.35 (2H, m), 3.24 (2H, m), 2.97 (1H, m), 2.46 (1H, m), 2.41 (2H, m), 2.36 (1H, m), 1.69 (10H, m). |
| 18 | 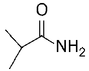 | 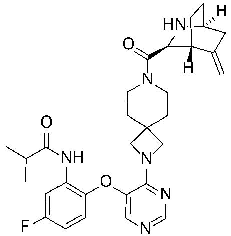 | 549.34 | ¹H NMR (500MHz CDCl₃) δ_{H} 8.45 (1H, s), 8.28 (3H, m), 7.86 (1H, s), 7.71 (1H, s), 6.72 (1H, m), 6.62 (1H, dd, J = 9.0, 5.0), 5.04 (1H, s), 4.95 (1H, s), 4.80 (1H, s), 3.96 (4H, m), 3.79 (1H, m), 3.52 (1H, m), 3.48 (1H, s), 3.27 (2H, m), 2.95 (1H, m), 2.59 (1H, m), 2.50 (1H, m), 2.43 (1H, m), 2.00 (2H, m), 1.84 (3H, m), 1.71 (1H, m), 1.64 (1H, m), 1.27 (6H, d, J = 7.0). |
| 19 | 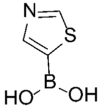 | 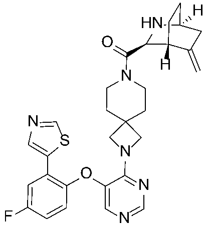 | 547.24 | |
| 20 | 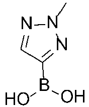 | 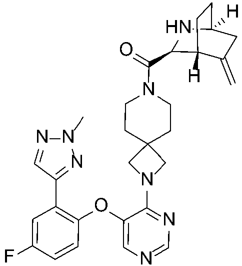 | 545.29 | ¹H NMR (500MHz CDCl₃) δ_{H} 8.42 (1H, s), 8.21 (2H, m), 7.93 (1H, s), 7.80 (2H, m), 7.01 (1H, m), 6.78 (1H, dd, J = 9.0, 4.5), 5.06 (1H, s), 4.96 (1H, s), 4.81 (1H, s), 4.26 (3H, s), 3.99 (4H, m), 3.85 (1H, m), 3.48 (1H, m), 3.30 (2H, m), 2.96 (1H, d, J = 18.0), 2.47 (2H, m), 2.04 (1H, m), 1.93 (1H, m), 1.84 (3H, m), 1.69 (3H, m). |
| 21 | 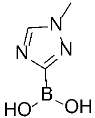 | 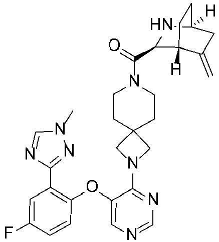 | 545.28 | ¹H NMR (500MHz CDCl₃) δ_{H} 8.40 (1H, s), 8.33 (1H, s), 7.68 (1H, s), 7.40 (1H, dd, J = 7.5, 2.5), 7.04 (1H, m), 6.87 (1H, dd, J = 9.0, 4.5), 5.05 (1H, s), 4.96 (1H, s), 4.75 (1H, s), 4.07 (4H, brs), 3.92 (2H, s), 3.83 (1H, s), 3.50 (1H, m), 3.35 (2H, m), 3.08 (1H, d, J = 17.5), 2.46 (2H, m), 2.07(1H, m), 1.98 (2H, m), 1.88 (4H, m), 1.69 (4H, m). |
| 22 | 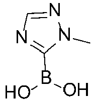 | 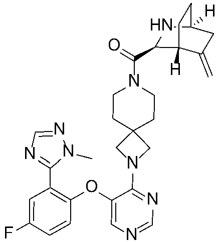 | 545.29 | ¹H NMR (500MHz CDCl₃) δ_{H} 8.39 (1H, s), 8.27 (2H, brs), 7.67 (1H, s), 7.41 (1H, dd, J = 7.5, 2.5), 7.04 (1H, m), 6.88 (1H, dd, J = 9.0, 4.5), 5.06 (1H, s), 4.96 (1H, s), 4.78 (1H, s), 4.08 (4H, brs), 3.91 (2H, m), 3.72 (1H, s), 3.52 (1H, m), 3.35 (2H, m), 3.02 (1H, d, J = 18.0), 2.47 (2H, m), 1.98 (6H, m), 1.70 (4H, m). |
| 23 | 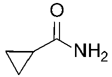 | 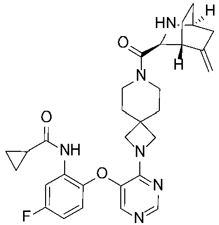 | 547.25 | |

### Example 24: Preparation of Compound 24

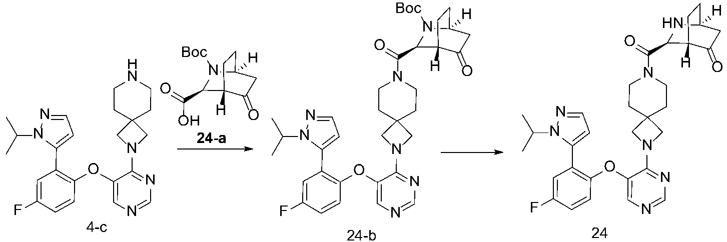

### Step 1: Preparation of compound 24-b

Referring to the preparation method for compound 4-d in Example 4, compound 2-b was replaced by compound 24-a to give compound 24-b.

### Step 2: Preparation of compound 24

Referring to the preparation method for compound 4 in Example 4, compound 24 was obtained. ESI-MS: m/z = 574.28[M+H]⁺. ¹H NMR (500 MHz CD₃CN): δ_{H} 8.41 (1H, s), 7.66 (1H, s), 7.55 (1H, d, J = 1.0), 7.32 (3H, m), 6.28 (1H, d, J = 1.0), 4.99 (1H, s), 4.37 (1H, m), 4.09 (5H, m), 3.61 (5H, m), 2.95 (1H, m), 2.62 (1H, m), 2.54 (1H, dd, J = 19.5, 2.0), 2.12 (1H, m), 1.83 (7H, m), 1.36 (6H, m).

### Example 25: Preparation of Compound 25

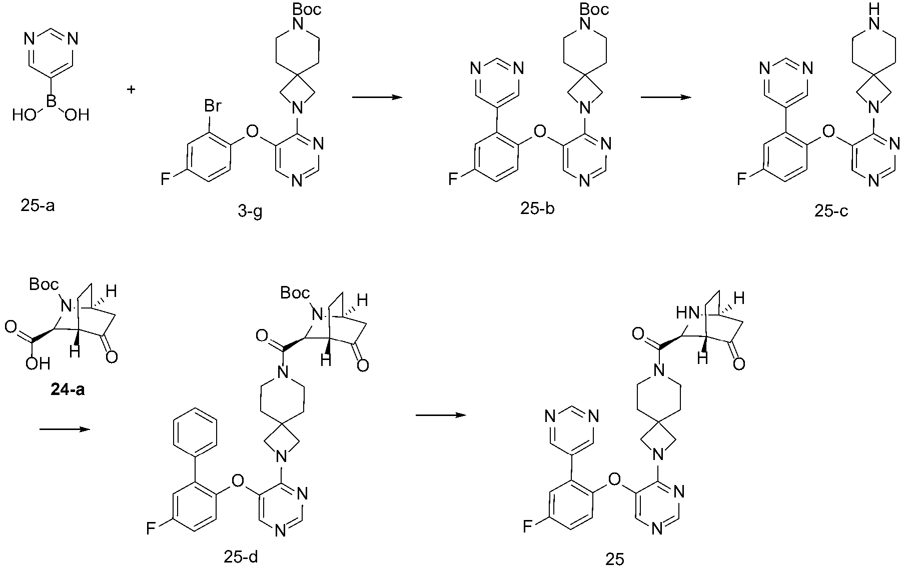

### Step 1: Preparation of compound 25-b

Referring to the preparation method for compound 4-b in Example 4, compound 4-a was replaced by compound 25-a to give compound 25-b.

### Step 2: Preparation of compound 25-c

Referring to the preparation method for compound 4-c in Example 4, compound 25-c was obtained.

### Step 3: Preparation of compound 25-d

Referring to the preparation method for compound 4-d in Example 4, compound 2-b was replaced by compound 24-a to give compound 25-d.

### Step 4: Preparation of compound 25

Referring to the preparation method for compound 4 in Example 4, compound 25 was obtained. ESI-MS: m/z = 544.60 [M+H]⁺.

¹H-NMR (CDCl₃): δ_{H} 1.22-2.05 (8H, m), 2.31 (1H, m), 2.35 (1H, d, J = 1.60 Hz), 2.72 (1H, d, J = 18.81 Hz), 3.28 (2H, m), 3.50 (1H, m), 3.65 (1H, m), 3.88 (4H, m), 4.20 (1H, s), 5.34 (1H, m), 6.93 (1H, dd, J = 4.45, 9.00 Hz), 7.15 (1H, m, J = 3.23 Hz), 7.20 (1H, dd, J = 2.98, 8.28 Hz), 7.73 (1H, s), 8.37 (1H, s), 8.90 (2H, s), 9.23 (1H, s).

### Example 26: Preparation of Compound 26

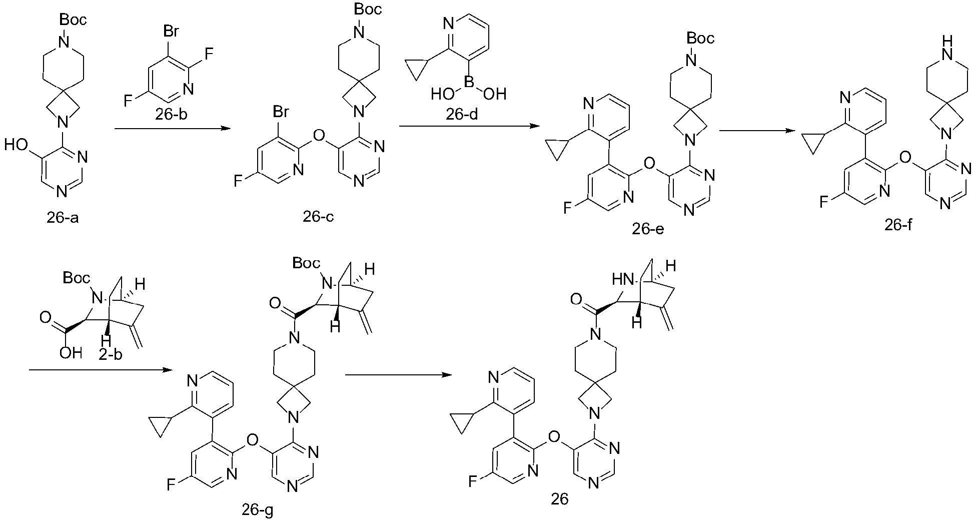

### Step 1: Preparation of compound 26-c

Intermediate 26-a (160 mg), 26-b (100 mg), Cs₂CO₃ (320 mg), and DMF (5 mL) were added to a reaction flask, and the mixture was stirred at 60 °C for 24 h. After the starting materials were consumed completely as detected, water and ethyl acetate were added to the reaction solution, followed by phase separation. The organic phase was concentrated to give intermediate 26-c (180 mg).

### Steps 2 to 5: Preparation of compound 26

Referring to the synthetic method of steps 5 to 8 in Example 3, compound 26 was obtained. ESI-MS: m/z = 582.31 [M+H]⁺.

¹H-NMR (CDCl₃): δ_{H} 0.84-0.89 (2H, m), 1.12 (2H, m), 1.22-1.94 (9H, m), 2.09 (1H, m), 2.51-2.57 (2H, m), 2.97 (1H, m), 3.13-3.22 (2H, m), 3.46 (1H, m), 3.65 (1H, m, J = 9.89 Hz), 3.93-4.08 (4H, m), 4.76 (1H, m), 5.03 (1H, s), 5.12 (1H, s), 7.54 (1H, m), 7.63 (1H, dd, J = 2.90, 7.05 Hz), 7.92 (1H, s), 8.18 (1H, d, J = 2.85 Hz), 8.54 (1H, s), 8.60 (1H, s), 8.70 (1H, s), 10.95 (1H, s).

### Example 27: Preparation of Compound 27

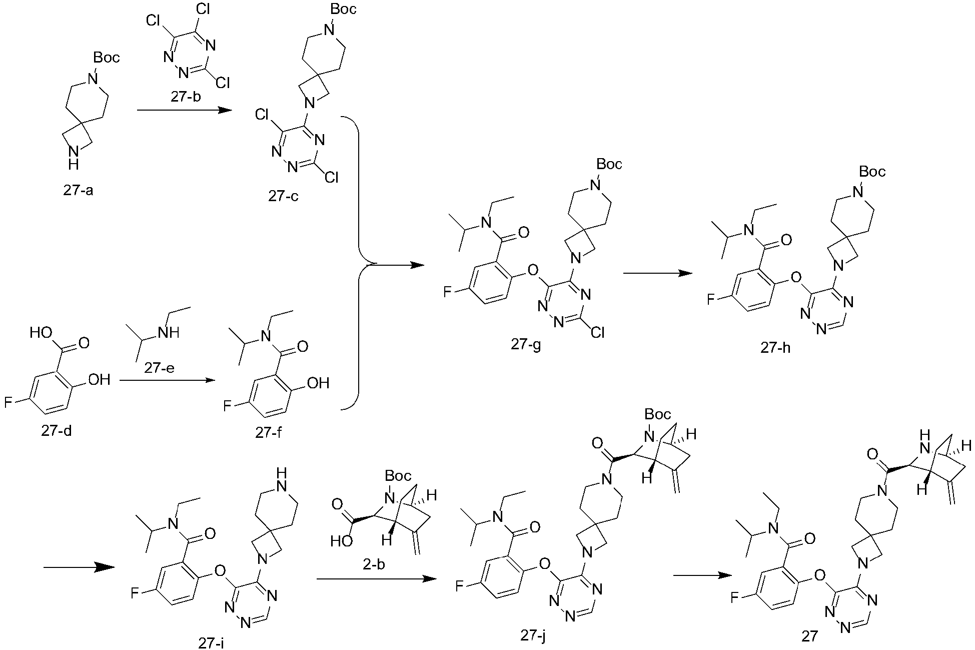

### Step 1: Preparation of compound 27-c

27-a (1.2 g), 27-b (1.0 g), triethylamine (0.8 g), and dichloromethane (25 mL) were added to a reaction flask, and the mixture was stirred at 0 °C for 24 h. After the starting materials were consumed completely as detected, water and dichloromethane were added to the reaction solution, followed by phase separation. The organic phase was washed with water and concentrated to give intermediate 27-c (1.5 g). ESI-MS: m/z = 374.11 [M+H]⁺.

### Step 2: Preparation of compound 27-f

Referring to the preparation method for intermediate 5-c in Example 5, intermediate 5-b was replaced by compound 27-e to give intermediate 27-f.

### Step 3: Preparation of compound 27-g

27-c (200 mg), 27-f(325 mg), DBU (159 mg), and tetrahydrofuran (10 mL) were added to a reaction flask, and the mixture was stirred at room temperature for 72 h. After the starting materials were consumed completely as detected, water and EA were added to the reaction solution, followed by phase separation. The aqueous phase was extracted with EA, and the organic phases were combined and purified by silica gel column chromatography to give compound 27-g (320 mg).

### Step 4: Preparation of compound 27-h

27-g (320 mg), 10% palladium on carbon (100 mg), triethylamine (1 mL), and methanol (10 mL) were added to a reaction flask, and 10% palladium on carbon was then added. The mixture was purged with H₂ and stirred at room temperature for 12 h. After the starting materials were consumed completely as detected, the reaction solution was filtered and concentrated to dryness to give compound 27-h (260 mg).

### Steps 5 to 7: Preparation of compound 27

Referring to the synthetic method of steps 3 to 5 in Example 5, compound 27 was obtained. ESI-MS: m/z = 578.43 [M+H]⁺.

¹H NMR (500 MHz CDCl₃): *δ*_{H} 8.50 (1H, s), 8.30 (1H, s), 7.15 (1H, m), 7.03 (2H, m), 5.06 (1H, m), 4.96 (1H, brs), 4.81 (1H, brs), 4.42 (3H, m), 3.94 (5H, m), 3.34 (6H, m), 2.97 (1H, d, *J* = 18.1), 2.46 (2H, m), 2.05 (1H, m), 1.90 (3H, m), 1.69 (3H, m), 1.14 (6H, m), 0.77 (2H, m).

### Examples 28-30

Referring to the preparation method for compound 27 in Example 27, compound 27-d was replaced by the starting compounds shown in the table below to give target compounds 28, 29, and 30.

| Example | Starting compound | Structures of compounds 28-30 | ESI-MS: m/z [M+H]⁺ | ¹H-NMR |
|---|---|---|---|---|
| 28 | 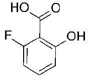 | 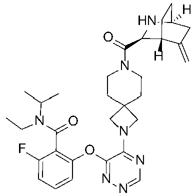 | 578.36 | |
| 29 | 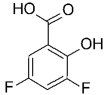 | 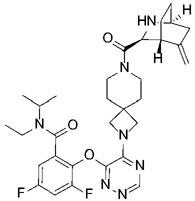 | 596.35 | ¹H-NMR (CDCl₃) :δ_{H} 0.72~1.99 (17H, m), 2.40 (1H, d, J=18.51 Hz), 2.44 (1H, s), 2.91 (1H, d, J=17.11 Hz), 3.15 (1H, m), 3.34 ~3.98 (9H, m), 4.31~4.41 (2H, m), 4.68 (1H, s), 4.91 (1H, s), 5.01 (1H, s), 6.83 (1H, m), 7.00 (1H, m), 8.51 (1H, s). |
| 30 | 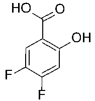 | 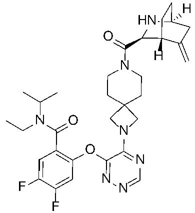 | 596.50 | ¹H NMR (500MHz CDCl₃) δ_{H} 8.51 (1H, m), 7.18 (1H, m), 7.12 (1H, m), 4.97 (1H, brs), 4.87 (1H, s), 4.41 (3H, m), 3.94 (4H, m), 3.53 (5H, m), 3.14 (1H, m), 2.85 (1H, d, J = 17.5), 2.37 (2H, m), 1.96 (6H, m), 1.63 (3H, m), 1.13 (7H, m), 0.80 (2H, m). |

### Example 31: Preparation of Compound 31

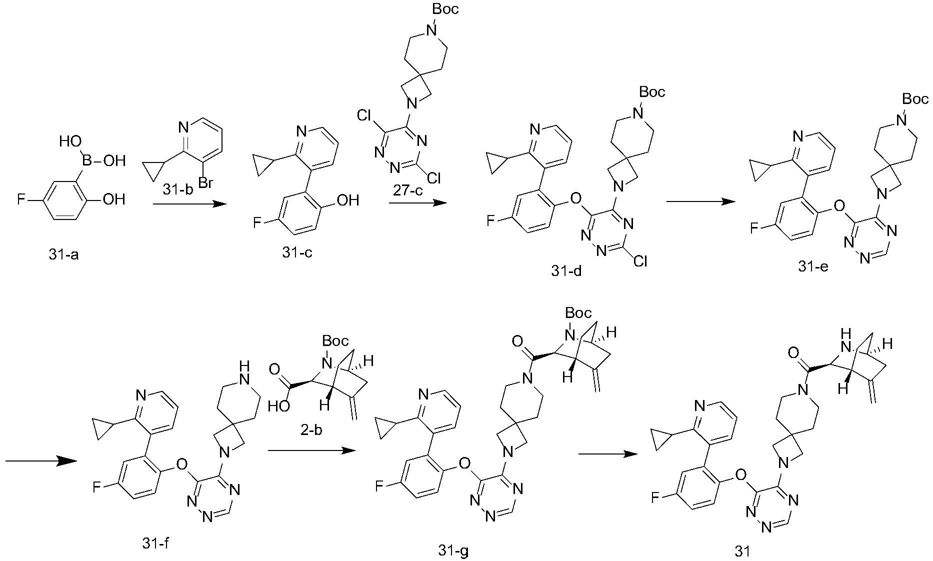

### Step 1: Preparation of compound 31-c

Intermediate 31-a (156 mg), 31-b (198 mg), Pd(dppf)Cl₂ (73 mg), sodium carbonate (212 mg), 1,4-dioxane (5 mL), and H₂O (1 mL) were added to a reaction flask, and the mixture was stirred at 90 °C for 12 h under a nitrogen atmosphere. After the reaction was completed as detected, ethyl acetate and water were added to the reaction solution, and the mixture was stirred and subjected to phase separation. The ethyl acetate phase was purified by silica gel column chromatography and concentrated to give intermediate 31-c (200 mg). ESI-MS: m/z = 230.06 [M+H]⁺.

### Steps 2 to 6: Preparation of compound 31

Referring to the synthetic method of steps 3 to 7 in Example 27, compound 31 was obtained. ESI-MS: m/z = 582.37[M+H]⁺.

¹H NMR (500 MHz CDCl₃): *δ*_{H} 8.46 (2H, m), 8.21 (1H, s), 7.48 (1H, dd, *J* = 8.6, 4.6), 7.39 (1H, d, *J* = 7.2), 7.19 (1H, td, *J =* 8.6, 2.9), 7.09 (2H, m), 5.03 (1H, s), 4.93 (1H, s), 4.70 (1H, s), 3.80 (5H, m), 3.39 (4H, m), 2.92 (1H, d, *J =* 17.6), 2.44 (2H, m), 2.00 (2H, m), 1.70 (7H, m), 0.89 (3H, m), 0.46 (1H, m).

### Examples 32-34

Referring to the preparation method in Example 31, compound 31-b was replaced by the starting compounds shown in the table below to give target compounds 32, 33, and 34.

| Example | Starting compound | Structures of compounds 32-34 | ESI-MS: m/z [M+H]⁺ | ¹H-NMR |
|---|---|---|---|---|
| 32 | 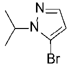 | 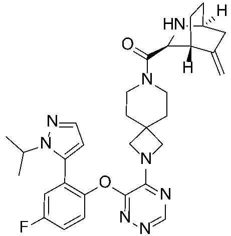 | 573.37 | ¹H-NMR (CDCl₃) :δ_{H} 1.30(6H, m), 1.63~2.06(8H,m), 2.44 (1H, d, J=17.86 Hz), 2.49 (1H, s), 2.95 (1H, d, J=17.81 Hz), 3.26~3.44(3H, m), 3.79~3.90 (6H, m), 4.26 (1H, brs), 4.75 (1H, s), 4.96 (1H, s), 5.06 (1H, s), 6.13 (1H, s), 7.07 (1H, dd, J=2.68, 8.03 Hz), 7.23 (1H, m), 7.42 (1H, m), 7.55 (1H, s), 8.49 (1H, s). |
| 33 | 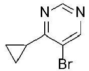 | 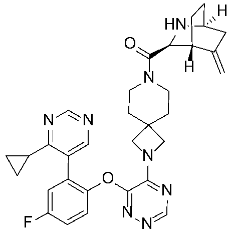 | 583.29 | ¹H NMR (500MHz CDCl₃) :*δ*_{H} 8.96 (1H, s), 8.48 (1H, s), 8.39 (1H, brs), 7.46 (1H, m), 7.23 (1H, m), 7.13 (1H, m), 4.89 (1H, s), 4.81 (1H, s), 4.01 (1H, s), 3.86 (4H, m), 3.57 (1H, m), 3.35 (4H, m), 2.71 (1H, m), 2.36 (1H, m), 2.25 (1H, brs), 1.73 (8H, m), 1.54 (2H, m), 0.99 (3H, m), 0.61 (1H, brs) |
| 34 | 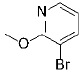 | 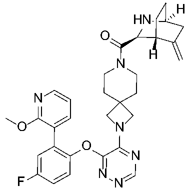 | 572.29 | ¹H NMR (500MHz CDCl₃) *δ*_{H} 8.48 (1H, s), 8.19 (1H, m), 7.49 (1H, dd,*J* = 7.0, 2.0), 7.44 (1H, dd, *J* = 9.0, 5.0), 7.15 (1H, m), 7.06 (1H, m), 6.95 (1H, m), 5.02 (1H, s), 4.93 (1H, s), 4.62 (1H, s), 3.83 (4H, m), 3.63 (5H, m), 3.37 (3H, m), 2.91 (1H, d, *J* = 17.5), 2.41 (2H, m), 1.99 (1H, m), 1.71 (8H, m). |

### Example 35: Preparation of Compound 35

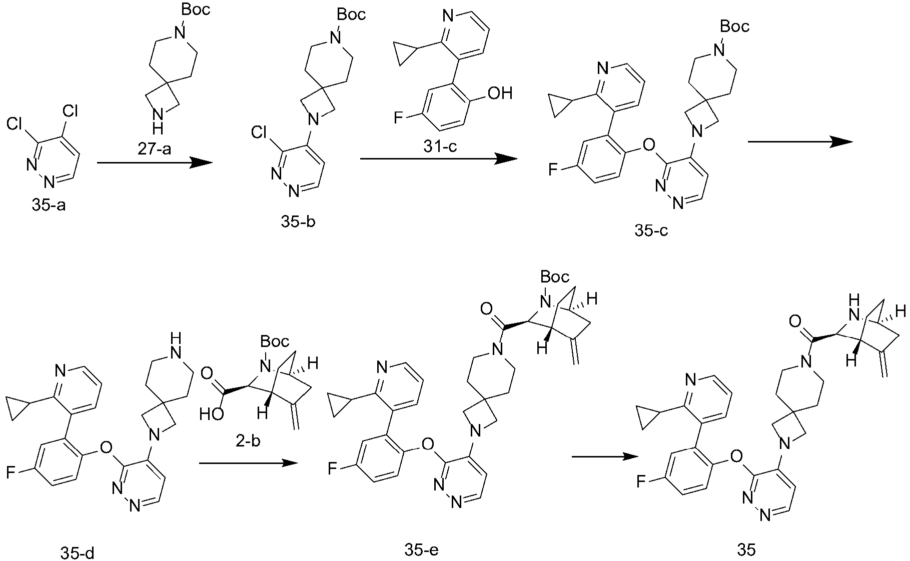

### Step 1: Preparation of compound 35-b

Intermediate 35-a (200 mg), 27-a (304 mg), potassium carbonate (371 mg), and DMF (5 mL) were added to a reaction flask, and the mixture was stirred at 25 °C for 18 h. After the reaction was completed as detected, the reaction solution was filtered, purified by silica gel column chromatography, and concentrated to give intermediate 35-b (300 mg). ESI-MS: m/z = 339.27 [M+H]⁺.

### Step 2: Preparation of compound 35-c

Intermediate 35-b (296 mg), 31-c (200 mg), cesium carbonate (159 mg), and DMF (5 mL) were added to a reaction flask, and the mixture was stirred at 130 °C for 72 h. After the reaction was completed as detected, the reaction solution was filtered, purified by silica gel column chromatography, and concentrated to give intermediate 35-c (200 mg). ESI-MS: m/z = 532.29 [M+H]⁺.

### Steps 3 to 5: Preparation of compound 35

Referring to the synthetic method of steps 3 to 5 in Example 5, compound 35 was obtained. ESI-MS: m/z = 581.31 [M+H]⁺. ¹H-NMR (CDCl₃): δ_{H} 8.43 (1H, d, J = 5.30 Hz), 8.40 (1H, m), 7.43 (1H, dd, J = 4.75, 9.00 Hz), 7.37 (1H, dd, J = 1.65, 7.60 Hz), 7.16 (1H, dt, J = 3.03, 8.43 Hz), 7.08 (1H, dd, J = 3.03, 8.38 Hz), 7.01 (1H, dd, J = 4.88, 7.48 Hz), 6.13 (1H, d, J = 5.45 Hz), 5.07 (1H, s), 4.97 (1H, s), 4.77 (1H, s), 3.83-3.87 (2H, m), 3.44-3.64 (5H, m), 3.22-3.23 (2H, m), 2.97 (1H, d, J = 18.16 Hz), 2.51 (1H, m), 2.45 (1H, m), 2.07 (1H, m), 1.64-1.91 (8H, m), 0.82-0.94 (3H, m), 0.45 (1H, m).

### Example 36

Referring to the preparation method for compound 35 in Example 35, compound 31-c was replaced by the starting compound shown in the table below to give target compound 36.

| Exam ple | Starting compound | Compound structure | ESI-MS: m/z [M+H]⁺ | ¹H-NMR |
|---|---|---|---|---|
| 36 | 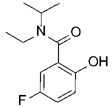 | 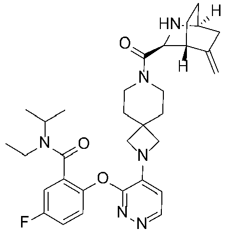 | 577.21 | ¹H NMR (500MHz CDCl₃) *δ*_{H} 8.54 (1H, s), 8.23 (1H, s), 7.14 (3H, m), 6.43 (1H, s), 5.10 (1H, s), 4.97 (1H, s), 4.76 (1H, s), 4.00 (7H, m), 3.18 (6H, m), 2.55 (2H, m), 1.90 (7H, m), 1.13 (10H, m). |

### Examples 37-41

Referring to the preparation method for compound 27 in Example 27, compound 27-e was replaced by the starting compounds shown in the table below to give target compounds 37-41.

| Exampl e | Starting compound | Compound structure | ESI-MS: m/z [M+H]⁺ | ¹H-NMR |
|---|---|---|---|---|
| 37 | 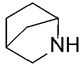 | 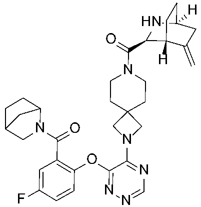 | 588.31 | ¹H NMR (500MHz CDCl₃) *δ*_{H} 8.48 (1H, s), 7.23 (1H, m), 7.16 (1H, m), 7.04 (1H, m), 4.98 (1H, s), 4.88 (1H, s), 4.45 (4H, m), 3.99 (3H, m), 3.54 (2H, m), 3.24 (3H, m), 3.02 (1H, m), 2.86 (1H, d, *J* = 17.5), 2.57 (1H, s), 2.40 (2H, m), 1.89 (5H, m), 1.44 (10H, m) |
| 38 | 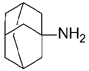 | 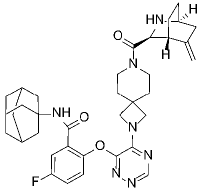 | 642.36 | ¹H-NMR (CDCl3) δ_{H} 8.52 (1H, s),7.21 (1H, d, J=7.40 Hz),7.13~7.15 (2H, m), 4.98 (1H, s), 4.88 (1H, s),4.45~4.55 (3H, m),3.88~4.01 (3H, m),3.34~3.55 (4H, m),2.86 (1H, d, J=17.81 Hz), 2.35~2.39 (2H, m), 1.56~2.04 (23H, m) |
| 39 | 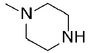 | 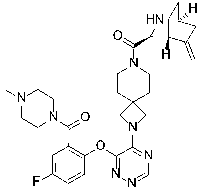 | 591.31 | |
| 40 | 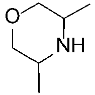 | 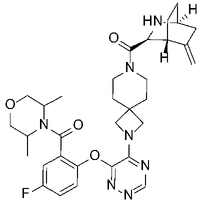 | 606.32 | ¹H-NMR (CDCl₃) *δ*_{H} 8.51 (1H, s) ,7.21 (1H, m), 7.14 (1H, m), 7.10 (1H, dd, J=2.80, 7.75 Hz), 5.00 (1H, d, J=6.50 Hz), 4.90 (1H, s), 4.72 (1H, m), 4.42 (2H, m), 3.69~3.81 (7H, m), 3.54 (1H, m), 3.32 (5H, m), 2.90 (1H, d, J=17.91 Hz), 2.43 (1H, s), 2.38 (1H, d, J=17.96 Hz), 1.97 (2H, m), 1.86 (3H, m), 1.63 (3H, m), 1.25 (6H, m). |
| 41 | 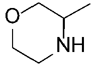 | 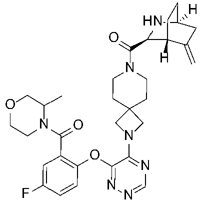 | 592.31 | ¹H-NMR (CDCl₃) *δ*_{H} 8.51 (1H, s), 7.25 (1H, m), 7.17 (1H, m), 7.01 (1H, m), 5.02 (1H, s), 4.92 (1H, s), 4.75 (1H, m), 4.26~4.47 (3H, m), 3.91~4.00 (4H, m), 3.04~3.77 (9H, m), 2.92 (1H, d, J=17.96 Hz), 2.46 (1H, s), 2.40 (1H, m), 1.90 ~1.99 (6H, m), 1.60~1.69 (3H, m), 1.26~1.34 (2H, m). |

### Example 42: Preparation of Compound 42

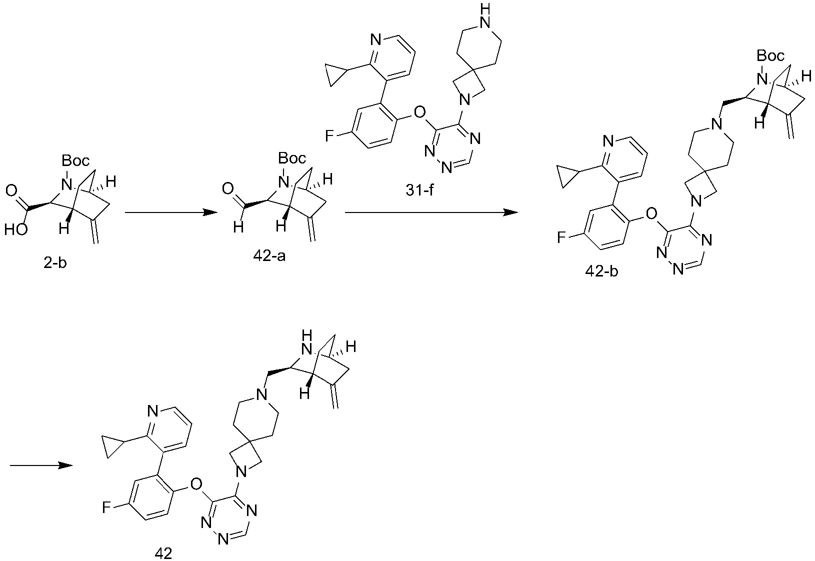

### Step 1: Preparation of compound 42-a

Intermediate 2-b (100 mg), DMAP (304 mg), and dichloromethane (5 mL) were added to a reaction flask, and the mixture was stirred at 25 °C for 10 min. 1-(Trifluoroacetyl)-4-(dimethylamino)pyridinium trifluoroacetate (100 mg), and pinacolborane (53 mg) were then added in sequence, and the resulting mixture was stirred at 25 °C for 10 min. Water (10 mL) was added to the reaction solution to quench the reaction, and the mixture was extracted with dichloromethane. The organic phases were combined, dried, filtered, and concentrated to give intermediate 42-a (30 mg).

### Step 2: Preparation of compound 42-b

Intermediate 31-f (30 mg), intermediate 42-a (22 mg), and methanol (5 mL) were added to a reaction flask, and the mixture was stirred at 25 °C for 10 min. Sodium cyanoborohydride (13 mg) and glacial acetic acid (1 drop) were then added in sequence, and the resulting mixture was stirred at 25 °C for 2 h. Water (10 mL) was added to the reaction solution to quench the reaction, and the mixture was extracted with dichloromethane. The organic phases were combined, dried, filtered, and concentrated to give intermediate 42-b (30 mg).

### Step 3: Preparation of compound 42-b

Intermediate 42-b (30 mg), trifluoroacetic acid (2 mL), and dichloromethane (5 mL) were added to a reaction flask, and the mixture was stirred at 25 °C for 30 min. After the reaction was completed as detected by TLC, the mixture was concentrated, purified, and separated to give compound 42 (10 mg). ESI-MS: m/z = 568.30[M+H]⁺.

¹H-NMR (CDCl₃): *δ*_{H} 8.42-8.43 (2H, m), 7.45 (1H, dd, J = 4.68, 8.88 Hz), 7.37 (1H, d, J = 6.50 Hz), 7.17 (1H, dt, J = 2.95, 8.39 Hz), 7.09 (1H, dd, J = 3.00, 8.35 Hz), 7.02 (1H, dd, J = 4.88, 7.43 Hz), 4.93 (1H, s), 4.82 (1H, s), 3.65-3.72 (3H, m), 3.42-3.47 (3H, m), 2.81 (1H, d, J = 17.56 Hz), 2.66 (1H, t, J = 11.40 Hz), 2.26-2.48 (7H, m), 1.94-2.01 (4H, m), 1.62-1.75 (5H, m), 0.99 (1H, m), 0.93 (1H, m), 0.81 (1H, m), 0.47 (1H, m).

### Example 43: Preparation of Compound 43

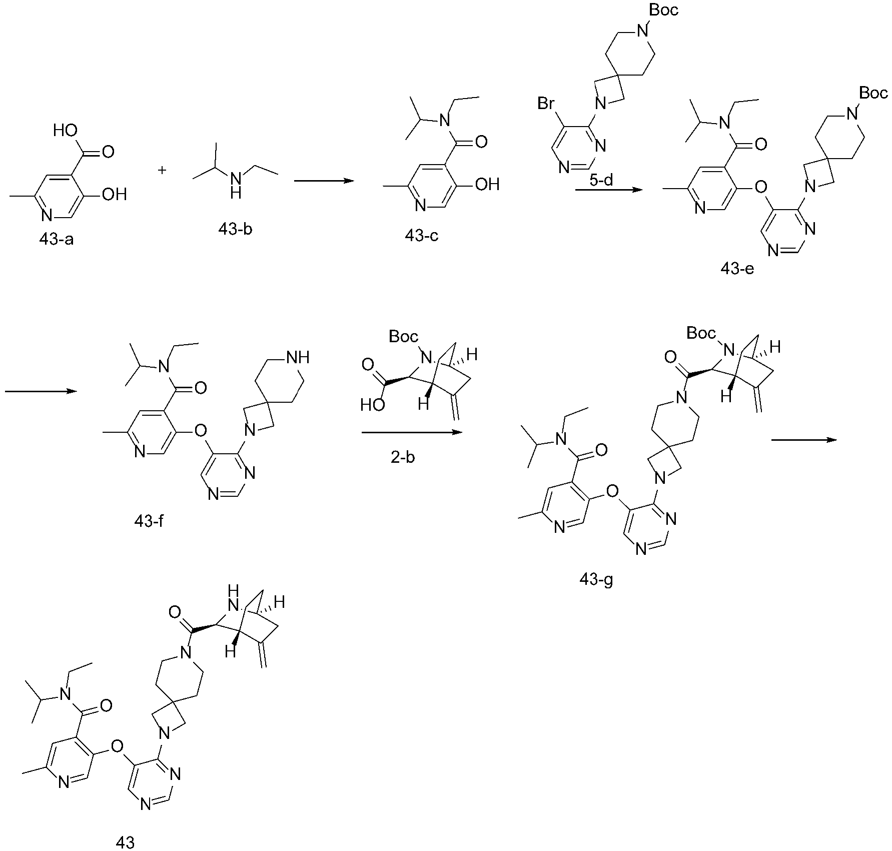

### Step 1: Preparation of compound 43-c

Referring to the preparation method for compound 5 in Example 5, compounds 5-a and 5-b were replaced by compounds 43-a and 43-b to give compound 43-c.

### Steps 2 to 5: Preparation of compound 43

Referring to the preparation method of steps 2 to 5 in Example 5, compound 43 was obtained. ESI-MS: m/z = 574.36 [M+H]⁺.

### Examples 44-46: Preparation of Compounds 44-46

Referring to the preparation method for compound 27 in Example 27, compound 27-e was replaced by the starting compounds shown in the table below to give target compounds 44-46.

| Exam ple | Starting compound | Compound structure | ESI-MS: m/z [M+H] + | ¹H-NMR |
|---|---|---|---|---|
| 44 | 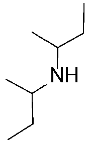 | 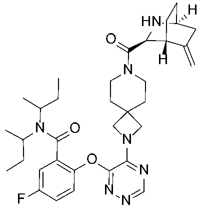 | 620.37 | |
| 45 | 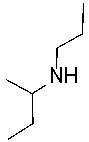 | 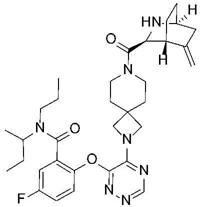 | 606.37 | ¹H-NMR (CDCl₃) *δ*_{H} 8.51 (1H, d, J=3.30 Hz), 7.28 (1H, m), 7.13 (1H, m), 7.01 (1H, m), 4.99 (1H, s), 4.90 (1H, s), 4.37~4.65 (3H, m), 3.94~3.97 (3H, m), 3.35~3.64 (6H, m), 2.88~2.91 (2H, m), 2.37~2.43 (2H, m), 1.86~1.89 (4H, m), 1.46~1.68 (6H, m), 1.12~1.17 (2H, m), 0.53~0.95 (9H, m). |
| 46 | 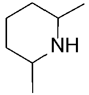 | 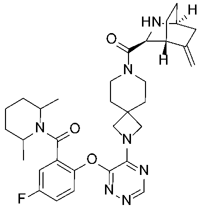 | 604.33 | ¹H-NMR (MeOD-*d*₄) *δ*_{H} 8.45 (1H, s), 7.46 (1H, m), 7.28~7.30 (2H, m), 5.18 (1H, s), 5.01 (1H, s), 4.41~4.45 (3H, m), 4.08 (1H, m), 4.04 (1H, m), 3.51~3.91 (7H, m), 2.80 (1H, d, J=18.21 Hz), 2.69 (1H, m), 2.59 (1H, d, J=17.81 Hz), 1.91~2.06 (9H, m), 1.72 (4H, m), 1.31~1.37 (7H, m). |

### Example 47: Preparation of Compound 47

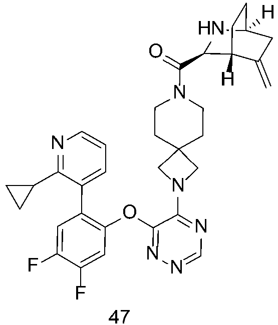

Referring to the preparation method for compound 31 in Example 31, compound 31-a was replaced by 4,5-difluoro-2-hydroxyphenylboronic acid to give compound 47. ESI-MS: m/z = 600.30 [M+H]⁺.

¹H-NMR (CDCl₃): *δ*_{H} 8.49 (1H, s), 8.45 (1H, m), 7.43 (1H, m), 7.36 (1H, d, J = 7.24 Hz), 7.21 (1H, dd, J = 8.75, 9.80 Hz) ,7.06 (1H, m), 5.02 (1H, s), 4.92 (1H, s), 4.58 (1H, m), 3.29-3.88 (9H, m), 2.90 (1H, d, J = 17.96 Hz), 2.39-2.42 (2H, m), 1.64-1.99 (9H, m), 0.47-0.99 (4H, m).

### Examples 48-49

Referring to the preparation method in Example 31, compound 31-b was replaced by the starting compounds shown in the table below to give target compounds 48 and 49.

| Example | Starting compound | Structures of compounds 32-34 | ESI-MS: m/z [M+H]⁺ | ¹H-NMR |
|---|---|---|---|---|
| 48 | 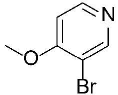 | 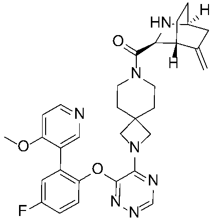 | 572.26 | ¹H-NMR (MeOD) :δ_{H} 1.61 (1H, s)~1.99 (8H, m), 2.49 (1H, d, J=17.96 Hz), 2.61 (1H, s), 2.74 (1H, d, J=17.51 Hz), 3.20~3.45 (4H,m), 3.56~3.62 (4H, m), 3.77~3.87 (4H, m), 4.44 (1H, s), 4.91 (1H, s), 5.09 (1H, s), 7.03 (1H, d, J=5.20 Hz), 7.09 (1H, d, J=7.40 Hz), 7.15 (1H, t, J=6.85 Hz), 7.36 (1H, s), 8.14 (1H, d, J=12.70 Hz), 8.22 (1H, s), 8.35 (1H, d, J=3.25 Hz). |
| 49 | 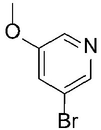 | 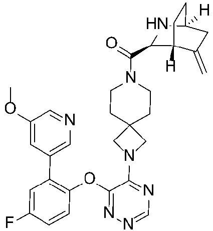 | 572.28 | ¹H-NMR (MeOD-d₄):*δ*_{H} 8.32 (1H, s),8.24 (1H, d, J=2.00 Hz), 3.96 (2H, d, J=22.96 Hz), 8.19 (1H, s),7.41~7.44 (2H, m), 7.28~7.35 (2H, m), 5.20 (1H, s), 5.02 (1H, s), 4.59 (1H, s), 4.31 (2H, d, J=16.71 Hz), 3.87 (3H, s), 3.80 (1H, d, J=12.65 Hz),3.73 (1H, s),3.49~3.60 (3H, m), 2.88 (1H, d, J=18.16 Hz),2.72 (1H, s), 2.59 (1H, dd, J=1.98, 18.18 Hz),2.10 (1H, m),1.94~1.96 (2H, m), 1.88 (2H, t, J=5.48 Hz), 1.73 (3H, m). |

### Examples 50-51

Referring to the preparation method for compound 27 in Example 27, compound 27-e was replaced by the starting compounds shown in the table below to give target compounds 50-51.

| Exam ple | Starting compound | Compound structure | ESI-MS: m/z [M+H] + | ¹H-NMR |
|---|---|---|---|---|
| 50 | 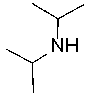 | 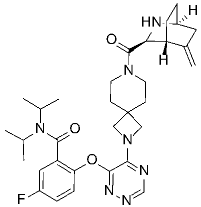 | 592.35 | ¹H-NMR (CDCl₃):*δ*_{H} 8.50 (1H, s),7.10~7.26 (2H, m), 6.96 (1H, dd, J=2.93, 7.78 Hz), 5.03 (1H, d, J=12.00 Hz), 4.93 (1H, s), 4.76 (1H, s),4.61 (1H, m), 4.38 (1H, d, J=9.60 Hz), 3.96~3.98 (3H, m), 3.80 (1H, m), 3.74 (1H, s), 3.36~3.56(4H, m), 2.94 (1H, d, J=18.06 Hz), 2.63 (1H, s),2.47 (1H, d, J=19.61 Hz),2.41 (1H, d, J=18.16 Hz), 1.87~2.02 (4H, m), 1.63~1.70 (3H, m),1.50 (3H, d, J=5.40 Hz), 1.37 (3H, m), 1.10 (3H, d, J=6.10 Hz),0.74 (3H, m). |
| 51 | 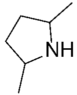 | 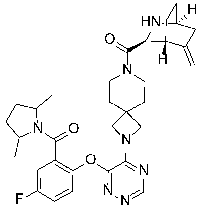 | 590.31 | |

### Example 52: Preparation of Compound 52

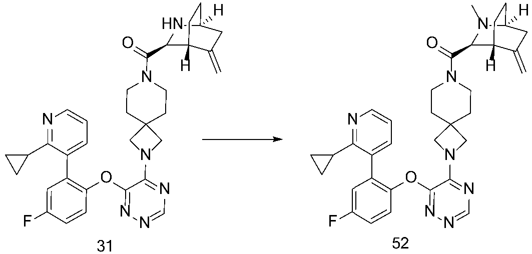

Compound 31 (50 mg), potassium carbonate (30 mg), iodomethane (1 drop), and DMF (5 mL) were added to a reaction flask, and the mixture was stirred at 25 °C for 3 h. After the reaction was completed as detected by TLC, the mixture was purified and separated to give compound 52 (18 mg). ESI-MS: m/z = 596.33 [M+H]⁺.

### Example 53: Preparation of Compound 53

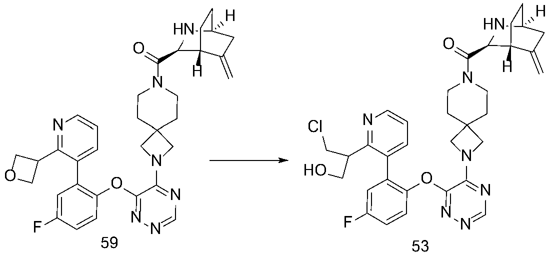

Compound 59 (50 mg), 1 M hydrochloric acid (1 mL), and THF (5 mL) were added to a reaction flask, and the mixture was stirred at 25 °C for 12 h. After the reaction was completed as detected by TLC, the mixture was purified and separated to give compound 53 (5 mg). ESI-MS: m/z = 634.22 [M+H]⁺.

¹H-NMR (CDCl₃): *δ*_{H} 8.57 (1H, s), 8.46 (1H, m), 7.54 (1H, t, J = 8.08 Hz),7.04-7.38 (5H, m), 5.04 (1H, s), 4.94 (1H, s), 4.68 (1H, s), 3.72-3.89 (7H, m), 3.31-3.45 (4H, m), 2.92 (1H, d, J = 17.11 Hz), 2.41-2.47 (2H, m), 1.67-2.00 (11H, s), 1.29(1H, m).

### Example 54

Referring to the preparation method in Example 31, compound 31-b was replaced by the starting compound shown in the table below to give target compound 54.

| Exampl e | Starting compound | Compound structure | ESI-MS: m/z [M+H]⁺ | ¹H-NMR |
|---|---|---|---|---|
| 54 | 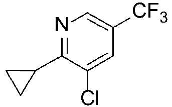 | 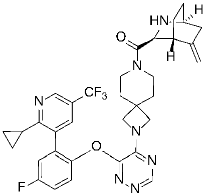 | 650.26 | ¹H-NMR (CDCl₃) *δ*_{H} 8.68 (1H, s), 8.46 (1H, s), 7.64 (1H, s), 7.44 (1H, m), 7.23 (1H, t, J=6.85 Hz), 7.14 (1H, dd, J=2.48, 7.93 Hz), 5.02 (1H, s), 4.92 (1H, s), 4.68 (1H, s), 3.83~3.90 (4H, m), 3.68 (1H, m), 3.50 (2H, m), 3.27 (2H, m), 2.91 (1H, d, J= 17.96 Hz), 2.38~2.42 (2H, m), 1.62~1.99 (9H, m), 0.94~1.06 (3H, m), 0.64 (1H, m). |

### Example 55: Preparation of Compound 55

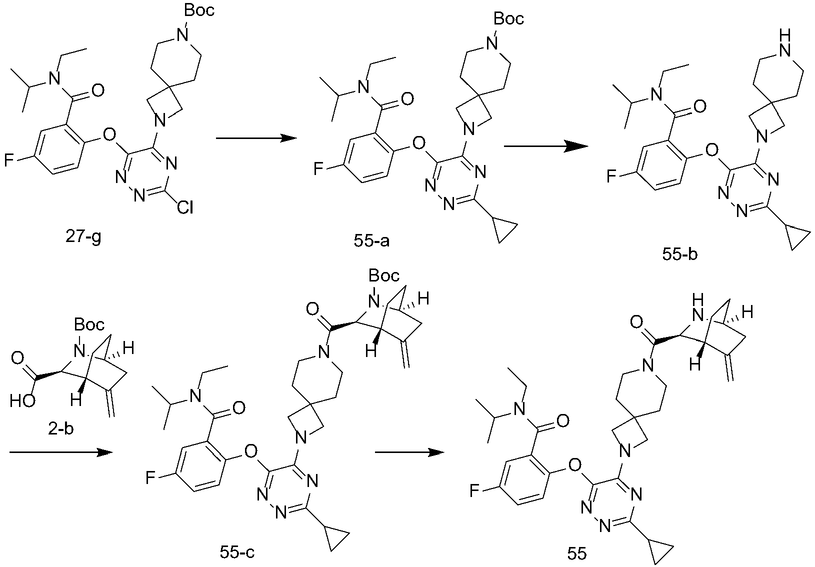

### Step 1: Preparation of compound 55-a

Compound 27-g (560 mg), cyclopropylboronic acid (172 mg), Pd(dppf)Cl₂ (73 mg), K₂CO₃ (280 mg), 1,4-dioxane (10 mL), and H₂O (2 mL) were added to a reaction flask, and the mixture was stirred at 100 °C for 5 h. After the reaction was completed as detected by TLC, the mixture was purified and separated to give compound 55-a (360 mg).

### Steps 2 to 4: Preparation of compound 55

Referring to the synthetic method of steps 3 to 5 in Example 5, compound 55 was obtained. ESI-MS: m/z = 618.32 [M+H]⁺.

¹H-NMR (CDCl₃): *δ*_{H} 7.05-7.29 (3H, m), 5.02 (1H, s), 4.92 (1H, s), 4.69 (1H, s), 3.92-4.49 (6H, m), 3.70 (1H, m), 3.13-3.52 (5H, m), 2.91 (1H, d, J = 17.96 Hz), 2.39-2.46 (2H, m), 1.99 (3H, m), 1.65-1.87 (6H, m), 0.93-1.17 (13H, m).

### Example 56: Preparation of Compound 56

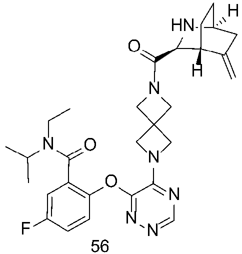

Referring to the preparation method for compound 27 in Example 27, compound 27-a was replaced by tert-butyl 2,6-diazaspiro[3.3]heptane-2-carboxylate to give compound 56. ESI-MS: m/z = 550.21 [M+H]⁺.

¹H-NMR (CDCl₃): *δ*_{H} 8.51 (1H, s), 7.24 (1H, m), 7.14 (1H, m), 7.00 (1H, m), 4.85-4.96 (4H, m), 3.86-4.54 (8H, m), 3.44 (2H, m), 3.18 (1H, m), 2.79 (1H, d, J = 17.01 Hz), 2.35-2.38 (2H, m), 1.60-1.93 (2H, m), 1.05-1.26 (9H, m), 0.79-0.88 (2H, m).

### Example 57: Preparation of Compound 57

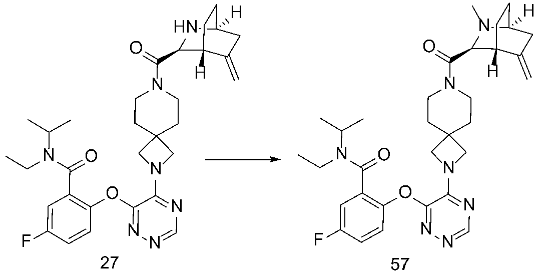

Compound 27 (50 mg), potassium carbonate (30 mg), iodomethane (1 drop), and DMF (5 mL) were added to a reaction flask, and the mixture was stirred at 25 °C for 3 h. After the reaction was completed as detected by TLC, the mixture was purified and separated to give compound 57 (12 mg). ESI-MS: m/z = 592.34 [M+H]⁺.

### Example 58: Preparation of Compound 58

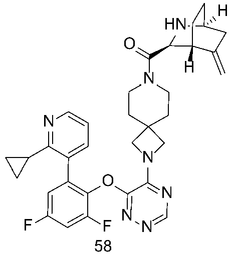

Referring to the preparation method for compound 31 in Example 31, compound 31-a was replaced by 3,5-difluoro-2-hydroxyphenylboronic acid to give compound 58. ESI-MS: m/z = 600.26 [M+H]⁺.

¹H-NMR (CDCl₃): *δ*_{H} 8.46-8.48 (2H, m), 7.37 (1H, m), 7.02-7.08 (2H, m), 6.94 (1H, d, J = 7.20 Hz), 5.04 (1H, s), 4.95 (1H, s), 4.74 (1H, s), 3.76-3.87 (4H, m), 3.32-3.49 (5H, m), 2.94 (1H, d, J = 17.71 Hz), 2.47 (1H, s), 2.43 (1H, d, J = 18.06 Hz), 1.63-2.01 (9H, m), 1.27 (1H, m), 0.88-0.99 (3H, m).

### Example 59

Referring to the preparation method in Example 31, compound 31-b was replaced by the starting compound shown in the table below to give target compound 59.

| Example | Starting compound | Compound structure | ESI-MS: m/z [M+H]⁺ | ¹H-NMR |
|---|---|---|---|---|
| 59 | 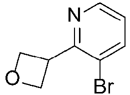 | 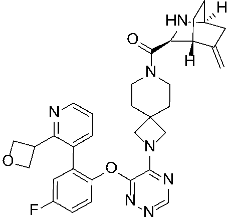 | 598.32 | ¹H-NMR (CDCl₃) *δ*_{H} 8.72 (1H, s), 8.45 (1H, s), 7.47 (1H, d, J=6.80 Hz), 7.36 (1H, m), 7.18 ~7.22 (2H, m), 6.90 (1H, dd, J=2.73, 8.03 Hz), 5.02 (1H, d, J=10.15 Hz), 4.92 (1H, s), 4.77~4.87 (3H, m), 4.67 (1H, m), 4.58 (1H, m), 4.32 (1H, m), 3.80~3.88 (4H, m), 3.67 (2H, m), 3.28 (2H, m), 2.91 (1H, d, J=17.81 Hz), 2.39~2.43 (2H, m), 1.62~1.89 (7H, m), 1.25~1.31 (2H, m). |

### Example 60: Preparation of Compound 60

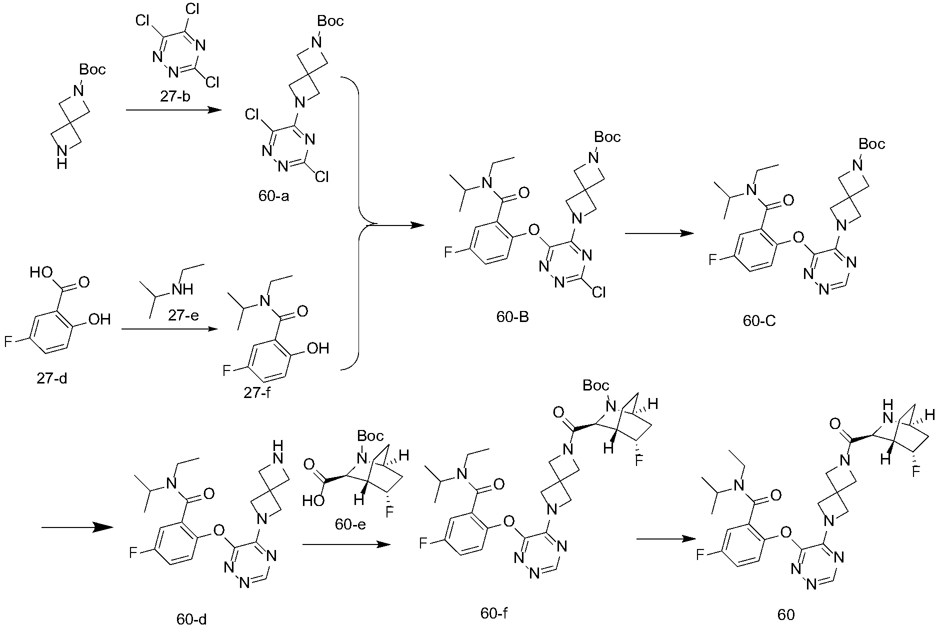

Referring to the preparation method for compound 27 in Example 27, compound 27-a was replaced by tert-butyl 2,6-diazaspiro[3.3]heptane-2-carboxylate, and compound 2-b was replaced by compound 60-e, thus giving compound 60. ESI-MS: m/z = 556.30 [M+H]⁺.

¹H-NMR (CDCl₃): *δ*_{H} 8.51 (1H, s), 7.26 (1H, dd, J = 4.03, 8.63 Hz), 7.15 (1H, m), 7.02 (1H, m), 4.68-4.99 (4H, m), 4.13-4.55 (6H, m), 3.86 (1H, m), 3.73 (1H, m), 3.46 (1H, m), 3.20 (1H, m), 2.16-2.33 (3H, m), 1.97 (1H, m), 1.78 **(1H, m),** 1.51 **(2H, m),** 1.05-1.12 **(7H, m),** 0.78 **(2H, m).**

### Example 61: Preparation of Compound 61

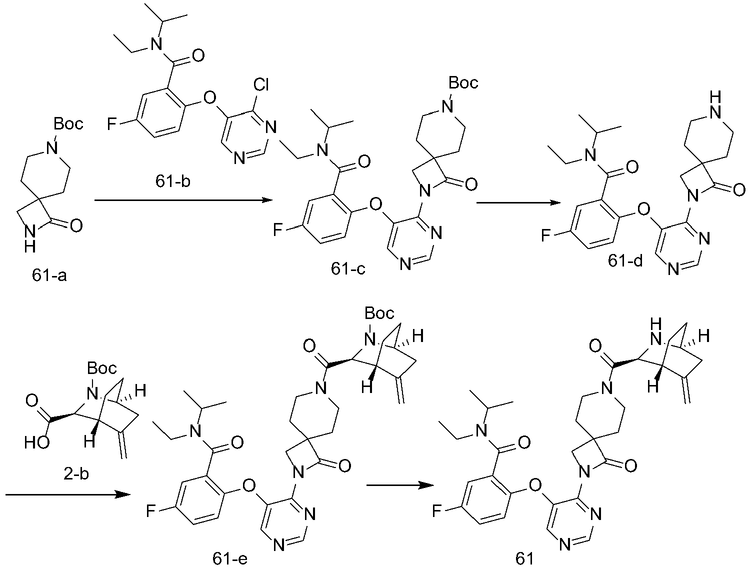

### Step 1: Preparation of compound 61-c

Referring to the preparation method for compound 27-c in Example 27, compound 27-a was replaced by compound 61-a, and compound 27-b was replaced by compound 61-b, thus giving compound 61-c.

### Steps 2 to 4: Preparation of compound 61

Referring to the synthetic method of steps 3 to 5 in Example 5, compound 5-e was replaced by compound 61-c to give compound 61. ESI-MS: m/z = 591.33 [M+H]⁺. ¹H-NMR (CDCl₃): *δ*_{H} 8.74 (1H, m), 8.24 (1H, m), 6.96-7.12 (3H, m), 5.06 (1H, s), 4.96 (1H, s), 4.83 (1H, s), 3.19 (1H, m), 3.60-3.88 (7H, m), 3.40 (2H, m), 2.97 (1H, d, J = 17.86 Hz), 2.54 (1H, s), 2.45 (1H, d, J = 18.21 Hz), 2.03 (4H, m), 1.70 (3H, m), 1.08-1.22 (10H, m).

### Examples 62-63

Referring to the preparation method for compound 61 in Example 61, compound 61-a was replaced by the starting compounds shown in the table below to give target compounds 62 and 63.

| Exam ple | Starting compound | Compound structure | ESI-MS: m/z [M+H]⁺ | ¹H-NMR |
|---|---|---|---|---|
| 62 | 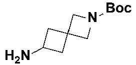 | 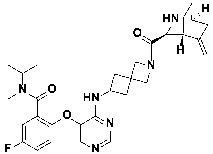 | 563.30 | ¹H-NMR (CDCl₃) *δ*_{H} 8.41 (1H, m) ,8.09 (1H, m), 7.55 (1H, m), 6.89~7.02 (2H, m), 4.96 (1H, d, J=7.40 Hz), 4.30~4.43 (2H, m), 3.91~4.26 (6H, m), 3.56 (1H, m), 3.52 (1H, m), 3.41 (1H, m), 2.85 (1H, d, J=17.76 Hz), 2.65 (2H, m), 2.34~2.40 (3H, m), 2.22 (1H, m), 1.93~2.00 (1H, m), 1.76 (1H, m), 1.59 (2H, m), 1.12~1.34 (9H, m). |
| 63 | 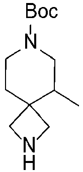 | 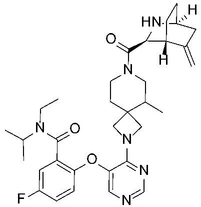 | 591.35 | ¹H-NMR (CDCl₃) *δ*_{H} 8.48 (1H, m), 7.95 (1H, m), 6.97 ~7.02 (2H, m), 6.57 (1H, m), 5.00 (1H, s), 4.89 (1H, s), 4.27 (1H, d, J=7.85 Hz), 3.24~4.11 (12H, m), 2.88 (1H, d, J=17.91 Hz), 2.37~2.43 (2H, m), 1.67 ~2.02 (6H, m), 1.11~1.28 (10H, m), 0.83~0.93 (3H, m). |

### Example 64: Preparation of Compound 64

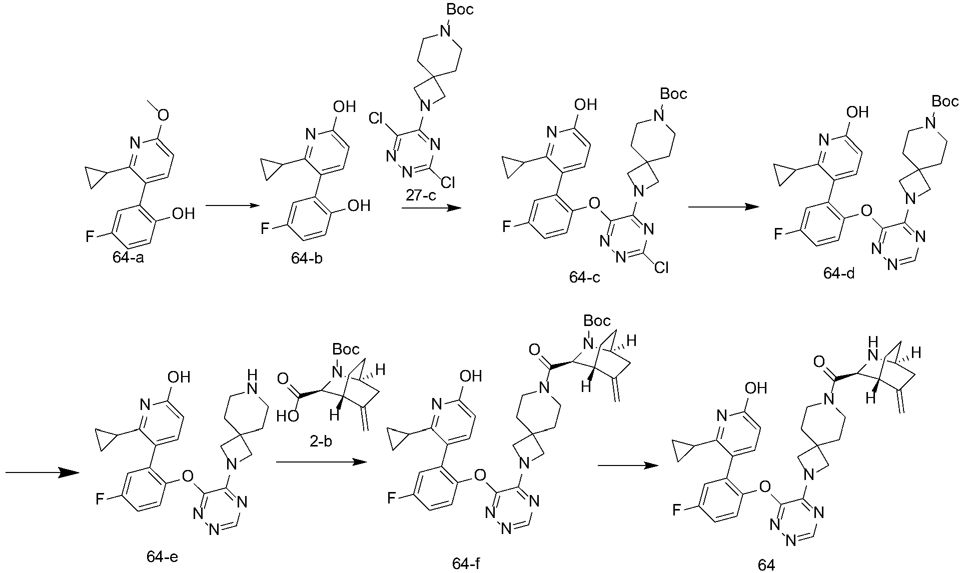

### Step 1: Preparation of compound 64-b

Compound 64-a (1 g), trimethyliodosilane (1 mL), and acetonitrile (10 mL) were added to a reaction flask, and the mixture was stirred at 80 °C for 10 h. After the reaction was completed as detected by TLC, the mixture was purified and separated to give compound 64-b (670 mg).

### Steps 2 to 6: Preparation of compound 64

Referring to the synthetic method of steps 3 to 7 in Example 27, compound 27-f was replaced by compound 64-b to give compound 64. ESI-MS: m/z = 598.28 [M+H]⁺.

¹H-NMR (CDCl₃): *δ*_{H} 8.47 (1H, s),7.36 (1H, m), 7.23 (1H, m), 7.15 (1H, dt, J = 2.77, 8.33 Hz), 7.10 (1H, dd, J = 2.90, 8.35 Hz), 6.35 (1H, d, J = 9.10 Hz), 4.98 (1H, s), 4.87 (1H, s), 4.50 (1H, m), 3.79-4.00 (5H, s), 3.32-3.41 (4H, m), 2.82 (1H, d, J = 17.36 Hz), 2.38-2.40 (2H, m), 1.61-2.01 (9H, m), 0.95 (4H, m).

### Examples 65-66

Referring to the preparation method in Example 31, compound 31-b was replaced by the starting compounds shown in the table below to give target compounds 65 and 66.

| Example | Starting compound | Compound structure | ESI-MS: m/z [M+H]⁺ | ¹H-NMR |
|---|---|---|---|---|
| 65 | 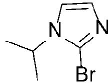 | 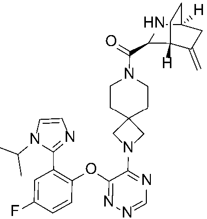 | 573.30 | ¹H-NMR (CDCl₃) *δ*_{H} 8.39 (1H, s), 7.39 (1H, m), 7.23 (1H, m), 7.08~7.11 (2H, m), 7.01 (1H, s), 4.97 (1H, s), 4.87 (1H, s), 4.42~4.55 (1H, m), 4.17~4.29 (4H, m), 3.86 (1H, m), 3.26~3.50 (5H, m), 2.84 (1H, d, J=17.76 Hz), 2.35~2.38 (2H, m), 1.56~1.97 (8H, m), 1.23~1.38 (6H, m). |
| 66 | 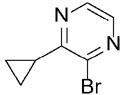 | 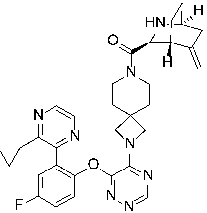 | 583.33 | ¹H-NMR (CDCl₃) *δ*_{H} 8.48(1H, s), 8.41(1H, s), 8.37(1H, s), 7.52(1H, s), 7.25(3H, m),4.97(1H, s), 4.88(1H, s), 4.43(1H, s), 3.82(4H, m), 3.32(4H, m), 2.84 (1H, d, J=17.5), 2.62(1H, s), 2.40(2H, m), 1.95(1H, m),1.59~1.84(8H, m), 0.92(2H, s), 0.65(2H, m). |

### Example 67: Preparation of Compound 67

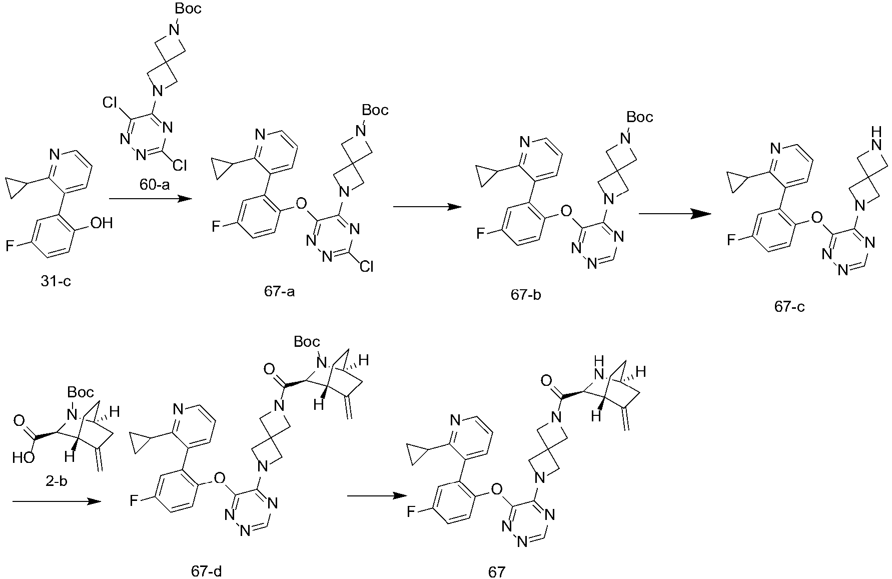

### Step 1: Preparation of compound 67-a

Referring to the preparation method for compound 27-c in Example 27, compound 27-f was replaced by compound 31-c, and compound 27-c was replaced by compound 60-a, thus giving compound 67-a.

### Steps 2 to 5: Preparation of compound 67

Referring to the synthetic method of steps 4 to 7 in Example 27, compound 67 was obtained. ESI-MS: m/z = 554.30 [M+H]⁺.

¹H-NMR (CDCl₃): *δ*_{H} 8.47 (2H, m), 7.42 (2H, m), 7.18 (1H, m), 7.10 (2H, m), 5.10 (1H, m), 4.91 (1H, s), 4.69-3.86 (8H, m), 3.57 (1H, s), 2.85 (1H, d, *J* = 17.0), 2.40 (2H, m), 1.77 (2H, m), 1.64 (2H, m), 1.27 (2H, m), 0.88 (3H, m), 0.50 (1H, brs).

### Example 68

Referring to the preparation method in Example 31, compound 31-b was replaced by the starting compound shown in the table below to give target compound 68.

| Example | Starting compound | Compound structure | ESI-MS: m/z [M+H]⁺ | ¹H-NMR |
|---|---|---|---|---|
| 68 | 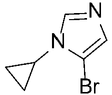 | 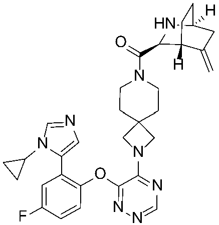 | 571.28 | ¹H-NMR (CDCl₃) *δ*_{H} 8.51(1H, s), 8.23(1H, s), 7.61(1H, dd, J=3.0,9.5), 7.52(1H, s), 7.09 (2H, m), 6.98 (1H, m), 5.07(1H, s),4.98(1H, s), 4.88(1H, s), 4.44(2H, brs), 4.03(3H, m), 3.83(2H, m), 3.54(1H, m), 3.33(3H, m), 3.00(1H, d, J=18.5),2.48(2H, m), 1.92~2.06(4H, m), 1.71(4H, brs), 0.97(2H, m). |

### Example 69: Preparation of Compound 69

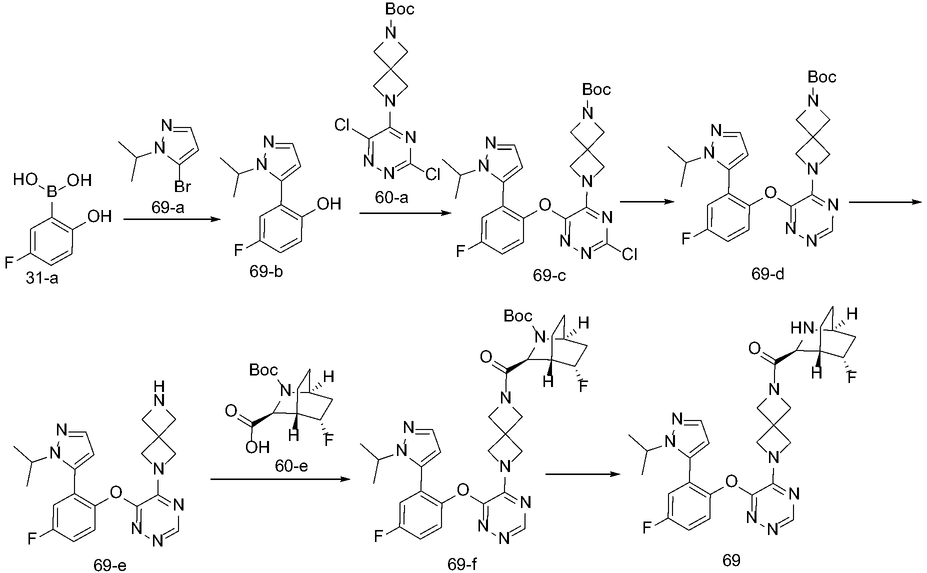

### Step 1: Preparation of compound 69-b

Referring to the preparation method for compound 31-c in Example 31, compound 31-b was replaced by 69-a to give compound 69-b.

### Steps 2 to 6: Preparation of compound 69

Referring to the synthetic method of steps 3 to 7 in Example 27, compound 27-c was replaced by compound 60-a, compound 27-f was replaced by compound 69-b, and compound 2-b was replaced by compound 60-e, thus giving compound 69. ESI-MS: m/z = 551.27 [M+H]⁺.

¹H-NMR (CDCl₃): δH 8.45 (1H, s), 7.53 (1H, d, J = 2.0), 7.33 (1H, dd, J = 5.0, 9.0), 7.21 (1H, m), 7.06 (1H, dd, J = 3.0, 8.5), 6.18 (1H, d, J = 2.0), 4.51 (4H, s), 3.30 (2H, t, J = 5.5), 3.13 (2H, q, J = 7.5), 2.62 (1H, s), 2.42 (2H, t, J = 6.0), 2.24 (6H, s), 1.79 (2H, m), 1.29, 1.31 (6H, m).

### Example 70

Referring to the preparation method in Example 31, compound 31-b was replaced by the starting compound shown in the table below to give target compound 70.

| Exampl e | Starting compound | Compound structure | ESI-MS: m/z [M+H]⁺ | ¹H-NMR |
|---|---|---|---|---|
| 70 | 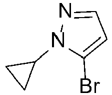 | 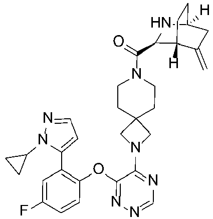 | 571.29 | ¹H-NMR (CDCl₃) δ_{H} 8.48(1H, s),8.44(1H, s), 7.48(1H, brs), 7.43(1H, d, J=2.0), 7.21(1H, m), 7.17(1H, dd, J=3.0,8.5), 6.17(1H, s), 5.02(1H, s), 4.92(1H, s), 4.56(1H, s), 3.27~ 3.90(7H, m), 2.91(1H, d, J= 18.0), 2.40(2H, m), 1.63~2.02(9H, m), 1.25(1H, m), 1.06(2H, m), 0.72(2H, m). |

### Example 71: Preparation of Compound 71

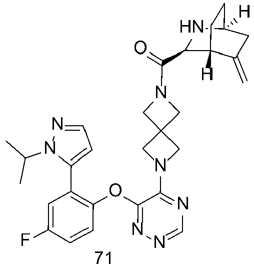

Referring to the preparation method for compound 69 in Example 69, compound 60-e was replaced by 2-b to give compound 71. ESI-MS: m/z = 545.21 [M+H]⁺.

¹H-NMR (CDCl₃): *δ*_{H} 8.49 (1H, s), 8.37 (1H, brs), 7.55 (1H, s), 7.34 (1H, brs), 7.21 (1H, m), 7.07 (1H, dd, *J* = 3.0, 8.0), 6.16 (1H, s), 5.02 (1H, s), 4.91 (1H, s), 4.52 (1H, d, *J=* 9.0), 4.29 (9H, m), 3.58 (1H, s), 2.87 (1H, d, *J=* 18.0), 2.39-2.45 (2H, m), 1.98 (1H, m), 1.78 (1H, m), 1.66 (2H, m), 1.28 (6H, m).

### Example 72: Preparation of Compound 72

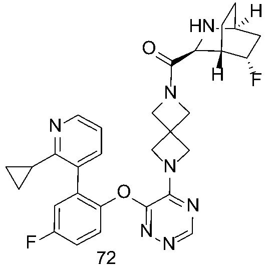

Referring to the preparation method for compound 67 in Example 67, compound 2-b was replaced by 60-e to give compound 72. ESI-MS: m/z = 560.28 [M+H]⁺.

¹H-NMR (CDCl₃): *δ*_{H} 8.47 (2H, s), 7.42 (2H, m), 7.18 (1H, m), 7.07-7.12 (2H, m), 4.84-4.95 (1H, m), 4.26 (10H, m), 3.27 (1H, s), 2.03-2.14 (1H, m), 1.74 (3H, m), 1.39 (3H, m), 0.88 (3H, m), 0.50 (1H, brs).

### Example 73: Preparation of Compound 73

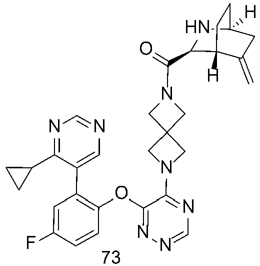

Referring to the preparation method for compound 69 in Example 69, compound 69-a was replaced by 5-bromo-4-cyclopropylpyrimidine, and compound 60-e was replaced by 2-b, thus giving compound 73. ESI-MS: m/z = 555.26 [M+H]⁺.

¹H-NMR (CDCl₃): *δ*_{H} 8.97 (1H, s), 8.49 (1H, s), 8.40 (1H, s), 7.36 (1H, m), 7.23 (1H, m), 7.47 (1H, dd, *J =* 3.0, 8.5), 4.94 (1H, s), 4.84 (1H, s), 4.29 (4H, m), 3.97 (1H, s), 3.33 (1H, s), 2.74 (1H, d, *J =* 18.0), 2.34-2.38 (2H, m), 1.73-1.90 (3H, m), 1.59 (4H, m), 1.26 (2H, m), 0.99-1.12 (3H, m), 0.71 (1H, m).

### Example 74

Referring to the preparation method in Example 31, compound 31-b was replaced by the starting compound shown in the table below to give target compound 74.

| Example | Starting compound | Compound structure | ESI-MS: m/z [M+H]⁺ | ¹H-NMR |
|---|---|---|---|---|
| 74 | 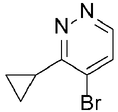 | 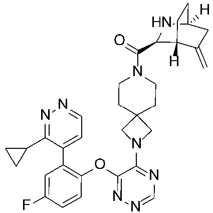 | 583.30 | ¹H-NMR (CDCl₃) *δ*_{H} 8.97(1H, d, *J*=5.0), 8.48,8.46 (1H, s), 7.47(1H, dd, J=4.5,8.5), 7.24(1H, dd, *J*=2.0,8.0),7.21(1H, d, *J=*5.0), 7.11(1H, dd, J=3.0,8.5), 5.03 (1H, s), 4.93(1H, s), 4.54 (1H, s), 3.85 (4H, s), 3.61 (2H, s), 3.29 (4H, m), 2.91(1H, d, *J =* 18.0), 2.41,2.44 (2H, s), 2.01 (1H, m), 1.61~1.78 (8H, m), 1.26 (2H, m), 0.96 (1H, brs), 0.64 (1H, brs). |

### Example 75: Preparation of Compound 75

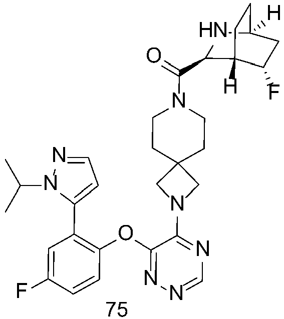

Referring to the preparation method for compound 31 in Example 31, compound 31-b was replaced by 5-bromo-1-isopropyl-1H-pyrazole, and compound 2-b was replaced by 60-e, thus giving compound 75. ESI-MS: m/z = 579.30 [M+H]⁺.

¹H-NMR (CDCl₃): *δ*_{H} 8.48 (1H, s), 8.36 (1H, s), 7.54 (1H, s), 7.47 (1H, s), 7.23 (1H, m), 7.07 (1H, dd, *J =* 3.0, 8.0), 6.13 (1H, d, *J* = 1.5), 4.87-4.99 (1H, m), 4.63 (1H, s), 4.26 (1H, m), 3.84 (6H, brs), 3.47 (3H, brs), 2.18 (4H, m), 1.76-1.88 (7H, m), 1.30-1.51 (6H, m).

### Example 76: Preparation of Compound 76

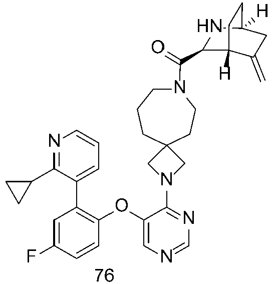

Referring to the preparation method for compound 3 in Example 3, compound 3-f was replaced by 7-Boc-2,7-diazaspiro[3.6]decane to give compound 76. ESI-MS: m/z = 595.34 [M+H]⁺.

¹H-NMR (CDCl₃): *δ*_{H} 8.45 (1H, dd, *J* = 1.0, 4.5), 8.27 (1H, s), 7.66 (1H, s), 7.38 (1H, dd, *J =* 1.5, 7.5), 7.11 (2H, m), 7.06 (1H, dd, *J=* 4.5, 7.5), 6.99 (1H, m), 4.95 (1H, s), 4.86 (1H, s), 3.70, 3.71 (1H, s), 3.22-3.88 (13H, m), 2.83-2.87 (1H, m), 2.35-2.42 (2H, m), 1.58-2.02 (8H,m), 1.08 (2H, brs), 0.85 (2H, brs).

### Example 77: Preparation of Compound 77

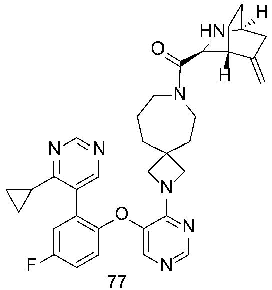

Referring to the preparation method for compound 3 in Example 3, compound 3-h was replaced by (4-cyclopropylpyrimidin-5-yl)boronic acid, and compound 3-f was replaced by 7-Boc-2,7-diazaspiro[3.6]decane, thus giving compound 77. ESI-MS: m/z = 596.31 [M+H]⁺.

¹H-NMR (CDCl₃): *δ*_{H} 8.98 (1H, s), 8.40 (1H, s), 8.31 (1H, s), 7.69 (1H, s), 7.15 (2H, m), 6.97 (1H, m), 6.42 (4H, brs), 4.99 (1H, s), 4.90 (1H, s), 4.56 (1H, m), 3.71-3.90 (5H, m), 3.50-3.63 (2H, m), 3.24-3.38 (2H, m), 2.90 (1H, m), 2.47 (1H, s), 2.40 (1H, d, *J* = 18.0), 1.62-2.02 (8H, m), 1.22 (2H, t, *J =* 4.0), 0.98 (2H, brs) .

### Test Example 1: In Vitro Cell Activity Assay

MOLM-13 cells or MV-4-11 cells (source: Nanjing Cobioer Biosciences Co., Ltd.) in a good growth state were taken and seeded into a 96-well plate (100 µL/well) at 2000-10000 cells/well. After the cells were cultured overnight in a cell incubator at 37 °C, the compounds were subjected to gradient dilution and added using a pipettor, with 2 parallel replicate wells in each group. Meanwhile, a control group was set. After the cells were cultured in a cell incubator at 37 °C for another 72-120 h, the detection reagent CCK-8 (manufacturer: Dojindo Laboratories, Japan; 10 µL/well) was added. After the cells were cultured at 37 °C for another 3-5 h, absorbance values were measured at 450 nM using a PerkinElmer Envision multi-functional microplate reader. Four-parameter analysis was performed in GraphPad Prism software to fit dose-response curves and calculate IC₅₀ values. The specific results are shown in Table 1. A represents that IC50 < 50 nM.

**Table 1. Inhibitory activity against cell proliferation**

| Compound No. | IC50 (nM) for inhibitory activity against proliferation of MOLM-13 cells | IC50 (nM) for inhibitory activity against proliferation of MV-4-11 cells |
|---|---|---|
| 2 | A | A |
| 3 | A | A |
| 4 | A | A |
| 6 | | A |
| 27 | A | A |
| 31 | A | A |
| 32 | | A |
| 33 | | A |
| 34 | | A |
| 35 | A | A |
| 36 | A | A |
| 46 | | A |
| 50 | A | A |
| 54 | A | A |
| 56 | A | A |
| 58 | | A |
| 60 | A | A |
| 64 | | A |
| 65 | | A |
| 66 | | A |
| 67 | A | A |
| 69 | | A |
| 70 | | A |
| 71 | A | A |
| 72 | A | A |
| 73 | A | A |
| 74 | A | A |
| 75 | A | A |
| 76 | A | A |
| 77 | A | A |

The results show that the compounds of the present disclosure exhibited relatively good inhibitory activity against the proliferation of MV-4-11 cells and/or MOLM-13 cells.

### Test Example 2: Detection of Ability of Compounds to Inhibit Menin-MLL Interaction by Fluorescence Polarization Assay

The binding activity of the compounds for the menin protein was determined by a fluorescence polarization assay. GST-MENIN (1-615 aa) and FITC-MLL1₅₋₄₃ were diluted with an FP buffer (50 mM Tris-HCl pH 7.5, 50 mM NaCl, 1 mM DTT, 0.01% BSA), and the mixed solution of GST-MENIN (1-615 aa) at a final concentration of 8 nM and FITC-MLL1₅₋₄₃ at a final concentration of 8 nM was added to a black 384-well microtiter plate at 10 µL/well. The plate was centrifuged at 2500 rpm for 1 min and incubated at room temperature in the dark for 1 h. 5 µL of diluted compound (7 concentration points, 4-fold serial dilution, with blank control and negative control wells set) was added, and the plate was centrifuged at 2500 rpm for 1 min and incubated at room temperature in the dark for 3 h. After incubation, FP (480/535) values were measured using a PerkinElmer Envision multi-functional microplate reader, and the ability of the compounds to inhibit the Menin-MLL interaction was determined by calculating the inhibition rate (%) for each compound concentration according to formula 1; formula 1: inhibition rate (%) = 1 - (Maximum mP Control - mP of administration group)/(Maximum mP Control - minimum mP Control) × 100%. Four-parameter analysis was performed in GraphPad Prism software to fit dose-response curves and calculate IC₅₀ values. The specific results are shown in Table 2. A represents that IC50 < 50 nM.

**Table 2. Inhibitory activity against Menin-MLL protein binding**

| Compound No. | IC50 (nM) for inhibitory activity against Menin-MLL protein binding | Compound No. | IC50 (nM) for inhibitory activity against Menin-MLL protein binding |
|---|---|---|---|
| 2 | A | 50 | A |
| 3 | A | 54 | A |
| 4 | A | 56 | A |
| 27 | A | 64 | A |
| 31 | A | 67 | A |
| 32 | A | 69 | A |
| 33 | A | 71 | A |
| 35 | A | 72 | A |
| 36 | A | 73 | A |
| 46 | A | 74 | A |
| 48 | A | | |

The results show that the compounds of the present disclosure exhibited good inhibitory activity against Menin-MLL binding.

### Test Example 3: In Vitro Liver Microsomal Stability

Liver microsome incubation samples were prepared by mixing a PBS buffer (pH 7.4), a liver microsome solution from various species (human, mouse, monkey, dog, and rat) (0.5 mg/mL), a test compound (final concentration: 1 µM), and an NADPH + MgCl₂ solution, followed by incubation at 37 °C and 300 rpm. Samples were collected from the reaction solution at the time points of 15 min and 60 min. Zero-hour samples were prepared by mixing a PBS buffer (pH 7.4), a liver microsome solution (0.5 mg/mL), and a test compound. An acetonitrile solution containing an internal standard was added to the samples, and supernatants were prepared by protein precipitation, diluted, and then assayed by LC-MS/MS.

**Table 3. In vitro metabolic stability of compounds in mouse liver microsomes**

| Compound No. | Residuals at 60 min (%) | Compound No. | Residuals at 60 min (%) |
|---|---|---|---|
| 6 | >60% | 64 | >60% |
| 27 | >60% | 65 | >60% |
| 33 | >60% | 66 | >60% |
| 48 | >60% | 73 | >60% |
| 60 | >60% | 75 | >60% |

**Table 4. In vitro metabolic stability of compounds in human liver microsomes**

| Compound No. | Residuals at 60 min (%) | Compound No. | Residuals at 60 min (%) |
|---|---|---|---|
| 6 | >40% | 60 | >40% |
| 27 | >40% | 64 | >40% |
| 48 | >40% | 69 | >40% |
| 56 | >40% | | |

The compounds of the present disclosure showed good properties in the *in vitro* liver microsomal stability assay.

### Test Example 4: In Vivo Pharmacokinetics

ICR mice weighing 18-22 g were randomized into groups of 9 after 3-5 days of acclimatization. The mice in the intragastric administration group were intragastrically given a related compound solution at a dose of 10 mg/kg, and the mice in the intravenous injection group were intravenously injected with a related compound solution at a dose of 1 mg/kg.

Blood collection time points for intragastric administration were: 15 min, 30 min, 1 h, 2 h, 4 h, 6 h, 8 h, 10 h, and 24 h; blood collection time points for intravenous injection were: 5 min, 15 min, 30 min, 1 h, 2 h, 4 h, 6 h, 8 h, 10 h, and 24 h; blood was collected from the orbit to prepare plasma samples to be tested.

30 µL of each of the plasma samples to be tested and a standard curve sample were taken, and an acetonitrile solution containing an internal standard was added. Supernatants were obtained by protein precipitation, diluted, and then assayed by LC/MS/MS.

Pharmacokinetic parameters were fitted using a non-compartmental model.

The test results show that the compounds of the present application exhibited good *in vivo* pharmacokinetic properties (bioavailability, AUC, T_{1/2}, Cₘₐₓ, and other parameters).

### Test Example 5: In Vivo Drug Effect Study

MV-4-11 human acute monocytic leukemia cells were inoculated subcutaneously into the right axilla of SPF-grade male NOD-SCID mice (source: Lingchang) at 5 × 10⁶ cells/mouse (inoculated by mixing with Matrigel at a ratio of 1:1). When the mean tumor volume reached about 250 mm³, the animals were grouped.

The day of grouping was defined as day 0, and intragastric administration was started on day 0 and performed once a day. The tumor volume and body weight were measured twice to thrice every week, and the data were recorded. The general behavior of the mice was observed and recorded every day. After the experiment was completed, the tumors were extracted, weighed, and photographed.

The detection parameters and the calculation formulas are as follows: $Tumor volume TV \left({mm}^{3}\right) = 1 / 2 \times \left(a\times {b}^{2}\right) ,$ where a represents the long diameter of the tumor, and b represents the short diameter of the tumor. $Relative tumor volume RTV = {TV}_{t} / {TV}_{0} ,$ wherein TV₀ represents the tumor volume on day 0, and TV₁ represents the tumor volume at each measurement. $Relative tumor proliferation rate T / C \left(%\right) = \left({T}_{RTV}/{C}_{RTV}\right) \times 100 % ,$ where T_{RTV} represents RTV of the treatment group, and C_{RTV} represents RTV of the vehicle control group. $Tumor growth inhibition rate TGI \left(%\right) = \left(1-TW/{TW}_{0}\right) \times 100 % ,$ where TW represents the tumor weight of the treatment group, and TW₀ represents the tumor weight of the vehicle control group. $Body weight change rate BWC \left(%\right) = \left({Wt}_{t}-{Wt}_{0}\right) / {Wt}_{0} \times 100 % ,$ wherein Wt₀ is the body weight of the animal on day 0, and Wtt is the body weight of the animal at each measurement.

The test results show that the compounds of the present application could inhibit the growth of tumors *in vivo.* On d21, the tumor volume inhibition rate was greater than 70%, and the tumor weight inhibition rate was greater than 75%.

## Claims

1. A compound of formula (III) or a pharmaceutically acceptable salt thereof: 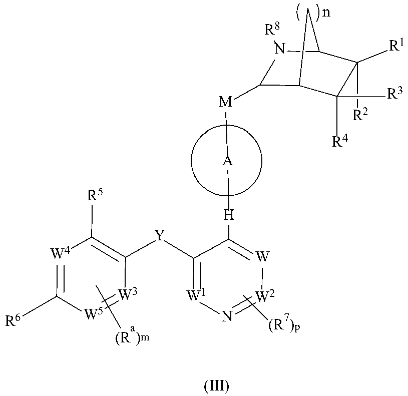 wherein
R¹, R², R³, and R⁴ are each independently selected from the group consisting of hydrogen, halogen, -OH, -CN, C₁₋₆ alkyl, C₂₋₆ alkenyl, C₂₋₆ alkynyl, -C₁₋₆ alkylene-C₃₋₁₀ cycloalkyl, -C₁₋₆ alkylene-3- to 10-membered heterocycloalkyl, -C₁₋₆ alkylene-C₆₋₁₀ aryl, -C₁₋₆ alkylene-5- to 10-membered heteroaryl, -OR^{b1}, -SR^{b1}, -NR^{c1}R^{d1}, -C(O)R^{b1}, -OC(O)R^{b1}, -C(O)OR^{b1}, -S(O)R^{b1}, -S(O)NR^{c1}R^{d1}, -N(R^{c1})S(O)R^{d1}, -S(O)₂R^{b1}, -S(O)₂NR^{c1}R^{d1}, -N(R^{c1})S(O)₂R^{d1}, -OC(O)NR^{c1}R^{d1}, -N(R^{c1})C(O)R^{d1}, and -C(O)NR^{c1}R^{d1}, wherein the C₁₋₆ alkyl, C₂₋₆ alkenyl, C₂₋₆ alkynyl, -C₁₋₆ alkylene-C₃₋₁₀ cycloalkyl, -C₁₋₆ alkylene-3- to 10-membered heterocycloalkyl, -C₁₋₆ alkylene-C₆₋₁₀ aryl, or -C₁₋₆ alkylene-5- to 10-membered heteroaryl is optionally substituted with one or more -OH, -CN, -NH₂, halogen, C₁₋₆ alkyl, or C₁₋₆ heteroalkyl;
or, R¹ and R² are taken together to form =O, =S, or =CR^{c2}R^{d2}; and/or, R³ and R⁴ are taken together to form =O, =S, or =CR^{c2}R^{d2};
R^{b1}, R^{c1}, and R^{d1} are each independently selected from the group consisting of hydrogen, C₁₋₆ alkyl, C₂₋₆ alkenyl, C₂₋₆ alkynyl, C₁₋₆ heteroalkyl, C₃₋₁₀ cycloalkyl, 3- to 10-membered heterocycloalkyl, C₆₋₁₀ aryl, and 5- to 10-membered heteroaryl, wherein the C₁₋₆ alkyl, C₂₋₆ alkenyl, C₂₋₆ alkynyl, C₃₋₁₀ cycloalkyl, 3- to 10-membered heterocycloalkyl, C₆₋₁₀ aryl, or 5- to 10-membered heteroaryl is optionally substituted with one or more halogen, -CN, -OH, -NH₂, C₁₋₆ alkyl, or C₁₋₆ heteroalkyl;
R^{c2} and R^{d2} are each independently selected from the group consisting of hydrogen, C₁₋₆ alkyl, C₂₋₆ alkenyl, C₂₋₆ alkynyl, and C₁₋₆ heteroalkyl, wherein the C₁₋₆ alkyl, C₂₋₆ alkenyl, C₂₋₆ alkynyl, or C₁₋₆ heteroalkyl is optionally substituted with one or more halogen, -CN, -OH, or -NH₂;
R⁸ is selected from the group consisting of hydrogen and C₁₋₆ alkyl, wherein the C₁₋₆ alkyl is optionally substituted with 1 or more groups selected from the group consisting of halogen, -CN, -OH, and -NH₂;
Y is selected from the group consisting of O, S, CR^{x}R^{y}, and NR^{z}, wherein R^{x}, R^{y}, or R^{z} is each independently selected from the group consisting of hydrogen and C₁₋₆ alkyl;
W, W¹, W², W³, W⁴, and W⁵ are each independently selected from the group consisting of N, C, and CH;
ring A is selected from the group consisting of the following groups optionally substituted with one or more R^{a1}: C₃₋₁₂ cycloalkylene, 3- to 12-membered heterocyclylene, C₆₋₁₀ arylene, and 5- to 12-membered heteroarylene;
H is selected from the group consisting of a bond, NR^{z}, C₃₋₈ cycloalkyl, and 3-8 heterocycloalkyl, wherein R^{z} is selected from the group consisting of hydrogen and C₁₋₆ alkyl;
each R^{a1} is independently selected from the group consisting of halogen, -OH, -CN, -NH₂, =O, =S, C₁₋₆ alkyl, C₂₋₆ alkenyl, C₂₋₆ alkynyl, and C₁₋₆ heteroalkyl;
R⁵ is selected from the group consisting of halogen, -OH, -CN, -NH₂, and the following groups optionally substituted with one or more R^{a"}: -C(O)NR^{a'}R^{b'}, -N(R^{b'})C(O)R^{b'}, -N(R^{b'})C(O)OR^{b'}, -OC(O)NR^{b'}R^{b'}, -C(O)R^{b'}, -S(O)R^{b'}, -S(O)₂R^{b'}, -N(R^{b'})S(O)₂R^{b'}, -N(R^{b'})S(O)R^{b'}, -S(O)NR^{b'}R^{b'}, -S(O)₂NR^{b'}R^{b'}, -OC(O)R^{b'}, -C(O)OR^{b'}, C₃₋₁₀ cycloalkyl, 3- to 10-membered heterocycloalkyl, C₆₋₁₀ aryl, and 5- to 10-membered heteroaryl;
R^{a'} is selected from the group consisting of hydrogen, C₁₋₆ alkyl, C₂₋₆ alkenyl, C₂₋₆ alkynyl, C₃₋₆ cycloalkyl, and C₃₋₆ heterocycloalkyl;
each R^{b'} is independently selected from the group consisting of hydrogen, C₁₋₆ alkyl, C₂₋₆ alkenyl, C₂₋₆ alkynyl, C₃₋₁₀ cycloalkyl, 3- to 10-membered heterocycloalkyl, C₆₋₁₀ aryl, and 5- to 10-membered heteroaryl, wherein the C₁₋₆ alkyl, C₃₋₁₀ cycloalkyl, 3- to 10-membered heterocycloalkyl, C₆₋₁₀ aryl, or 5- to 10-membered heteroaryl is optionally substituted with one or more halogen, -CN, -OH, or -NH₂;
each R^{a"} is independently selected from the group consisting of halogen, -OH, -CN, -NH₂, -NH(C₁₋₆ alkyl), -N(C₁₋₆ alkyl)₂, C₁₋₆ alkyl, C₂₋₆ alkenyl, C₂₋₆ alkynyl, C₁₋₆ alkyl-O-, C₃₋₁₀ cycloalkyl, 3- to 10-membered heterocycloalkyl, C₆₋₁₀ aryl, and 5- to 10-membered heteroaryl, wherein the -NH(C₁₋₆ alkyl), -N(C₁₋₆ alkyl)₂, C₁₋₆ alkyl, C₂₋₆ alkenyl, C₂₋₆ alkynyl, C₁₋₆ alkyl-O-, C₃₋₁₀ cycloalkyl, 3- to 10-membered heterocycloalkyl, C₆₋₁₀ aryl, or 5- to 10-membered heteroaryl is optionally substituted with one or more halogen, -OH, -CN, or -NH₂;
R⁶ is selected from the group consisting of hydrogen, halogen, -OH, -CN, -NH₂, C₁₋₆ alkyl, C₂₋₆ alkenyl, C₂₋₆ alkynyl, C₁₋₆ heteroalkyl, C₃₋₁₀ cycloalkyl, 3- to 10-membered heterocycloalkyl, C₆₋₁₀ aryl, and 5- to 10-membered heteroaryl, wherein the C₁₋₆ alkyl, C₂₋₆ alkenyl, C₂₋₆ alkynyl, C₁₋₆ heteroalkyl, C₃₋₁₀ cycloalkyl, 3- to 10-membered heterocycloalkyl, C₆₋₁₀ aryl, or 5- to 10-membered heteroaryl is optionally substituted with one or more halogen, - CN, -OH, -NH₂, C₁₋₆ alkyl, C₁₋₆ alkyl-O-, C₂₋₆ alkenyl, C₂₋₆ alkynyl, C₃₋₁₀ cycloalkyl, or 3- to 10-membered heterocycloalkyl;
R^{a} and R⁷ are each independently selected from the group consisting of halogen, -CN, -OH, -NH₂, C₁₋₆ alkyl, C₂₋₆ alkenyl, C₂₋₆ alkynyl, -OC₁₋₆ alkyl, C₃₋₆ cycloalkyl, and 3- to 6-membered heterocycloalkyl, wherein the C₁₋₆ alkyl, C₂₋₆ alkenyl, C₂₋₆ alkynyl, -OC₁₋₆ alkyl, C₃₋₆ cycloalkyl, or 3- to 6-membered heterocycloalkyl is optionally substituted with one or more halogen, -CN, -OH, or -NH₂;
M is selected from the group consisting of -C(O)- and -(CR^{u}R^{v})_{q}-; R^{u} and R^{v} are each independently selected from the group consisting of hydrogen, halogen, -CN, -OH, -NH₂, C₁₋₆ alkyl, C₂₋₆ alkenyl, C₂₋₆ alkynyl, -OC₁₋₆ alkyl, C₃₋₁₀ cycloalkyl, and 3- to 10-membered heterocycloalkyl;
n is selected from the group consisting of 1, 2, 3, and 4;
m and p are each independently selected from the group consisting of 0, 1, 2, and 3;
q is selected from the group consisting of 1, 2, and 3;
provided that when 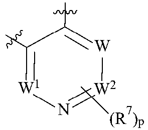 is 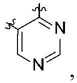 R⁵ is not selected from

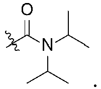

2. The compound or the pharmaceutically acceptable salt thereof according to claim 1, wherein R¹, R², R³, and R⁴ are each independently selected from the group consisting of hydrogen, halogen, -OH, -CN, -NH₂, C₁₋₄ alkyl, C₂₋₄ alkenyl, C₂₋₄ alkynyl, -C₁₋₄ alkylene-C₃₋₆ cycloalkyl, -C₁₋₄ alkylene-3- to 6-membered heterocycloalkyl, -C₁₋₄ alkylene-phenyl, -C₁₋₄ alkylene-5- to 6-membered heteroaryl, -OR^{b1}, -SR^{b1}, -NR^{c1}R^{d1}, -C(O)R^{b1}, -OC(O)R^{b1}, - C(O)OR^{b1}, -S(O)R^{b1}, -S(O)NR^{c1}R^{d1}, -N(R^{c1})S(O)R^{d1}, -S(O)₂R^{b1}, -S(O)₂NR^{c1}R^{d1}, -N(R^{c1})S(O)₂R^{d1}, -OC(O)NR^{c1}R^{d1}, -N(R^{c1})C(O)R^{d1}, and -C(O)NR^{c1}R^{d1}, wherein the C₁₋₄ alkyl, C₂₋₄ alkenyl, C₂₋₄ alkynyl, -C₁₋₄ alkylene-C₃₋₆ cycloalkyl, -C₁₋₄ alkylene-3- to 6-membered heterocycloalkyl, -C₁₋₄ alkylene-phenyl, or -C₁₋₄ alkylene-5- to 6-membered heteroaryl is optionally substituted with one or more -OH, -CN, -NH₂, halogen, C₁₋₆ alkyl, or -C₁₋₆ heteroalkyl;
or, R¹ and R² are taken together to form =O, =S, or =CR^{c2}R^{d2}; and/or, R³ and R⁴ are taken together to form =O, =S, or =CR^{c2}R^{d2};
optionally, R¹, R², R³, and R⁴ are each independently selected from the group consisting of hydrogen, halogen, -OH, -CN, -NH₂, C₁₋₃ alkyl, C₂₋₃ alkenyl, C₂₋₃ alkynyl, -CH₂-C₃₋₆ cycloalkyl, -CH₂-3- to 6-membered heterocycloalkyl, - CH₂-phenyl, and -CH₂-5- to 6-membered heteroaryl, wherein the C₁₋₃ alkyl, C₂₋₃ alkenyl, C₂₋₃ alkynyl, -CH₂-C₃₋₆ cycloalkyl, -CH₂-3- to 6-membered heterocycloalkyl, -CH₂-phenyl, or -CH₂-5- to 6-membered heteroaryl is optionally substituted with one or more halogen, OH, -CN, -NH₂, or C₁₋₄ alkyl;
or, R¹ and R² are taken together to form =O or =CH₂; and/or, R³ and R⁴ are taken together to form =O or =CH₂;
optionally, R¹, R², R³, and R⁴ are each independently selected from the group consisting of hydrogen, halogen, -OH, -CN, -NH₂, and C₁₋₃ alkyl;
or, R¹ and R² are taken together to form =O or =CH₂; and/or, R³ and R⁴ are taken together to form =O or =CH₂;
optionally, R³ and R⁴ are selected from hydrogen, and R¹ and R² are taken together to form =O or =CH₂; or, R¹ and
R² are selected from hydrogen, and R³ and R⁴ are taken together to form =O or =CH₂.

3. The compound or the pharmaceutically acceptable salt thereof according to claim 1 or 2, wherein R^{b1}, R^{c1}, and R^{d1} are each independently selected from the group consisting of hydrogen, C₁₋₆ alkyl, C₂₋₆ alkenyl, C₂₋₆ alkynyl, C₃₋₁₀ cycloalkyl, 3- to 10-membered heterocycloalkyl, C₆₋₁₀ aryl, and 5- to 10-membered heteroaryl, wherein the C₁₋₆ alkyl, C₂₋₆ alkenyl, C₂₋₆ alkynyl, C₃₋₁₀ cycloalkyl, 3- to 10-membered heterocycloalkyl, C₆₋₁₀ aryl, or 5- to 10-membered heteroaryl is optionally substituted with one or more halogen, -CN, -OH, -NH₂, C₁₋₆ alkyl, or C₁₋₆ heteroalkyl;
or, R^{b1}, R^{c1}, and R^{d1} are each independently selected from the group consisting of hydrogen, C₁₋₄ alkyl, C₂₋₄ alkenyl, C₂₋₄ alkynyl, C₃₋₆ cycloalkyl, 3- to 6-membered heterocycloalkyl, phenyl, and 5- to 6-membered heteroaryl, wherein the C₁₋₆ alkyl, C₂₋₆ alkenyl, C₂₋₆ alkynyl, C₃₋₆ cycloalkyl, 3- to 6-membered heterocycloalkyl, phenyl, or 5- to 6-membered heteroaryl is optionally substituted with one or more halogen, -CN, -OH, -NH₂, C₁₋₄ alkyl, or C₁₋₄ heteroalkyl;
or, R^{b1}, R^{c1}, and R^{d1} are each independently selected from the group consisting of hydrogen, C₁₋₄ alkyl, C₂₋₄ alkenyl, and C₂₋₄ alkynyl, wherein the C₁₋₄ alkyl, C₂₋₄ alkenyl, or C₂₋₄ alkynyl is optionally substituted with one or more halogen, -CN, -OH, or -NH₂;
or, R^{b1}, R^{c1}, and R^{d1} are each independently selected from the group consisting of hydrogen and C₁₋₃ alkyl, wherein the C₁₋₃ alkyl is optionally substituted with one or more halogen, -CN, -OH, or -NH₂;
or, R^{b1}, R^{c1}, and R^{d1} are each independently hydrogen or C₁₋₃ alkyl;
or, R^{b1}, R^{c1}, and R^{d1} are each independently methyl.

4. The compound or the pharmaceutically acceptable salt thereof according to any one of claims 1-3, wherein R^{c2} and R^{d2} are each independently selected from the group consisting of hydrogen, C₁₋₄ alkyl, C₂₋₄ alkenyl, C₂₋₄ alkynyl, and C₁₋₄ heteroalkyl, wherein the C₁₋₄ alkyl, C₂₋₄ alkenyl, C₂₋₄ alkynyl, or C₁₋₄ heteroalkyl is optionally substituted with one or more halogen, -CN, -OH, or -NH₂;
or, R^{c2} and R^{d2} are each independently selected from the group consisting of hydrogen, C₁₋₄ alkyl, C₂₋₄ alkenyl, C₂₋₄ alkynyl, and C₁₋₄ heteroalkyl;
or, R^{c2} and R^{d2} are each independently selected from the group consisting of hydrogen and C₁₋₃ alkyl;
or, R^{c2} and R^{d2} are each independently selected from hydrogen;
optionally, R⁸ is selected from the group consisting of hydrogen and C₁₋₄ alkyl; or, R⁸ is selected from the group consisting of hydrogen and methyl; or, R⁸ is hydrogen.

5. The compound or the pharmaceutically acceptable salt thereof according to any one of claims 1-4, wherein Y is selected from the group consisting of O, S, CR^{x}R^{y}, and NR^{z}, wherein R^{x}, R^{y}, and R^{z} are each independently selected from the group consisting of hydrogen and C₁₋₃ alkyl;
or, Y is selected from the group consisting of O, S, CR^{x}R^{y}, and NR^{z}, wherein R^{x}, R^{y}, and R^{z} are each independently selected from the group consisting of hydrogen and methyl;
or, Y is selected from the group consisting of O, S, CH₂, and NH;
or, Y is selected from O;
optionally, M is selected from the group consisting of -CH₂- and -C(O)-;
or, M is selected from -C(O)-;
optionally, R^{u} and R^{v} are each independently selected from the group consisting of hydrogen, halogen, -CN, -OH, - NH₂, and C₁₋₃ alkyl;
or, R^{u} and R^{v} are selected from hydrogen.

6. The compound or the pharmaceutically acceptable salt thereof according to any one of claims 1-5, wherein W is selected from the group consisting of N, C, and CH; or, W is selected from the group consisting of N and CH;
optionally, W¹ is selected from the group consisting of N, C, and CH; or, W¹ is selected from the group consisting of N and CH;
optionally, W² is selected from the group consisting of N, C, and CH; or, W² is selected from CH; optionally, W³ is selected from the group consisting of N and CH;
optionally, W and W¹ are N, and W² is CH or C;
optionally, W⁴ is CH or C, and W³ and W⁵ are selected from the group consisting of N, C, and CH;
optionally, W⁴ and W⁵ are selected from CH;
optionally, W¹, W², W³, W⁴, and W⁵ are selected from CH;
or, W is N, and W¹, W², W³, W⁴, and W⁵ are each independently selected from the group consisting of C and CH;
or, W¹ is N, and W, W², W³, W⁴, and W⁵ are each independently selected from the group consisting of C and CH;
or, W and W¹ are N, and W², W³, W⁴, and W⁵ are each independently selected from the group consisting of C and CH;
or, W and W³ are each independently selected from the group consisting of N and CH, and W¹, W², W³, W⁴, and W⁵ are selected from CH;
or, W, W¹, and W³ are each independently selected from the group consisting of CH and N, and W², W⁴, and W⁵ are selected from CH.

7. The compound or the pharmaceutically acceptable salt thereof according to any one of claims 1-6, wherein the ring A is selected from the group consisting of the following groups optionally substituted with one or more R^{a1}: C₅₋₁₂ membered cycloalkylene, 5- to 12-membered heterocyclylene, C₆ arylene, and 5- to 6-membered heteroarylene; or, the ring A is selected from 7- to 9-membered heterospirocycloalkylene optionally substituted with one or more R^{a1}; optionally, the ring A contains 1, 2, or 3 heteroatoms selected from the group consisting of N, O, and S; or, the ring A is selected from 9-membered heterospirocycloalkylene optionally substituted with one or more R^{a1};
or, the ring A is selected from 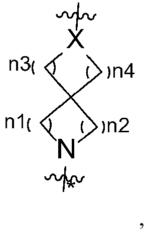 wherein n1, n2, n3, and n4 are each independently selected from the group consisting of 1, 2, and 3, and X is selected from the group consisting of N and CH;
or, the ring A is selected from the group consisting of 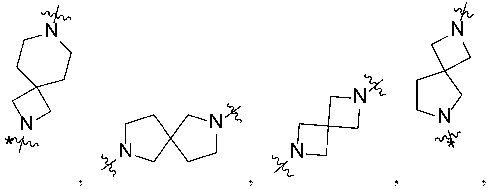 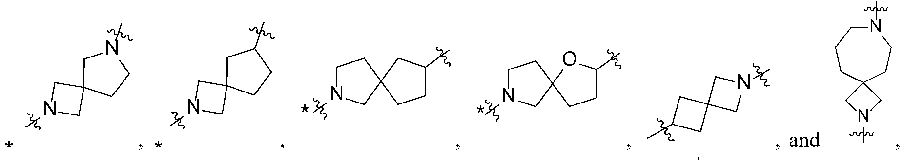 wherein * indicates that a bond at the position is linked to the structural fragment 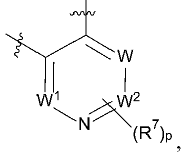 and a bond on the other side is linked to the structural fragment 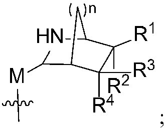 or, the ring A is 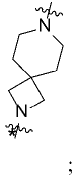
optionally, each R^{a1} is independently selected from the group consisting of halogen, -OH, -CN, -NH₂, =O, =S, and C₁₋₆ alkyl;
or, each R^{a1} is independently selected from the group consisting of halogen, -OH, -CN, -NH₂, =O, =S, and C₁₋₃ alkyl.

8. The compound or the pharmaceutically acceptable salt thereof according to any one of claims 1-7, wherein R⁵ is selected from the group consisting of halogen, -OH, -CN, -NH₂, and the following groups optionally substituted with one or more R^{a"}: -C(O)NR^{a'}R^{b'}, -N(R^{b'})C(O)R^{b'}, -N(R^{b'})(CO)OR^{b'}, -O(CO)NR^{b'}R^{b'}, -C(O)R^{b'}, -S(O)R^{b'}, -S(O)₂R^{b'}, - N(R^{b'})S(O)₂R^{b'}, -N(R^{b'})S(O)R^{b'}, -S(O)NR^{b'}R^{b'}, -S(O)₂NR^{b'}R^{b'}, -OC(O)R^{b'}, -C(O)OR^{b'}, C₃₋₆ cycloalkyl, 3- to 6-membered heterocycloalkyl, phenyl, and 5- to 6-membered heteroaryl;
or, R⁵ is selected from the group consisting of halogen, -OH, -CN, -NH₂, and the following groups optionally substituted with one or more R^{a"}: -C(O)R^{b'}, -C(O)NR^{a'}R^{b'}, -N(R^{b'})C(O)R^{b'}, -N(R^{b'})C(O)OR^{b'}, and 5- to 6-membered heteroaryl;
or, R⁵ is selected from the group consisting of halogen and the following groups optionally substituted with one or more R^{a"}: 4- to 8-membered heterocycloalkyl C(O)-, C₈₋₁₀ cycloalkyl NHC(O)-, -C(O)N(C₁₋₄ alkyl)₂, -C(O)N(C₁₋₄ alkyl)C₃₋₆ cycloalkyl, -C(O)N(C₁₋₄ alkyl)(4- to 6-membered heterocycloalkyl), -NHC(O)C₁₋₄ alkyl, -NHC(O)C₃₋₆ cycloalkyl, -N(C₁₋₄ alkyl)C(O)OC₁₋₄ alkyl, and 5- to 6-membered heteroaryl;
or, R⁵ is selected from the group consisting of halogen, -C(O)R^{b'}, -C(O)NR^{a'}R^{b'}, -N(R^{b'})C(O)OR^{b'}, -N(R^{b'})C(O)R^{b'}, and 5- to 6-membered heteroaryl, wherein the -C(O)NR^{a'}R^{b'}, -C(O)R^{b'}, -N(R^{b'})C(O)OR^{b'}, -N(R^{b'})C(O)R^{b'}, or 5- to 6-membered heteroaryl is optionally substituted with one or more of the following groups: halogen, -OH, -CN, -NH₂, C₁₋₃ alkyl optionally substituted with one or more halogen or -OH, -OC₁₋₃ alkyl, C₃₋₆ cycloalkyl, or 4- to 6-membered heterocycloalkyl;
or, R⁵ is selected from the group consisting of F, Cl, Br, 5- to 7-membered heterocycloalkyl C(O)-, -C(O)N(C₁₋₄ alkyl)₂, C₉₋₁₀ cycloalkyl NHC(O)-, -C(O)N(C₁₋₃ alkyl)C₃₋₅ cycloalkyl, -C(O)N(C₁₋₃ alkyl)(4- to 6-membered heterocycloalkyl), -NHC(O)C₁₋₃ alkyl, -NHC(O)C₃₋₅ cycloalkyl, -N(C₁₋₃ alkyl)C(O)OC₁₋₃ alkyl, and 5- to 6-membered heteroaryl, wherein the 5- to 7-membered heterocycloalkyl C(O)-, -C(O)N(C₁₋₄ alkyl)₂, C₉₋₁₀ cycloalkyl NHC(O)-, -C(O)N(C₁₋₃ alkyl)C₃₋₅ cycloalkyl, -C(O)N(C₁₋₃ alkyl)(4- to 6-membered heterocycloalkyl), -NHC(O)C₁₋₃ alkyl, -NHC(O)C₃₋₅ cycloalkyl, -N(C₁₋₃ alkyl)C(O)OC₁₋₃ alkyl, or 5- to 6-membered heteroaryl is optionally substituted with one or more of the following groups: halogen, -OH, -CN, -NH₂, C₁₋₃ alkyl optionally substituted with one or more halogen or -OH, -OC₁₋₃ alkyl, C₃₋₄ cycloalkyl, or 4- to 5-membered heterocycloalkyl;
or, R⁵ is selected from the group consisting of Br, pyrazolyl, imidazolyl, thiazolyl, triazolyl, pyridinyl, pyrimidinyl, pyrazinyl, pyridazinyl, -C(O)N(C₁₋₄ alkyl)₂, -C(O)-pyrrolidinyl, -C(O)-piperidinyl, -C(O)-morpholinyl, -C(O)-piperazinyl, -C(O)azabicycloheptyl, adamantyl NHC(O)-, -C(O)N(C₁₋₃ alkyl)cyclopropyl, -C(O)N(C₁₋₃ alkyl)oxetanyl, -NHC(O)C₁₋₃ alkyl, -NHC(O)-cyclopropyl, and -N(C₁₋₃ alkyl)C(O)OC₁₋₃ alkyl, wherein the pyrazolyl, imidazolyl, thiazolyl, triazolyl, pyridinyl, pyrimidinyl, pyrazinyl, pyridazinyl, -C(O)-pyrrolidinyl, -C(O)-piperidinyl, -C(O)-morpholinyl, or -C(O)-piperazinyl is optionally substituted with 1 or 2 groups selected from the group consisting of F, Cl, -OH, -CN, methyl, -CF₃, -OCH₃, cyclopropyl, isopropyl, 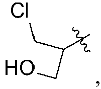 and oxetanyl;
or, R⁵ is selected from the group consisting of Br, 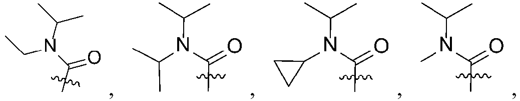 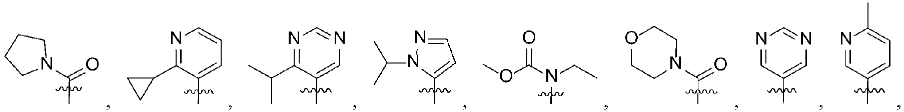 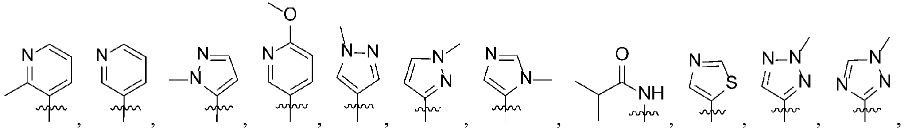 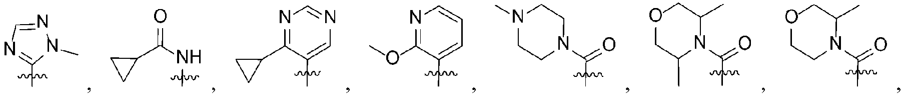 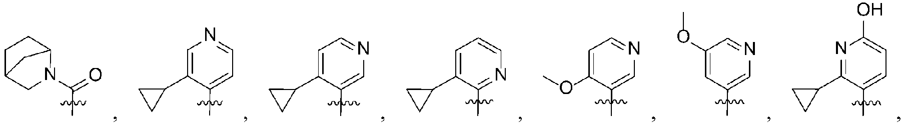 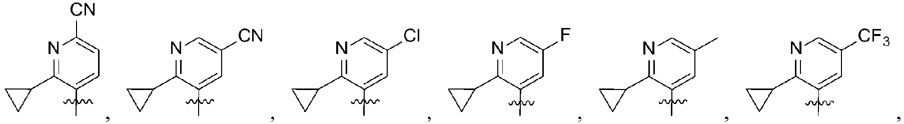 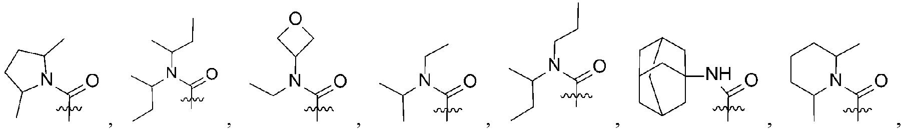 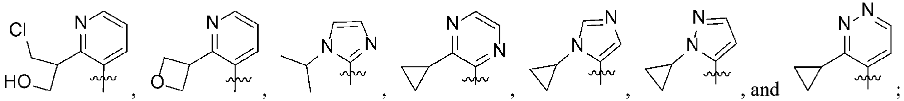
optionally, each R^{a'} is independently selected from the group consisting of hydrogen, C₁₋₆ alkyl, C₃₋₆ cycloalkyl, and 3- to 6-membered heterocycloalkyl;
or, each R^{a'} is independently selected from the group consisting of hydrogen, C₁₋₄ alkyl, and C₃₋₄ cycloalkyl;
or, each R^{a'} is independently selected from the group consisting of hydrogen, methyl, ethyl, isopropyl, 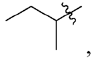 and cyclopropyl;
optionally, each R^{b'} is independently selected from the group consisting of hydrogen, C₁₋₆ alkyl, C₃₋₁₀ cycloalkyl, 4-to 10-membered heterocycloalkyl, phenyl, and 5- to 6-membered heteroaryl, wherein the C₁₋₆ alkyl, C₃₋₁₀ cycloalkyl, 4- to 10-membered heterocycloalkyl, phenyl, and 5- to 6-membered heteroaryl is optionally substituted with one or more halogen, -CN, -OH, or -NH₂;
or, each R^{b'} is independently selected from the group consisting of hydrogen and the following groups optionally substituted with one or more halogen: C₁₋₆ alkyl, C₃₋₉ cycloalkyl, or 4- to 9-membered heterocycloalkyl;
or, each R^{b'} is independently selected from the group consisting of hydrogen, methyl, ethyl, n-propyl, isopropyl, 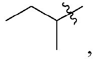 cyclopropyl, 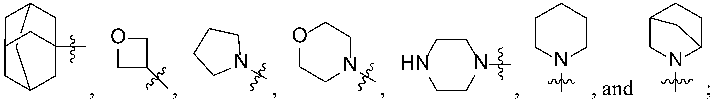 optionally, each R^{a"} is independently selected from the group consisting of halogen, -OH, -CN, -NH₂, -NH(C₁₋₄ alkyl), -N(C₁₋₄ alkyl)₂, C₁₋₄ alkyl, C₂₋₄ alkenyl, C₂₋₄ alkynyl, C₁₋₄ alkyl-O-, C₃₋₆ cycloalkyl, and 3- to 6-membered heterocycloalkyl, wherein the -NH(C₁₋₄ alkyl), -N(C₁₋₄ alkyl)₂, C₁₋₄ alkyl, C₂₋₄ alkenyl, C₂₋₄ alkynyl, C₁₋₄ alkyl-O-, C₃₋₆ cycloalkyl, or 3- to 6-membered heterocycloalkyl is optionally substituted with one or more halogen, -OH, -CN, or -NH₂;
or, each R^{a"} is independently selected from the group consisting of halogen, -OH, -CN, -NH₂, C₁₋₄ alkyl, -NH(C₁₋₄ alkyl), -OC₁₋₄ alkyl, C₃₋₆ cycloalkyl, and 3- to 6-membered heterocycloalkyl, wherein the C₁₋₄ alkyl, -NH(C₁₋₄ alkyl), -OC₁₋₄ alkyl, C₃₋₆ cycloalkyl, or 3- to 6-membered heterocycloalkyl is optionally substituted with one or more halogen or -OH;
or, each R^{a"} is independently selected from the group consisting of halogen, -OH, -CN, -NH₂, C₁₋₄ alkyl, -OC₁₋₄ alkyl, C₃₋₅ cycloalkyl, and 3- to 5-membered heterocycloalkyl, wherein the C₁₋₄ alkyl, -OC₁₋₄ alkyl, C₃₋₅ cycloalkyl, or 3- to 5-membered heterocycloalkyl is optionally substituted with 1, 2, or 3 groups selected from the group consisting of: halogen, -OH, -CN, and -NH₂;
or, each R^{a"} is independently selected from the group consisting of F, Cl, -OH, -CN, -OCH₃, -CF₃, methyl, isopropyl, cyclopropyl, 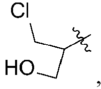 and oxetanyl.

9. The compound or the pharmaceutically acceptable salt thereof according to any one of claims 1-8, wherein R⁶ is selected from the group consisting of hydrogen, halogen, -OH, -CN, -NH₂, C₁₋₆ alkyl, C₂₋₆ alkenyl, C₂₋₆ alkynyl, and C₁₋₆ heteroalkyl, wherein the C₁₋₆ alkyl, C₂₋₆ alkenyl, C₂₋₆ alkynyl, or C₁₋₆ heteroalkyl is optionally substituted with one or more halogen, -CN, -OH, or -NH₂;
or, R⁶ is selected from the group consisting of hydrogen, halogen, -OH, -CN, -NH₂, C₁₋₄ alkyl, C₂₋₄ alkenyl, C₂₋₄ alkynyl, and C₁₋₄ heteroalkyl, wherein the C₁₋₄ alkyl, C₂₋₄ alkenyl, C₂₋₄ alkynyl, or C₁₋₄ heteroalkyl is optionally substituted with one or more halogen, -CN, -OH, or -NH₂;
or, R⁶ is selected from the group consisting of hydrogen, halogen, -OH, -CN, -NH₂, C₁₋₆ alkyl, and C₁₋₆ haloalkyl;
or, R⁶ is selected from the group consisting of hydrogen, halogen, -OH, -CN, -NH₂, C₁₋₄ alkyl, C₂₋₄ alkenyl, C₂₋₄ alkynyl, and C₁₋₄ heteroalkyl;
or, R⁶ is selected from the group consisting of hydrogen, halogen, and C₁₋₆ alkyl;
or, R⁶ is selected from the group consisting of hydrogen, F, Cl, Br, and methyl; or, R⁶ is selected from the group consisting of hydrogen, F, and methyl.

10. The compound or the pharmaceutically acceptable salt thereof according to any one of claims 1-9, wherein R^{a} and R⁷ are each independently selected from the group consisting of halogen, -CN, -OH, -NH₂, C₁₋₄ alkyl, C₂₋₄ alkenyl, C₂₋₄ alkynyl, -OC₁₋₄ alkyl, C₃₋₆ cycloalkyl, and 3- to 6-membered heterocycloalkyl, wherein the C₁₋₄ alkyl, C₂₋₄ alkenyl, C₂₋₄ alkynyl, -OC₁₋₄ alkyl, C₃₋₆ cycloalkyl, or 3- to 6-membered heterocycloalkyl is optionally substituted with one or more halogen, -CN, -OH, or -NH₂;
optionally, each R⁷ is independently selected from the group consisting of halogen, -CN, -OH, -NH₂, C₁₋₄ alkyl, C₂₋₄ alkenyl, C₂₋₄ alkynyl, -OC₁₋₄ alkyl, C₃₋₆ cycloalkyl, and 3- to 6-membered heterocycloalkyl;
or, each R⁷ is independently selected from cyclopropyl;
optionally, each R^{a} is independently selected from the group consisting of halogen, -CN, -OH, -NH₂, C₁₋₄ alkyl, C₂₋₄ alkenyl, C₂₋₄ alkynyl, and -OC₁₋₄ alkyl;
or, each R^{a} is independently selected from the group consisting of halogen, -CN, -OH, -NH₂, and C₁₋₄ alkyl;
or, each R^{a} is independently selected from the group consisting of F, Cl, and Br; or, each R^{a} is independently F;
optionally, m is selected from the group consisting of 0, 1, and 2; or, m is selected from the group consisting of 0 and 1; or, m is selected from 0; or, m is selected from 1;
optionally, p is selected from the group consisting of 0, 1, and 2; or, p is selected from the group consisting of 0 and 1; or, p is selected from 0;
optionally, n is selected from the group consisting of 1 and 2; or, n is selected from 2;
optionally, q is selected from 1.

11. The compound or the pharmaceutically acceptable salt thereof according to any one of claims 1-10, wherein H is selected from a bond; optionally, R⁸ is selected from hydrogen; R¹ and R² are hydrogen; and R³ and R⁴ are taken together to form =CH₂; W and W¹ are N, and W² is CH or C.

12. The compound or the pharmaceutically acceptable salt thereof according to any one of claims 1-11, wherein the compound or the pharmaceutically acceptable salt thereof is selected from the group consisting of compounds of formula (I), formula (I'), formula (II), formula (II'), formula (III'), formula (IA), formula (IIA), formula (IB), formula (IIB), formula (IC), formula (IID), formula (IIC), formula (IA-1), formula (IIA-1), formula (IA-2), formula (IB-1), and formula (IC-1) or pharmaceutically acceptable salts thereof: 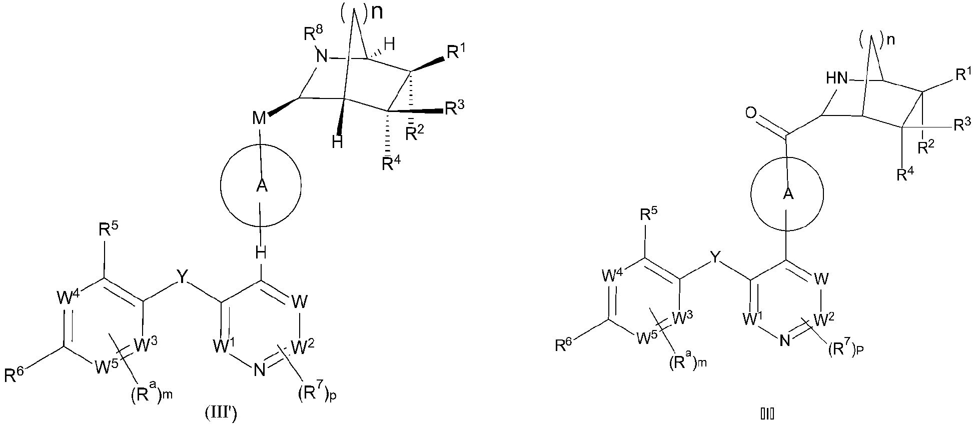 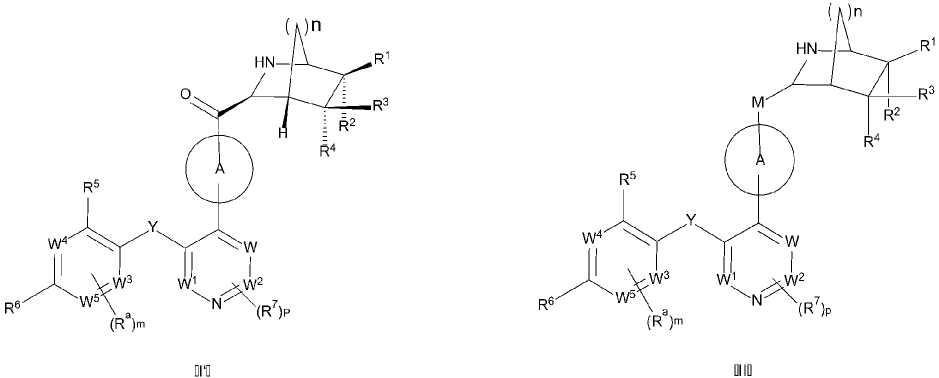 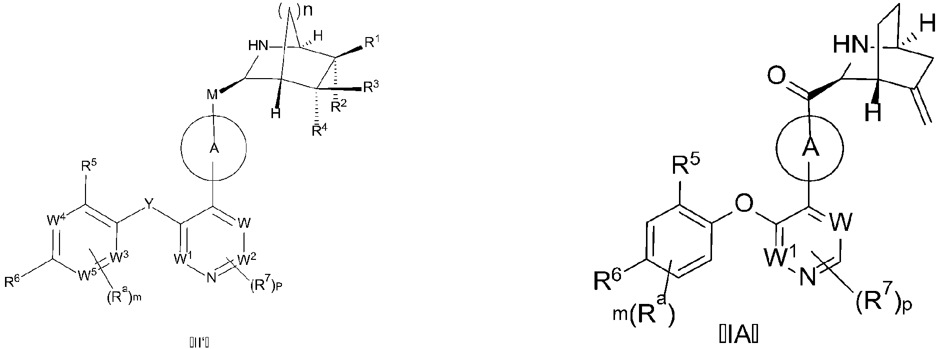 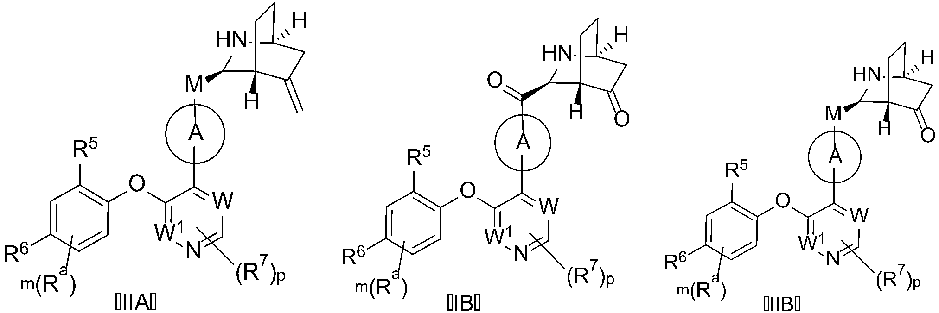 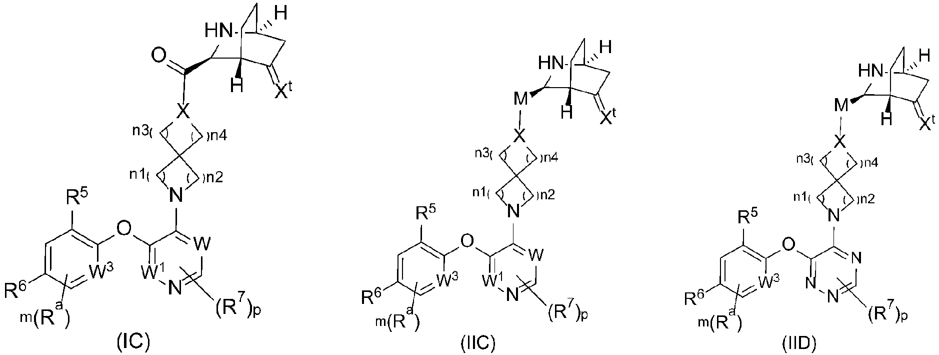 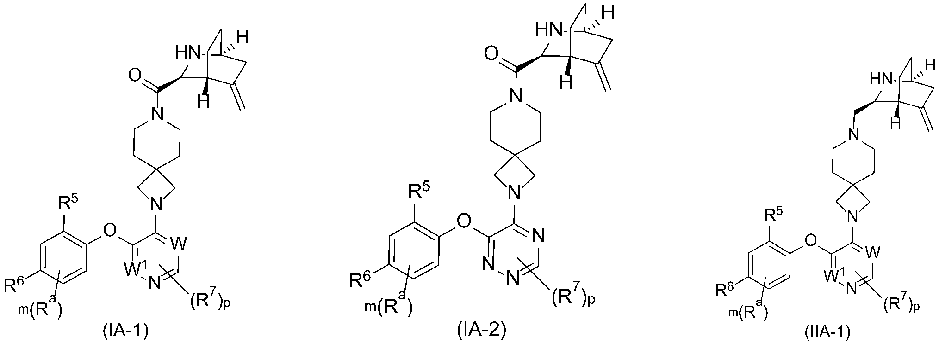 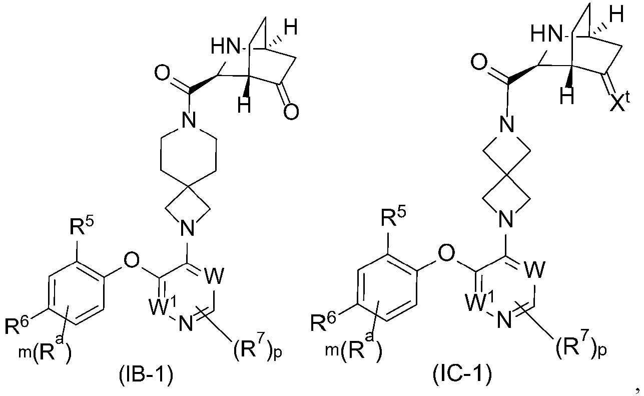 wherein
X^{t} is selected from the group consisting of O and CH(R^{c2}), wherein R^{c2} is selected from the group consisting of hydrogen, C₁₋₆ alkyl, C₂₋₆ alkenyl, C₂₋₆ alkynyl, and C₁₋₆ heteroalkyl, wherein the C₁₋₆ alkyl, C₂₋₆ alkenyl, C₂₋₆ alkynyl, or C₁₋₆ heteroalkyl is optionally substituted with one or more halogen, -CN, -OH, or -NH₂;
or, R^{c2} is selected from the group consisting of hydrogen and C₁₋₃ alkyl; or, R^{c2} is selected from hydrogen.

13. A compound as below or a pharmaceutically acceptable salt thereof:

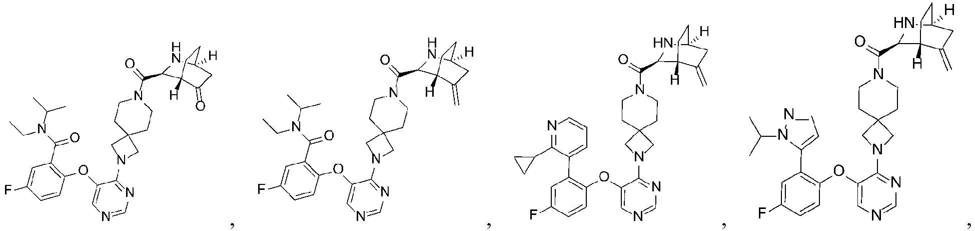

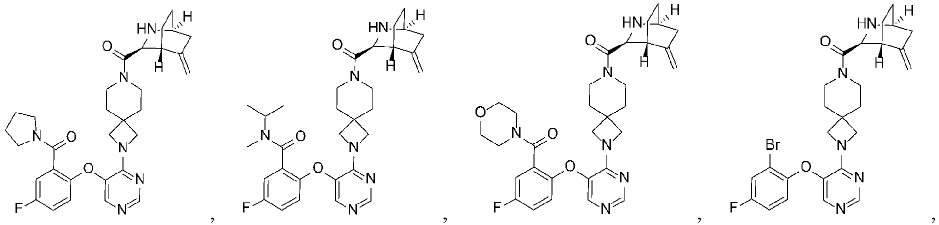

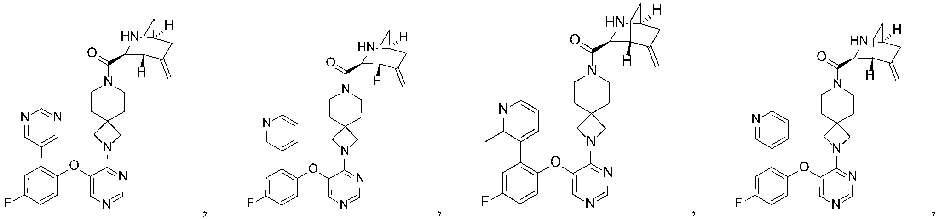

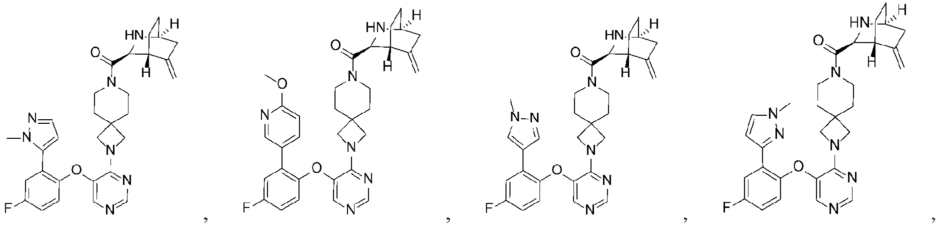

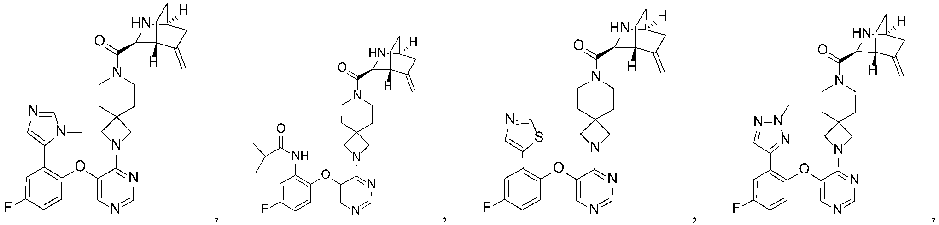

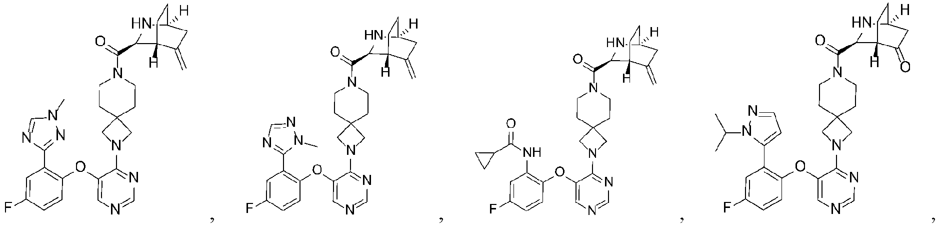

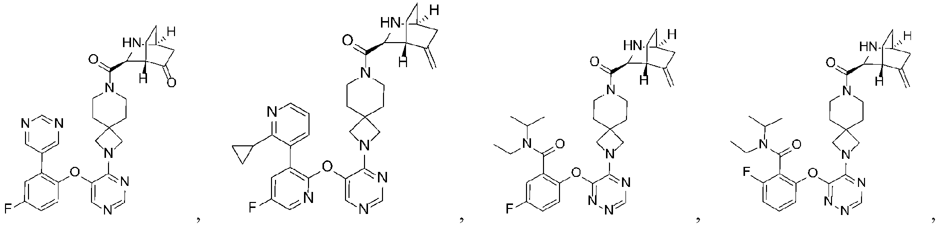

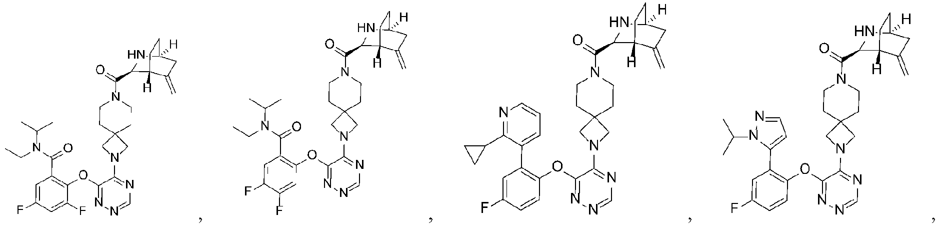

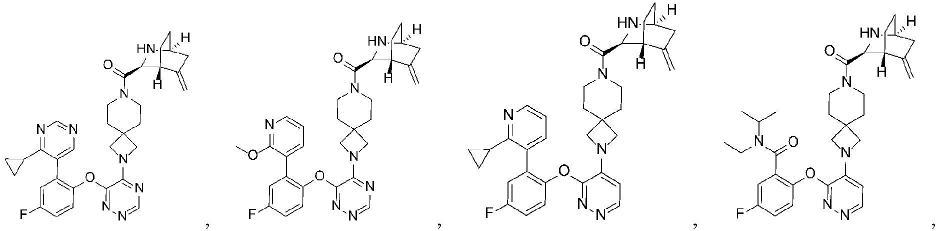

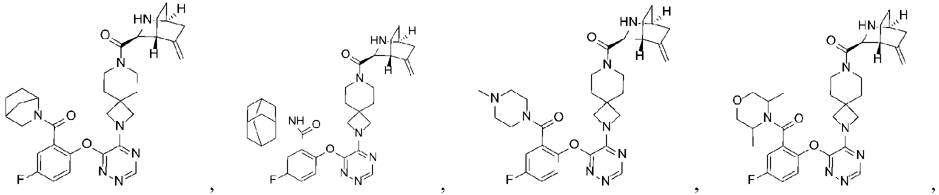

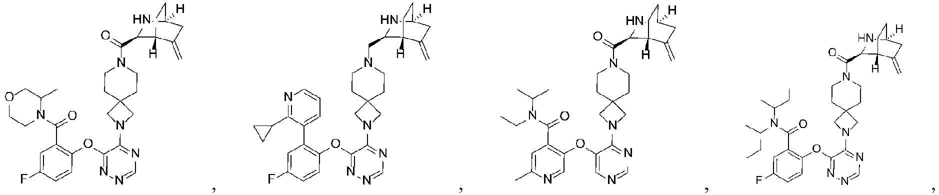

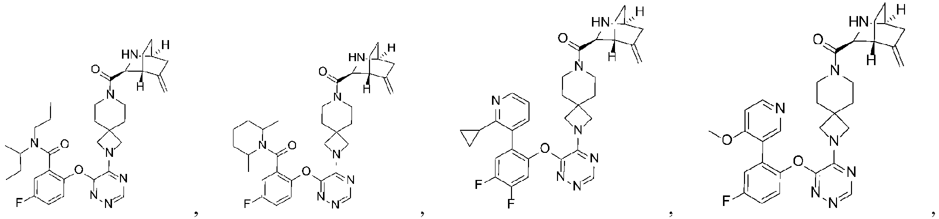

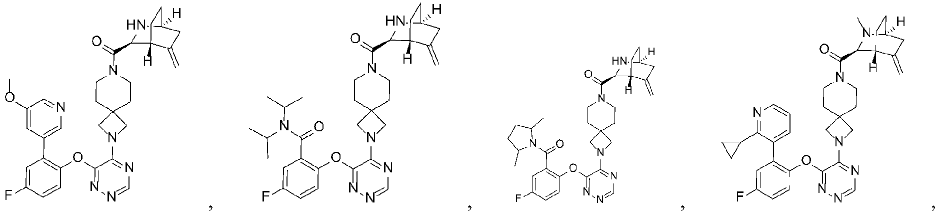

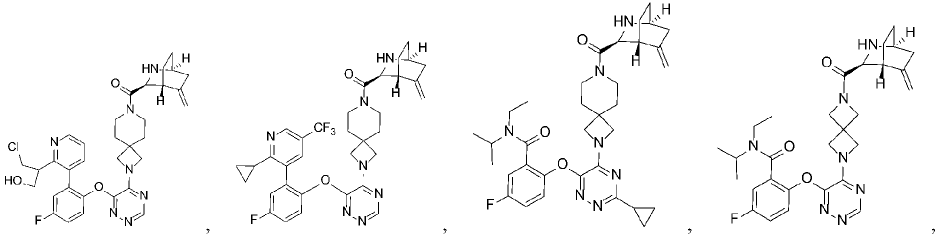

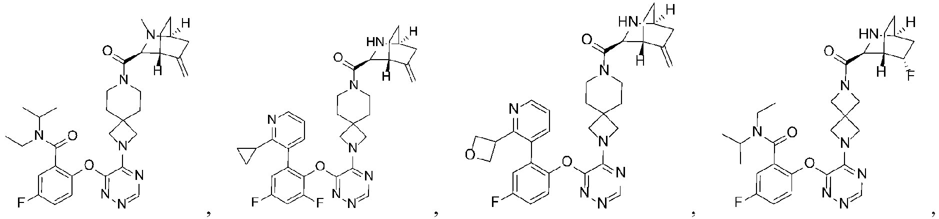

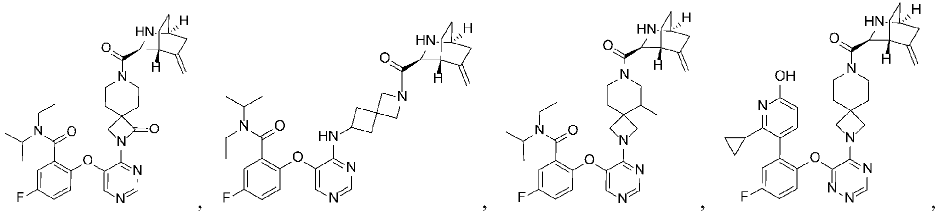

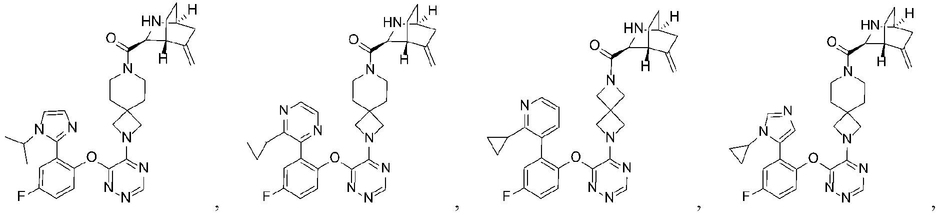

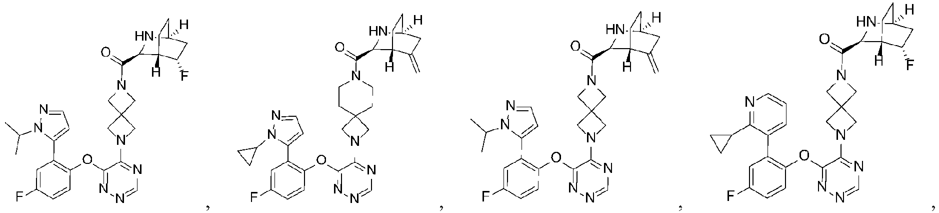

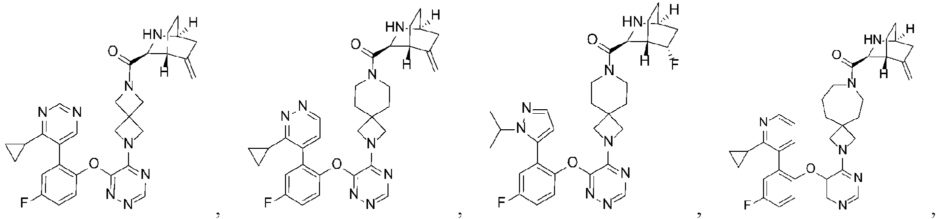

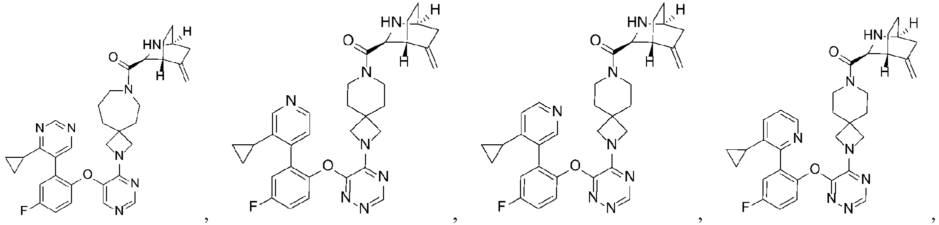

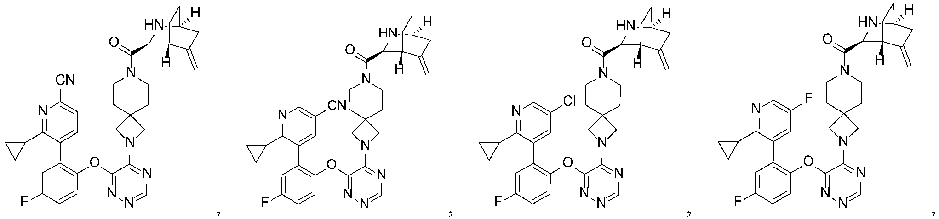

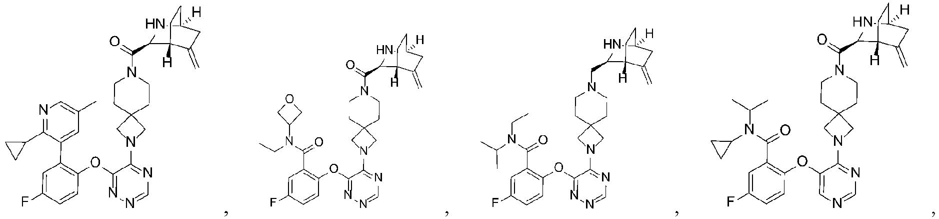

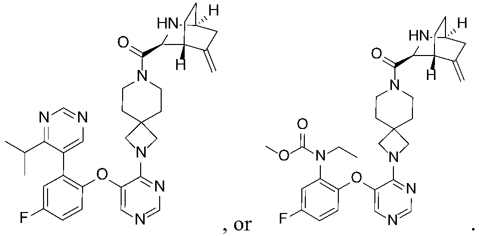

14. A pharmaceutical composition, comprising the compound or the pharmaceutically acceptable salt thereof according to any one of claims 1-13.

15. Use of the compound or the pharmaceutically acceptable salt thereof according to any one of claims 1-13 or the pharmaceutical composition according to claim 14 for preparing a medicament for preventing or treating a disease, wherein optionally, the disease is selected from cancer; optionally, the cancer is selected from leukemia.
